# EUROPEAN PATENT SPECIFICATION

(11) **EP 2 364 149 B1**
(45) Date of publication and mention of the grant of the patent: **07.05.2014**
(21) Application number: 09796056.1
(22) Date of filing: 30.11.2009
(51) Int. Cl.: A61K 31/426, C07D 277/56, A61P 35/00

(54) **TNIK INHIBITOR AND THE USE**
TNIK-INHIBITOR UND SEINE VERWENDUNG
INHIBITEUR DE TNIK ET SON UTILISATION

(30) Priority: 01.12.2008 US 118809 P; 15.07.2009 US 503374
(43) Date of publication of application: 14.09.2011
(73) Proprietor: National Cancer Center, Tokyo 104-0045 (JP); Carna Biosciences Inc., Kobe-shi, Hyogo 650-0047 (JP)
(72) Inventor: YAMADA, Tesshi, Chuo-ku, Tokyo 104-0045 (JP); SHITASHIGE, Miki, Chuo-ku, Tokyo 104-0045 (JP); YOKOTA, Koichi, Osaka 591-8033 (JP); SAWA, Masaaki, Osaka 567-0046 (JP); MORIYAMA, Hideki, Hyogo 652-0043 (JP)
(74) Representative: TBK
(86) International application number: PCT/IB2009/007597
(87) International publication number: WO 2010/064111

(56) References cited:
- JP-A- 2003 335 790
- EREMEEV ET AL.: "Addition of isocanates and isothiocyanates to 2-amino-3-phenylcarbamoylazirines" CHEMISTRY OF HETEROCYCLIC COMPOUNDS (NEW YORK, NY, UNITED STATES), vol. 22, no. 2, 1986, pages 227-228, XP002568477
- SCHAUMANN, ERNST ET AL: "Cycloaddition reactions of heterocumulenes. XXI. [2:1]- and [3:1]-Adducts from isocyanates and 3-dimethylamino-2H-azirines" LIEBIGS ANNALEN DER CHEMIE , (2), 264-76 CODEN: LACHDL; ISSN: 0170-2041, 1981, XP001536739
- DATABASE CA [Online] CHEMICAL ABSTRACTS SERVICE, COLUMBUS, OHIO, US; 1 January 1976 (1976-01-01), SEN, A. K. ET AL: "Synthesis of compounds related to 4(5)-aminoimidazole-5(4)-carboxamides: Part II" XP002568480 retrieved from STN Database accession no. 1976:543031 & INDIAN JOURNAL OF CHEMISTRY, SECTION B: ORGANIC CHEMISTRY INCLUDING MEDICINAL CHEMISTRY , 14B(5), 351-3 CODEN: IJSBDB; ISSN: 0376-4699, 1976,
- HICKMAN J A ET AL: "Protection against the effects of the antitumour agent CB 1954 by certain imidazoles and related compounds" BIOCHEMICAL PHARMACOLOGY, PERGAMON, OXFORD, GB, vol. 24, no. 21, 1 November 1975 (1975-11-01), pages 1947-1952, XP023850099 ISSN: 0006-2952 [retrieved on 1975-11-01]
- DATABASE CA [Online] CHEMICAL ABSTRACTS SERVICE, COLUMBUS, OHIO, US; 1 January 1949 (1949-01-01), COOK, A. H. ET AL: "Azole series. XXIII. New synthesis of 6-aminopurines" XP002568481 retrieved from STN Database accession no. 1950:19902 & JOURNAL OF THE CHEMICAL SOCIETY 3001-7 CODEN: JCSOA9; ISSN: 0368-1769, 1949,
- COOK A H ET AL: "Studies in the azole series. Part XVII. The preparation and cyclisation reactions of aminocyanoacetamide" JOURNAL OF THE CHEMICAL SOCIETY, CHEMICAL SOCIETY, LETCHWORTH; GB, 1 January 1949 (1949-01-01), pages 1440-1442, XP002234179 ISSN: 0368-1769
- KARAMAN MAZEN W ET AL: "A quantitative analysis of kinase inhibitor selectivity" NATURE BIOTECHNOLOGY, vol. 26, no. 1, January 2008 (2008-01), pages 127-132, XP002568478 ISSN: 1087-0156 -& KARAMAN MAZEN W ET AL.: "A quantitative analysis of kinase inhibitor selectivity" NATURE BIOTECHNOLOGY SUPPLEMENTARY INFORMATION, [Online] vol. 26, no. 1, 8 January 2008 (2008-01-08), XP002573235 Retrieved from the Internet: URL:http://www.nature.com/nbt/journal/v26/ n1/extref/nbt1358-S1.pdf>
- YOSHIKAWA ET AL.: "Polysaccharide-K" AACR MEETING ABSTRACTS ONLINE, no. abstr.727, 2005, XP002568479 Retrieved from the Internet: URL:http://www.aacrmeetingabstracts.org/cg i/content/abstract/2005/1/171-b>
- DATABASE REGISTRTY [Online] 1 May 2007 (2007-05-01), XP002568482 retrieved from STN Database accession no. 933853-24-8
- DATABASE REGISTRY [Online] 1 May 2007 (2007-05-01), XP002573236 retrieved from STN Database accession no. 933886-42-1
- DATABASE REGISTRY [Online] 1 May 2007 (2007-05-01), XP002573237 retrieved from STN Database accession no. 933918-05-9
- DATABASE REGISTRY [Online] 1 May 2007 (2007-05-01), XP002573238 retrieved from STN Database accession no. 933950-32-4
- DATABASE REGISTRY [Online] 21 August 2007 (2007-08-21), XP002568483 retrieved from STN Database accession no. 945119-78-8
- DATABASE REGISTRY [Online] 21 August 2007 (2007-08-21), XP002573239 retrieved from STN Database accession no. 945194-02-5
- DATABASE REGISTRY [Online] 26 December 2007 (2007-12-26), XP002568484 retrieved from STN Database accession no. 959549-11-2
- DATABASE REGISTRY [Online] 26 December 2007 (2007-12-26), XP002573240 retrieved from STN Database accession no. 959492-92-3
- DATABASE REGISTRY [Online] 29 June 2008 (2008-06-29), XP002568485 retrieved from STN Database accession no. 1031596-46-9
- DATABASE REGISTRY [Online] 29 June 2008 (2008-06-29), XP002573241 retrieved from STN Database accession no. 1031651-86-1
- DATABASE REGISTRY [Online] 1 July 2008 (2008-07-01), XP002573242 retrieved from STN Database accession no. 1031938-75-6
- DATABASE REGISTRY [Online] 24 February 2009 (2009-02-24), XP002568486 retrieved from STN Database accession no. 1111249-44-5
- DATABASE REGISTRY [Online] 24 February 2009 (2009-02-24), XP002573243 retrieved from STN Database accession no. 1111029-31-2
- DATABASE REGISTRY [Online] 25 February 2009 (2009-02-25), XP002574566 retrieved from STN Database accession no. 1111407-92-1
- DATABASE REGISTRY [Online] 24 February 2009 (2009-02-24), XP002574567 retrieved from STN Database accession no. 1112421-23-4

## Description

### FIELD OF THE INVENTION

The present invention relates to the use of Traf2- and Nck-interacting kinase (TNIK) inhibitors in the treatment of cancer patients. More particularly, the present invention relates to the use of pharmaceutical compositions comprising TNIK inhibitor and a pharmaceutically acceptable carrier for treating cancer patients, especially to solid cancer patients such as colorectal cancer, pancreatic cancer, non-small cell lung cancer, prostate cancer or breast cancer.

And the present invention relates to the medical use of aminothiazole derivatives.

### BACKGROUND OF THE INVENTION

Wnt proteins are a large family of secreted glycoproteins that activate signal transduction pathways to control a wide variety of cellular processes such as determination of cell fate, proliferation, migration, and polarity. Wnt proteins are capable of signaling through several pathways, the best-characterized being the canonical pathway through β-catenin (Wnt/β-catenin signaling). Deregulation of Wnt/β-catenin signaling is frequently found in many human cancers like colorectal cancer, pancreatic cancer, non-small cell lung cancer, prostate cancer, breast cancer, and many others.

TNIK is known as one of STE20 family kinases that activates the c-Jun N-terminal kinase pathway and regulates the cytoskeleton. Recently, TNIK was identified as one of 70 proteins immunoprecipitated commonly with anti-TCF4 in two colorectal cancer cell lines DLD1 and HCT-116 (Shitashige M, et al., Gastroenterology 2008, 134:1961-71).

The followings are referential descriptions about evaluation methods for utility of the present invention, which relates to compositions and methods for the treatment of cancer patients with TNIK inhibitors. Though these referential descriptions have already been described from filing of provisional application, these are described in also WO 2009/104413 filed by the present inventors before filing of provisional application and published after filing of provisional application. The details of the following evaluation methods for utility of the present invention are described in "REFERENTIAL METHODS" after "EXAMPLE" of present invention.

JP2003335790 (A) describes a phosphonic acid diester derivative is expressed by general formula (1) acting as an ACAT-1 inhibitor.

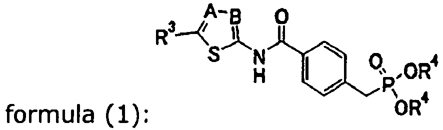

Ermeev et al. ("Addition of isocanates and isothiocyanates to 2-amino-3-phenylcarbamoylazirines" CHEMISTRY OF HETEROCYCLIC COMPOUNDS (NEW YORK, NY, US), vol. 22, no. 2, 1986, pages 227-228), Schaumann Erst et al. ("Cycloaddition reactions of heterocumulenes. XXI. [2:1]- and [3:1]-Adducts from isocyanates and 3-dimethylamino-2H-azirines"; LIEBIGS ANNALEN DER CHEMIE, (2), 264-76, 1981), Sen, A. K. et al. ("Synthesis of compounds related to 4(5)-aminoimidazole-5(4)-carboxamides: Part II" INDIAN JOURNAL OF CHEMISTRY, SECTION B: ORGANIC CHEMISTRY INCLUDING MEDICINAL CHEMISTRY , 14B(5), 351-3, 1976), Hickman J. A. et al: ("Protection against the effects of the antitumour agent CB 1954 by certain imidazoles and related compounds" BIOCHEMICAL PHARMACOLOGY, PERGAMON, OXFORD, GB, vol. 24, no. 21, 1 November 1975, pages 1947-1952), Cook, A. H. et al ("Azole series. XXIII. New synthesis of 6-aminopurines" JOURNAL OF THE CHEMICAL SOCIETY 3001-7, 1949), and Cook A. H. et al. ("Studies in the azole series. Part XVII. The preparation and cyclisation reactions of aminocyanoacetamide" JOURNAL OF THE CHEMICAL SOCIETY, CHEMICAL SOCIETY, LETCHWORTH; GB, 1 January 1949, pages 1440-1442) provide background information for the synthesis of aminothiazole compounds.

Karaman Mazan W. et al. ("A quantitative analysis of kinase inhibitor selectivity" NATURE BIOTECHNOLOGY, vol. 26, no. 1, January 2008 (2008-01), pages 127-132) inter alia investigates the selectivity of the ABL1/SCR inhibitor dasatinib coming to the conclusion that this unselective compound also binds to TNIK wit Kd 2000 nM.

Yoshikawa et al. ("Polysaccharide-K" AACR MEETING ABSTRACTS ONLINE, no. abstr.727, 2005) mentions that inter alia TNIK is upregulated in colorectal cancer patients.

### REFERENTIAL DESCRIPTION ABOUT AN EVALUATION METHOD FOR UTILITY OF THE PRESENT INVENTION

More than 80% of colorectal cancers show mutation of the adenomatous polyposis coli (*APC*) gene, and half of the remainder do so in the *CTNNB1* gene, resulting in accumulation of β-catenin and constitutive activation of Wnt signaling. β-Catenin exerts its oncogenic activity by forming complexes with T-cell factor (TCF)/lymphoid enhancer factor (LEF) family DNA-binding proteins and by transactivating their target genes. TCF4 is a TCF/LEF family member commonly expressed in colorectal cancer cells and implicated in colorectal carcinogenesis. We previously identified TNIK as one of 70 proteins immunoprecipitated commonly with anti-TCF4 and anti-β-catenin antibodies in two colorectal cancer cell lines (DLD1 and HCT-116) (Shitashige M, et al., Gastroenterology 2008, 134:1961-71). DLD1 has a truncating mutation in the *APC* gene and loss of the other allele, and HCT-116 has a missense mutation in the *CTNNB1* gene. TNIK was detected in the immunoprecipitates with anti-TCF4 or anti-β-catenin antibody, but not with control IgG. Conversely, β-catenin and TCF4 proteins were immunoprecipitated with anti-TNIK antibody, indicating that TCF4, β-catenin and TNIK proteins form a complex in colorectal cancer cells. Two-hybrid assay revealed that TNIK interacted with TCF4 through the amino acids 1-289 including the kinase domain. The amino acids 100-216 of TCF4 were necessary for interaction with TNIK.

TCF4 protein was phosphorylated by TNIK (WT, wild type), but not by the catalytically inactive mutant of TNIK with substitution (K54R) of the conserved lysine 54 residue in the ATP-binding pocket of the kinase domain. Tandem mass spectrometry (MS/MS) revealed that the serine 154 residue of TCF4 was phosphorylated by TNIK (WT). Consistently, substitution of the serine 154 residue by alanine (S154A) abolished the phosphorylation of TCF4 by TNIK. TCF4 was phosphorylated upon transfection of DLD1 cells with TNIK (WT), but not with TNIK (K54R).

Auto-phosphorylation of TNIK seems to be necessary for its nuclear translocation and interaction with TCF4. DLD1 and HCT-116 cells were transfected with TNIK (WT) or catalytically inactive TNIK (K54R) and analysed by immunoblotting and immunofluorescence microscopy. We found that TNIK (WT) induced the phosphorylation of its own serine 764 residue (TNIKpS764) (by anti-TNIKpS764). The phosphorylated TNIK was incorporated into the nuclei, whereas the K54R substitution significantly inhibited the phosphorylation and nuclear translocation of TNIK and reduced the amount of TNIK interacting with TCF4. Endogenous TNIK protein was distributed along the filamentous cytoskeleton, whereas phosphorylated TNIK (TNIKpS764) was detected mainly in the nuclei and co-localized with TCF4. TNIKpS764 was detected in colorectal cancer cells, but not in untransformed HEK293 cells.

The expression and localization of TNIK were examined in clinical specimens of colorectal cancer. Although the overall expression level of TNIK protein did not differ significantly between cancer and normal mucosa, the expression of phosphorylated TNIK (pS764) was increased in cancer cells compared to neighboring normal intestinal epithelial cells. Nuclear TNIKpS764 was detected most predominantly in the invasive front of colorectal cancer, where β-catenin was accumulated in the nucleus and cytoplasm.

We then investigated the effects of TNIK on the transcriptional activity of the β-catenin and TCF4 complex. HEK293 and HeLa cells have wild-type *APC* and *CTNNB1* genes. Transient transfection of these cells with β-catenin stabilized by deletion of the N-terminal glycogen synthase kinase 3β (GSK3β)-phosphorylation site (β-cateninΔN134) increased the luciferase activity of the canonical TCF/LEF reporter (TOP-FLASH) in comparison with mock transfection, but did not increase the luciferase activity of the mutant reporter (FOP-FLASH). Co-transfection with hemagglutinin (HA)-tagged wild-type TNIK (WT), but not with TNIK (K54R), further enhanced the β-catenin-evoked transcriptional activity and colony formation by HEK293 and HeLa cells. TNIK did not significantly affect transcriptional activity or colony formation in the absence of β-cateninΔN 134, indicating that the effects of TNIK are dependent upon activation of Wnt signaling. Transient transfection of DLD1 and HCT-116 cells with TNIK, but not with TNIK (K54R), also enhanced their TCF/LEF transcriptional activity and colony formation.

Conversely, knockdown of TNIK by short interfering RNA (siRNA) against TNIK (constructs 12 and 13) abolished the β-cateninΔN134-evoked TCF/LEF transcriptional activity of HEK293 and HeLa cells. Knockdown of TNIK by short hairpin RNA (shRNA) against TNIK (constructs T1, T2 and T3) abolished the β-cateninΔN134-evoked colony formation, but did not significantly affect the proliferation of HEK293 and HeLa cells that were not co-transfected with β-cateninΔN134. Knockdown of TNIK suppressed the TCF/LEF transcriptional activity and proliferation of DLD1 and HCT-116 cells. The expression of known target genes of the β-catenin and TCF/LEF complexes, such as axis inhibitor-2 (*AXIN2*), c-myc (*MYC*), c-jun *(JUN*) and matrilysin (*MMP7*), except for cyclin D1 (*CCND1*), was significantly reduced by transient transfection of siRNA against TN1K.

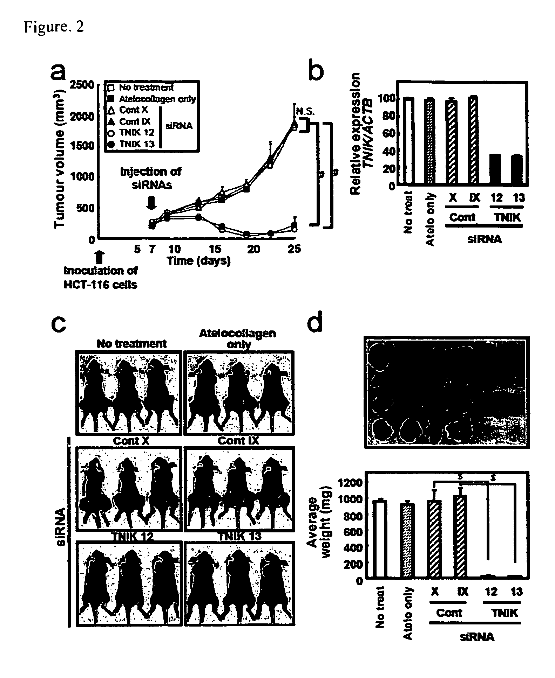

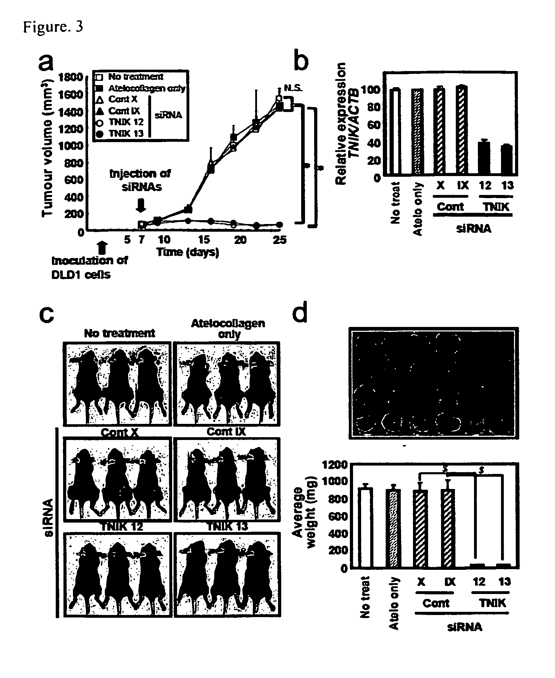

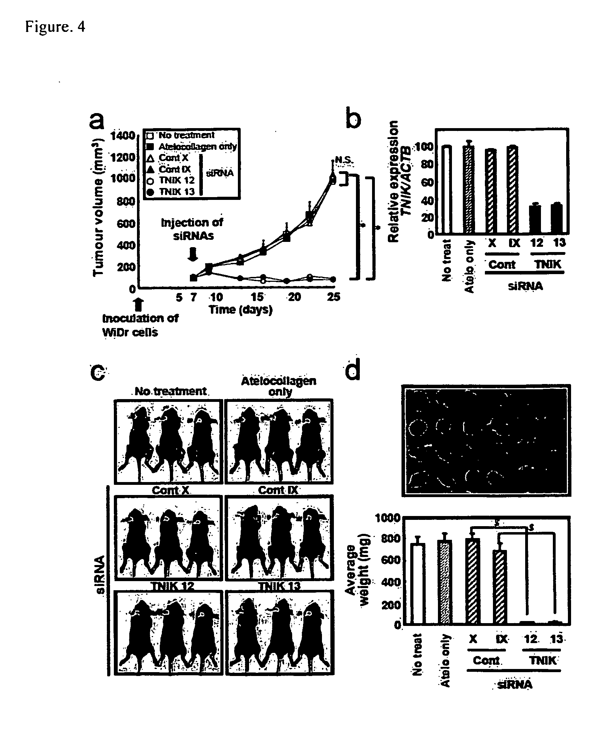

We next examined the effects of TNIK on the growth of human colon cancer cells *in vivo* (Fig. 2). HCT-116 cells were implanted in the flank of immunodeficient mice. One week after the inoculation, siRNA against TNIK (12 or 13) mixed with atelocollagen (Takeshita, F. et al. Efficient delivery of small interfering RNA to bone-metastatic tumors by using atelocollagen in vivo. Proc Natl Acad Sci U S A 102, 12177 (2005)) was injected directly into the tumours (224.5±8.9 mm³ in size). Three days after the siRNA injection, some tumours were excised and the silencing of TNIK mRNA was confirmed by real-time PCR (Fig. 2b). The volume of xenografts was monitored for 18 days after siRNA injection (Fig. 2a). We found that the tumours regressed almost completely after a single injection of siRNA against TNIK (12 or 13). Figure 2c and 2d shows the appearance of representative mice and excised tumours. Tumours treated with siRNA against TNIK (12 or 13) were significantly smaller than those not treated (No treat), treated with only atelocollagen (Atelo only) or treated with control RNA (X or IX) (Fig. 2d). We observed similar regression of established tumours after a single injection of siRNA against TNIK in two other colorectal cancer cell lines, DLDI and WiDr (Figs. 3 and 4).

Finally, the functional involvement of TNIK in Wnt signaling was examined in *Xenopus* embryos. There was a registry homologous to human TNIK in the Unigene *Xenopus laevis* database (named hypothetical protein LOC443633). The kinase domain (amino acids 25-289) was highly conserved (98.9%) between human and *Xenopus. Xenopus* TNIK (XTNIK) was expressed maternally and the expression was maintained throughout the tadpole stages. Ectopic activation of Wnt signaling in the ventral marginal zone is known to induce axis duplication. Co-injection with XTNIK (WT) mRNA enhanced X β -catenin-induced secondary axis formation, whereas catalytically inactive XTNIK (K54R) completely inhibited it. Embryos injected only with XTNIK (WT) or XTNIK (K54R) (without Xβ-catenin) developed normally. In the animal cap assay, injection of X β -catenin induced the expression of known target genes of Wnt signaling: *Siamois* and *Xnr3.* Co-injection with XTNIK (WT) enhanced the expression of these genes, whereas XTNIK (K54R) shut down their expression.

Injection of XTNIK (K54R) mRNA into the dorsal blastomeres of 8-cell-stage embryos inhibited the initiation of gastrulation at stage 10. Embryos injected dorsally with XTNIK (WT) showed a marked increase in the expression of *Siamois* and *Xnr3,* whereas XTNIK (K54R) decreased their expression. Embryos that received an injection of XTNIK (K54R) mRNA into the dorsal blastomeres at the 8-cell stage developed significant axis defects: complete loss of head and axis structures, typical phenotypes resulting from dorsal inhibition of Wnt signaling.

Antisense morpholino oligonucleotides (MOs) to XTNIK (MO1 and MO3) blocked secondary axis formation induced by Xβ -catenin when co-injected into the ventral marginal zone of 8-cell-stage embryos. The blockage was abrogated by co-injection of HA-tagged XTNIK_{ORF (open reading frame)} mRNA [lacking the 5'UTR (5'-untranslated region) targeted by MO1 and MO3]. Embryos injected dorsally with either XTNIK-MO failed to initiate gastrulation at stage 10 and developed into abnormal tadpoles with significantly reduced head and axis structures. The defects caused by XTNIK-MOs were rescued by co-injection of XTNIK_{ORF}. Embryos injected with control MOs with nucleotide mismatches (5mis-Control-1 and -3) did not show the effects observed in TNIK-MO1 and -MO3. The reduction of *Siamois* and *Xnr3* expression by XTNIK-MOs was reversed by co-injection of XTNIK_{ORF}.

Synthetic ATP-competitors of protein kinases have been incorporated successfully into oncological practice. For example, imatinib, which blocks the Bcr-Abl fusion kinase of chronic myeloid leukemia (CML), is currently a first-line therapeutic drug for CML. The epidermal growth factor receptor (EGFR) tyrosine kinase inhibitors, gefitinib and erlotinib, have been used in the treatment of non-small cell lung cancer. Wnt signaling is a major force driving colorectal carcinogenesis. TNIK was activated in colorectal cancer, and colorectal cancer cells were highly dependent upon the expression and kinase activity of TNIK for proliferation. Our results indicate the feasibility of developing drugs that target TNIK.

Though we explain our drawings as follows, Figure 2-4 are referential figures about evaluation methods of utility of present invention.

### BRIEF DESCRIPTION OF THE DRAWINGS

**Figure 1** **|** **Figure 1** **shows inhibition of β-catenin/TCF4-mediated transcription of colorectal cancer cells by Compound 1.**
**Figure 2** **| Referential figure) Inhibition of colorectal cancer growth by siRNA against TNIK.**
   a, HCT-116 cells were inoculated into BALB/c nu/nu nude mice subcutaneously. The developed tumours were not treated, or treated with only atelocollagen, control RNA (X or IX) or siRNA against TNIK (12 or 13). The volume of tumours (n=8 per group) was monitored as indicated. #, *P*<0.001 (Mann-Whitney U-test); Bars, SE; N.S., not significant.
   **b,** mRNA expression of *TNIK* in tumours injected with siRNA (n=3 per group).
   **c, d,** Representative appearance of mice and excised tumours 18 days after the injection of siRNA. Columns indicate the average weight of excised tumours (n=8 per group). $, P<0.005 (Mann-Whitney U-test); Bars, SE.
**Figures 3** **and** **4** **| (Referential figure) Growth inhibition and regression of colorectal tumours by siRNA against TNIK.**
   DLD1 **(****Fig.3****)** or WiDr **(****Fig.4****)** cells were inoculated into the flanks of BALB/c nu/nu nude mice on day 0. Developed tumours (DLD1, 64.0±1.9 mm³; WiDr, 95.9±1.6 mm³) were not treated or treated with only atelocollagen, control RNA (X or IX) or siRNA against TNIK (12 or 13) on day7.
   **a**, Volume of tumours (n=8 per group) measured on days 7, 9, 13, 16, 19, 22 and 25. *, *P*<0.0001 (Mann-Whitney U-test); Bars, SE; N.S., not significant.
   **b**, mRNA expression of *TNIK* in tumours (n=3 per group) determined by real-time PCR 3 days after siRNA injection.
   **c, d,** Representative appearance of mice **(c)** and excised tumours **(d)** and the weight of excised tumours (n=8 per group) **(d)** on day 25 (18 days after the injection of siRNA). $, P<0.005 (Mann-Whitney U-test); Bars, SE.

### SUMMARY OF THE INVENTION

These inventors have found that, TNIK is the essential protein kinase in the Wnt signaling pathway, is deeply concerned with proliferation of cancer, especially a solid tumor, for example pancreatic cancer, non-small cell lung cancer, prostate cancer or breast cancer (especially colorectal cancer), and proliferation of cancer, especially a solid tumor (especially colorectal cancer) can be controlled by inhibiting the action of TNIK.

### DETAILED DESCRIPTION OF THE INVENTION

Based on the knowledge concerned, the present inventors have screened the compounds which have a TNIK inhibitory activity, have found that the aminothiazole derivative shown by the following general formula (I) 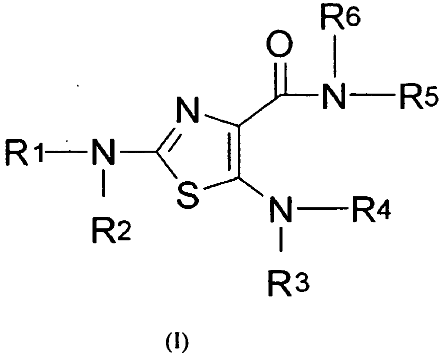 (wherein R1, R2, R3, R4, R5, and R6 independently represents a hydrogen atom or a substituent, respectively) or a pharmaceutically acceptable salt thereof has a TNIK inhibitory activity, have confirmed that the aminothiazole derivative suppresses proliferation of a cancer cell, and have completed this invention.

As a substituent in aminothiazole derivative (I), the following substituents can be mentioned, respectively.

As the substituent of R1 and R2, a substituted or unsubstituted alkyl group, a substituted or unsubstituted cycloalkyl group, a substituted or unsubstituted acyl group, a substituted or unsubstituted alkoxycarbonyl group, a substituted or unsubstituted carbamoyl group, a substituted or unsubstituted thiocarbamoyl group, a substituted or unsubstituted sulfonyl group, the substituted or unsubstituted heterocyclic ring, a substituted or unsubstituted aryl group, and substituted or unsubstituted heteroaromatic ring are mentioned.

As the substituent of R3 and R4, a substituted or unsubstituted alkyl group, a substituted or unsubstituted cycloalkyl group, a substituted or unsubstituted acyl group, a substituted or unsubstituted alkoxycarbonyl group, a substituted or unsubstituted carbamoyl group, a substituted or unsubstituted thiocarbamoyl group, a substituted or unsubstituted sulfonyl group, a substituted or unsubstituted heterocyclic ring, a substituted or unsubstituted aryl group, and a substituted or unsubstituted heteroaromatic ring are mentioned.

As the substituent of R5 and R6, a substituted or unsubstituted alkyl group, a substituted or unsubstituted cycloalkyl group, a substituted or unsubstituted heterocyclic ring, a substituted or unsubstituted aryl group, and a substituted or unsubstituted heteroaromatic ring are mentioned.

As the illustrative compound of aminothiazole derivative (I) or a pharmaceutically acceptable salt thereof, the following compounds or a pharmaceutically acceptable salt thereof are mentioned.
5-(4-acetamidobenzamido)-2-(phenylamino)thiazole-4-carboxam ide
5-(3-methylbenzamido)-2-(phenylamino)thiazole-4-carboxamide
5-(2-fluorobenzamido)-2-(phenylamino)thiazole-4-carboxamide
5-(4-methoxybenzamido)-2-(phenylamino)thiazole-4-carboxamide
5-(3-methoxybenzamido)-2-(phenylamino)thiazole-4-carboxamide
2-(phenylamino)-5-(2-(thiophen-2-yl)acetamido)thiazole-4-carboxamide
5-(3-methylbutanamido)-2-(phenylamino)thiazole-4-carboxamide
5-(2-cyclopentylacetamido)-2-(phenylamino)thiazole-4-carboxamide
2-(p-toluidino)-5-(2-fluorobenzamido)thiazole-4-carboxamide
2-(p-toluidino)-5-(4-acetamidobenzamido)thiazole-4-carboxamide
2-(p-toluidino)-5-(2-chlorobenzamido)thiazole-4-carboxamide
2-(p-toluidino)-5-(3-bromobenzamido)thiazole-4-carboxamide
2-(p-toluidino)-5-(2,6-difluorobenzamido)thiazole-4-carboxamide
2-(p-toluidino)-5-(3,4-dimethoxybenzamido)thiazole-4-carboxamide
2-(p-toluidino)-5-(thiophene-2-carboxamido)thiazole-4-carboxamide
2-(p-toluidino)-5-(2-ethylbutanamido)thiazole-4-carboxamide
2-(ethylamino)-5-(8-methyl-2-phenylquinoline-4-carboxamido)thiazole-4-carboxamide

Each of aminothiazole derivative (I) of this invention or a pharmaceutically acceptable salt thereof are well-known compounds, and can also be received from TimTec (Delaware, USA), Aurora Fine Chemicals (California, USA), etc.

Moreover, aminothiazole derivative (I) or a pharmaceutically acceptable salt thereof can be manufactured also by the procedure of illustrating below.

In the manufacturing method shown below, if a desired substituent is changed under the conditions of the methods or is unsuitable for proceeding the method, it can be manufactured easily by adding the procedure usually used in synthetic organic chemistry, for example the well-known procedure such as protection or deprotection of a functional group (T. W.Greene, Protective Groups in Organic Synthesis 3rd Edition, John Wiley & Sons, Inc., 1999 references).

Moreover, if needed, an order of reaction processes such as substituent induction is also changeable arbitrarily.

Compound (I) can be obtained with, for example, the manufacturing method shown in a process 1.

### (Process 1)

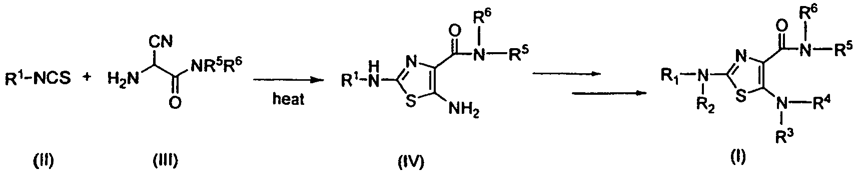

(R1, R2, R3, R4, R5, and R6 are as defined above.)

Compound (II) and (III) which are the starting materials of a process I be obtained as a commercial products (for example, Acros Organics product and URL:http://www.acros.com/), or obtained by either a well-known procedure or the procedure according to it.

Compound (IV) can be manufactured according to, for example, the procedure published in the paper (J. Chem. Soc. 1949, 3001 references) etc.

That is to say, compound (IV) can be obtained by carrying out the reaction of compound (II) and compound (III) among inert organic solvents, such as ethyl acetate.

Compound (I) can be obtained from compound (IV) by setting the conditions of acylation or alkylation well used in synthetic organic chemistry, if needed, repeating protection and deprotection of a functional group above mentioned.

Moreover, the pharmaceutically acceptable salt thereof illustrated below by the procedure well used in synthetic organic chemistry, if needed, can be obtained.

The pharmaceutically acceptable acid addition salt includes a salt with an inorganic acid such as hydrochloric acid, hydrobromic acid, sulfuric acid, or a salt with an organic acid such as maleic acid, fumaric acid, succinic acid, citric acid.

A further object of the present invention is the medical use of aminothiazole derivatives shown by the following general formula (I'): 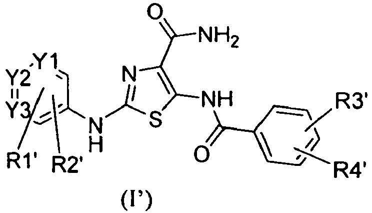 (wherein R I' and R2' independently represent a hydrogen atom, a halogen atom, a hydroxy group, a substituted or unsubstituted alkyl group, C2-C4 alkenyl group, C2-C4 alkynyl group, a substituted or unsubstituted alkoxy group, a substituted or unsubstituted amino group, an acylamino group, a nitro group, a substituted or unsubstituted alkoxycarbonylamino group, R3' and R4' independently represent a hydrogen atom, a halogen atom, a hydroxy group, a substituted or unsubstituted alkyl group, a substituted or unsubstituted alkoxy group, a substituted or unsubstituted amino group, a substituted or unsubstituted acylamino group or a substituted or unsubstituted alkyl sulfonamido group, a nitro group and Y1, Y2 and Y3 independently represent a nitrogen atom or a carbon atom.), or a pharmaceutically acceptable salt thereof.

The substituent as used herein includes, for example, a halogen atom (such as F, Cl, Br), a substituted or unsubstituted C1-C8 alkyl group, C2-C4 alkenyl group (such as vinyl, allyl), C2-C4 alkynyl group (such as ethynyl, propargyl), a substituted or unsubstituted C1-C8 alkoxy group, a substituted or unsubstituted C1-C4 acylamino group, or C1-C2 alkyl sulfonamido group. The substituted or unsubstituted amino group as used herein includes, for example, dimethylamino group, 4-methylpiperazin-1-yl group, 2-hydroxyethylamino group, 2- (dimethylamino)ethylamino group, 2-morpholinoethylamino group, 4-morpholino group, 2-(pyrrolidin-1-yl)ethylamino group, (2-hydroxyethyl)piperazin-1-yl group, 2-methoxyethylamino group, 2-aminoethylamino group, 4-(hydroxymethyl)piperidin-1-yl group, 2-(piperidin-1-yl)ethylamino group, 2-(pyridin-4-yl)ethylamino group or 2-(methylthio)ethylamino group. The substituted or unsubstituted C1-C8 alkoxy group as used herein includes, for example, methoxy group, benzyloxy group, 2-morpholinoethoxy group 2-(pyrrolidin-1-yl)ethoxy group or tetrahydro-2H-pyran-4-yloxy group. The substituted or unsubstituted acylamino group as used herein includes, for example, acetamido group, 2-hydroxyacetamido group, 2-(dimethylamino)acetamido group, 2-morpholinoacetamido group, 2-(pyrrolidin-1-yl)acetamido group, 2-(piperidin-1-yl)acetamido group or 2-(4-methylpiperazin-1-yl)acetamido group. The substituted or unsubstituted alkoxycarbonylamino group as used herein includes, for example, a substituted or unsubstituted C1-C8 alkoxycarbonylamino group (such as tert-butoxycarbonylamino group).

The following general reaction schemes detail the synthetic approaches to the aminothiazole derivatives disclosed herein. Compounds (I') disclosed herein can be prepared as shown in Schemes 1 - 6 and as illustrated in the Examples by using standard synthetic methods and the starting materials, which are either commercially available or can be synthesized from commercially available precursors using synthetic methods known in the art, or variations thereof as appreciated by those skilled in the art.

Although these schemes often indicate exact structures, those skilled in the art will appreciate that the methods apply widely to analogous compounds of Formula I', by being given appropriate consideration to protection and deprotection or reactive functional groups by methods standard to the art of organic chemistry. For example, hydroxy groups, in order to prevent unwanted side reactions, generally need to be converted to ethers or esters during chemical reactions at other sites in the molecule. The hydroxyl protecting group is readily removed to provide the free hydroxy group. Amino groups and carboxylic acid groups are similarly derivatized to protect them against unwanted side reactions. Typical protecting groups and methods for attaching and cleaving them are described fully by T. W. Greene, Protective Groups in Organic Synthesis 3rd Edition, John Wiley and Sons, Inc., New York (1999).

Each variable in the following schemes refers to any group consistent with the description of the compounds provided herein. Tautomers and solvates (e.g., hydrates) of the compounds of formula I are also within the scope of the invention.

Any compound of any formula disclosed herein can be obtained using procedures provided in the reaction Schemes, as well as procedures provided in the Examples, by selecting suitable starting materials and following analogous procedures. Thus, any compound of any formula disclosed or exemplified herein, can be obtained by using the appropriate starting materials and appropriate reagents, with the desired substitutions, and following procedures analogous to those described herein.

Compounds of Formula (I') are generally synthesized by the formation of the amide from 5-aminothiazole intermediate (II') and a substituted benzoyl chloride (III'-a), as shown in Scheme 1: 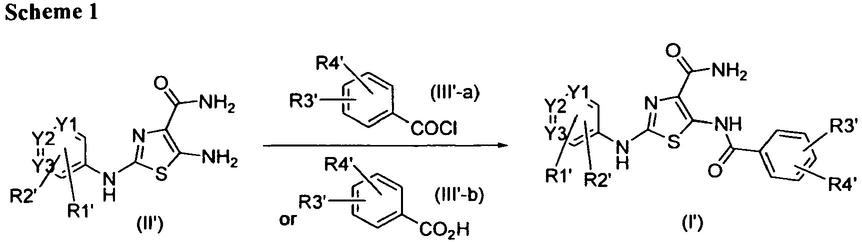 wherein R1', R2', R3', R4', Y1, Y2, and Y3 are the same as defined in the formula (I').

The same type of amide-coupling reaction may be done with a substituted benzoic acid (III'-b) under general amide coupling conditions such as 1-ethyl-3-(3-dimethylaminopropyl) carbodiimide hydrochloride (EDC), hydroxybenzotriazole (HOBT) and a base such as diisopropylethylamine or triethylamine to afford the compounds of Formula I'.

In another approach, compounds of Formula (I') may be prepared from the ester intermediate (IV') by a direct aminolysis with ammonia, as shown in Scheme 2: 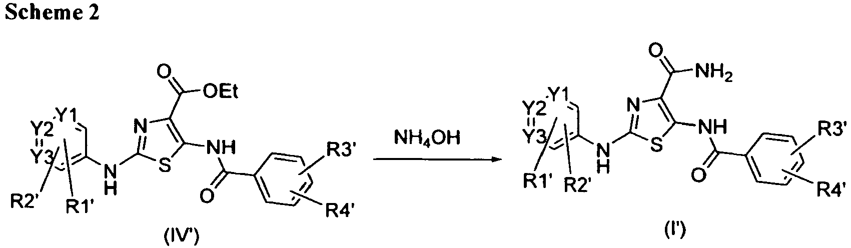 wherein R1', R2', R3', R4', Y1, Y2, and Y3 are the same as defined in the formula (I').

The aminolysis reaction is carried out by using concentrated ammonium hydroxide solution or ammonia in methanol in presence of a solvent such as THF, or dioxane. The reaction is stirred and heated in a sealed tube at a temperature from 80°C to 150°C, for 1-24 hours, preferably under microwave irradiation at 80°C for 150 minutes using a microwave synthesizer.

The compounds represented by the formula (II') in Scheme 1, which are used as starting materials of the amide-coupling reaction, may be prepared in a similar manner as described by Cook et al. (J. Chem. Soc. 1949, 3001). For example, the compounds represented by the formula (II') may be prepared by the scheme 3 below: 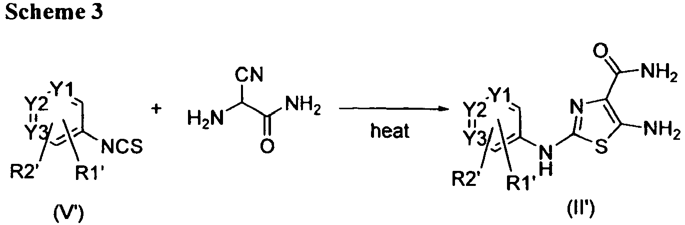 wherein R1', R2', Y1, Y2, and Y3 are the same as defined in the formula (I').

Thus, a mixture of thioisocyanate (V') and aminocyanoacetamide is stirred in a suitable solvent such as ethyl acetate, and heated to reflux condition for 0.5 - 2 hours to give the compounds represented by the formula (II').

The thioisocyanate (V') may be commercially available, or may be prepared from the corresponding amine by the methods well known in the field of organic synthesis, such as a thiophosgene treatment.

The substituted aminothiazole compounds (IV') may be prepared via a palladium-catalyzed reaction with an aniline or amino-heteroaromatic compound (VII') and 2-halogeno-thiazole compound (VI'), as shown in Scheme 4: 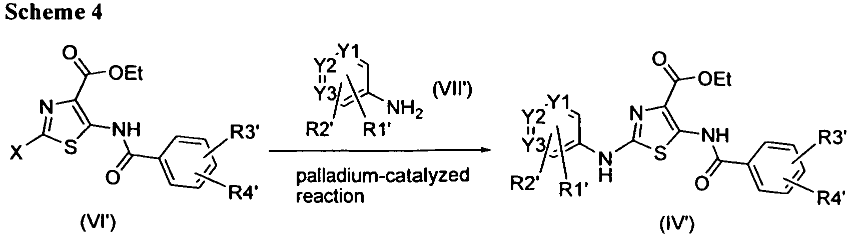 wherein R1', R2', R3', R4', Y1, Y2, and Y3 are the same as defined in the formula (I') and X is a halogen selected from Cl, Br and 1.

These Buckwald/Hartwig type reactions are well-known to those skilled in the art and are performed in inert solvents such as toluene, THF or dioxane and involve a palladium catalyst such as tris(dibenzylideneacetone)dipalladium (0), tetrakis(triphenylphosphine)palladium (0), palladium (II) acetate, and a base such as sodium, potassium or cesium carbonate and a ligand such as 4,5-Bis(diphenylphosphino)-9,9-dimethylxanthene (XANTPHOS). The same type of palladium-coupling reaction may be done with a corresponding halogeno-aromatic/heteroaromatic compound and a corresponding 2-aminothiazole analog to give the same desired aminothiazole intermediates (IV').

The compound represented by the formula (VI') may be prepared by the scheme 5 below: 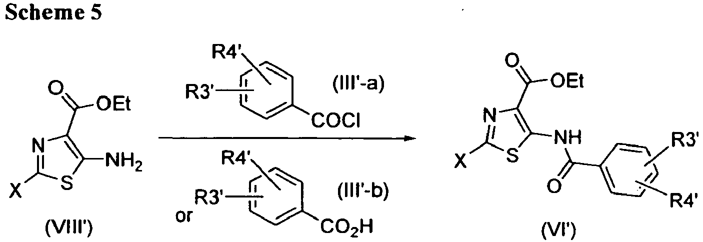 wherein R3' and R4' are the same as defined in the formula (I') and X is a halogen selected from Cl, Br and I.

Thus, the compound represented by the formula (VI') may be synthesized by the formation of the amide from 5-aminothiazole intermediate (VIII') and a substituted benzoyl chloride (III'-a). The same type of amide-coupling reaction may be done with a substituted benzoic acid (III'-b) under general amide coupling conditions such as EDC, HOBT and a base such as diisopropylethylamine, or triethylamine.

The compound represented by the formula (VIII') may be prepared from 5-aminothiazole-4-carboxylic acid ethyl ester by the scheme 6 below: 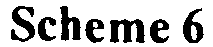 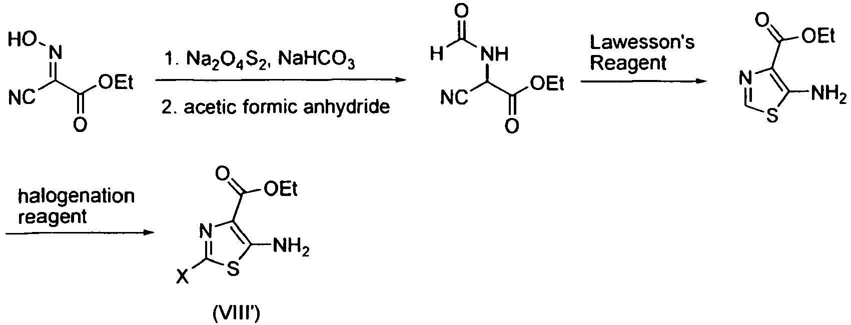 wherein X is a halogen selected from Cl, Br and I.

5-Aminothiazole-4-carboxylic acid ethyl ester is prepared according to the procedure described by Golankiewicz et al. (Tetrahedron, 41 (24), 5989-5994 (1985)). Thus, commercially available ethyl cyano(hydroxyimino)acetate is treated with sodium dithionate in sat. sodium bicarbonate aqueous solution to give ethyl 2-amino-2-cyanoacetate, which is then converted to the corresponding formamide with acetic formic anhydride. Subsequently, the obtained ethyl 2-cyano-2-formamidoacetate is treated with Lawesson's reagent, followed by treating with a halogenation reagent such as NCS, NBS to give the desired product.

The invention is further defined in the following Examples. It should be understood that these Examples are given by way of illustration only. From the above discussion and this Example, one skilled in the art can ascertain the essential characteristics of this invention, and without departing from the spirit and scope thereof, can make various changes and modifications to the invention to adapt the invention to various uses and conditions. As a result, the present invention is not limited by the illustrative examples set forth herein below, but rather defined by the claims appended hereto.

Specific examples of the compounds represented by the formula I' are given in Table I below:

**Table 1.**

| No. (Ex. No.) | Structure | Name |
|---|---|---|
| A1 (Ex.1) | 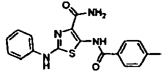 | 5-(4-methylbenzam ido)-2-(phenylam ino)thiazole-4-carboxamide |
| A2 (Ex.2) | 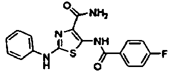 | 5-(4-fluorobenzamido)-2-(phenylamino)thiazole-4-carboxamide |
| A3 (Ex.3) | 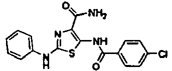 | 5-(4-chlorobenzamido)-2-(phenylamino)thiazole-4-carboxamide |
| A4 (Ex.4) | 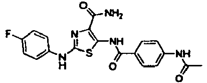 | 5-(4-acetamidobenzamido)-2-(4-fluorophenylami no)thiazole-4-carboxamide |
| A5 (Ex.5) | 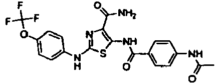 | 5-(4-acetamidobenzamido)-2-(4-(trifluoromethox y)phenylamino)thiazole-4-carboxamide |
| A6 (Ex.6) | 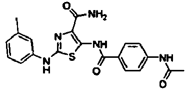 | 2-(m-toluidino)-5-(4-acetamidobenzamido)thiazo le-4-carboxamide |
| A7 (Ex.7) | 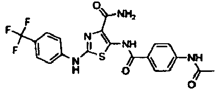 | 5-(4-acetamidobenzamido)-2-[4-(trifluoromethyl) phenylamino]thiazole-4-carboxamide |
| A8 (Ex.8) | 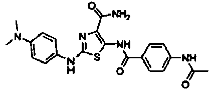 | 5-(4-acetamidobenzamido)-2-[4-(dimethylamino) phenylamino]thiazole-4-carboxamide |
| A9 (Ex.9) | 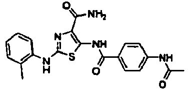 | 2-(o-toluidino)-5-(4-acetamidobenzamido)thiazol e-4-carboxamide |
| A10 (Ex.10) | 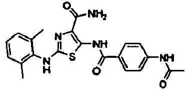 | 5-(4-acetamidobenzamido)-2-(2,6-dimethylpheny lamino)thiazole-4-carboxamide |
| A11 (Ex.11) | 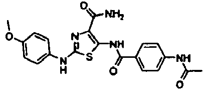 | 5-(4-acetamidobenzamido)-2-(4-methoxyphenyla mino)thiazole-4-carboxamide |
| A12 (Ex.12) | 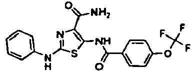 | 2-(phenylamino)-5-[4-(trifluoromethoxy)benzami do]thiazole-4-carboxamide |
| A13 (Ex.13) | 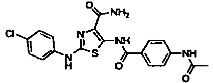 | 5-(4-acetamidobenzamido)-2-(4-chlorophenylami no)thiazole-4-carboxamide |
| A14 (Ex.14) | 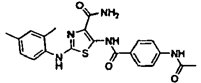 | 5-(4-acetamidobenzamido)-2-(2,4-dimethylpheny lamino)thiazole-4-carboxamide |
| A15 (Ex.15) | 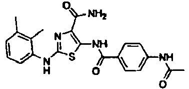 | 5-(4-acetamidobenzamido)-2-(2,3-dimethylpheny lamino)thiazole-4-carboxamide |
| A16 (Ex.16) | 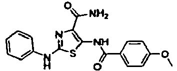 | 5-(4-methoxybenzamido)-2-(phenylamino)thiazol e-4-carboxamide |
| A17 (Ex.17) | 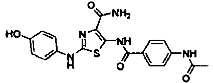 | 5-(4-acetamidobenzamido)-2-(4-hydroxyphenyla mino)thiazole-4-carboxamide |
| A18 (Ex.18) | 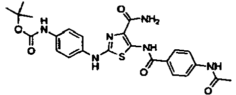 | tert-butyl 4-[5-(4-acetamidobenzamido)-4-carbamoylthiazo l-2-ylamino]phenylcarbamate |
| A19 (Ex.19) | 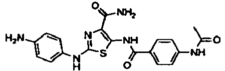 | 5-(4-acetamidobenzamido)-2-(4-aminophenylami no)thiazole-4-carboxamide |
| A20 (Ex.20) | 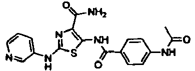 | 5-(4-acetamidobenzamido)-2-(pyridin-3-ylamino) thiazole-4-carboxamide |
| A21 (Ex.21) | 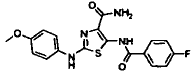 | 5-(4-fluorobenzamido)-2-(4-methoxyphenylamin o)thiazole-4-carboxamide |
| A22 (Ex.22) | 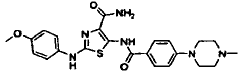 | 2-(4-methoxyphenylam ino)-5-[4-(4-methylpipera zin-1-yl)benzamido]thiazole-4-carboxamide |
| A23 (Ex.23) | 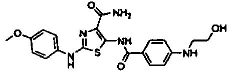 | 5-[4-(2-hydroxyethylamino)benzamido]-2-(4-met hoxyphenylamino)thiazole-4-carboxamide |
| A24 (Ex.24) | 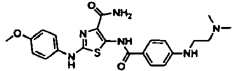 | 5-{4-[2-(dimethylamino)ethylamino]benzamido}-2-(4-methoxyphenylamino)thiazole-4-carboxami de |
| A25 (Ex.25) | 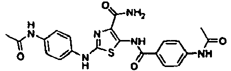 | 5-(4-acetamidobenzamido)-2-(4-acetamidophenyl amino)thiazole-4-carboxamide |
| A26 (Ex.26) | 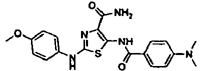 | 5-[4-(dimethylamino)benzamido]-2-(4-methoxyp henylamino)thiazole-4-carboxamide |
| A27 (Ex.27) | 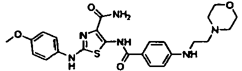 | 2-(4-methoxyphenylamino)-5-[4-(2-morpholinoet hylamino)benzamido]thiazole-4-carboxamide |
| A28 (Ex.28) | 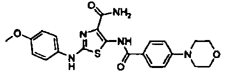 | 2-(4-methoxyphenylamino)-5-(4-morpholinobenz amido)thiazole-4-carboxamide |
| A29 (Ex.29) | 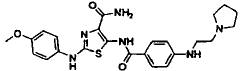 | 2-(4-methoxyphenylamino)-5-{4-[2-(pyrrolidin-1 -yl)ethylamino]benzamido}thiazole-4-carboxami de |
| A30 (Ex.30) | 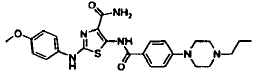 | 5-{4-[4-(2-hydroxyethyl)piperazin-1-yl]benzamid o}-2-(4-methoxyphenylamino)thiazole-4-carboxa mide |
| A31 (Ex.31) | 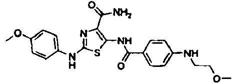 | 5-[4-(2-methoxyethylamino)benzamido]-2-(4-met hoxyphenylamino)thiazole-4-carboxamide |
| A32 (Ex.32) | 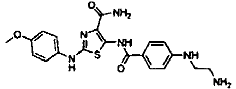 | 5-[4-(2-aminoethylamino)benzamido]-2-(4-metho xyphenylamino)thiazole-4-carboxamide |
| A33 (Ex.33) | 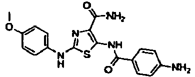 | 5-(4-aminobenzamido)-2-(4-methoxyphenylamin o)thiazole-4-carboxamide |
| A34 (Ex.34) | 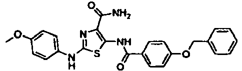 | 5-[4-(benzyloxy)benzamido]-2-(4-methoxypheny lamino)thiazole-4-carboxamide |
| A35 (Ex.35) | 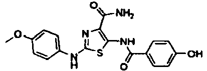 | 5-(4-hydroxybenzamido)-2-(4-methoxyphenylam ino)thiazole-4-carboxamide |
| A36 (Ex.36) | 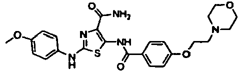 | 2-(4-methoxyphenylamino)-5-[4-(2-morpholinoet hoxy)benzamido]thiazole-4-carboxamide |
| A37 (Ex.37) | 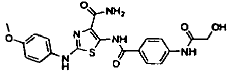 | 5-[4-(2-hydroxyacetamido)benzamido]-2-(4-meth oxyphenylamino)thiazole-4-carboxamide |
| A38 (Ex.38) | 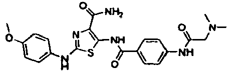 | 5-{4-[2-(dimethylamino)acetamido]benzamido}-2-(4-methoxyphenylamino)thiazole-4-carboxami de |
| A39 (Ex.39) | 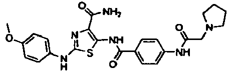 | 2-(4-methoxyphenylamino)-5-[4-(2-(pyrrolidin-1-yl)acetamido)benzamido)thiazole-4-carboxamide |
| A40 (Ex.40) | 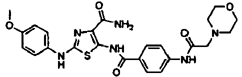 | 2-(4-methoxyphenylamino)-5-[4-(2-morpholinoa cetamido)benzamido]thiazole-4-carboxamide |
| A41 (Ex.41) | 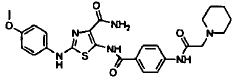 | 2-(4-methoxyphenylamino)-5-{4-[2-(piperidin-1-yl)acetamido]benzamido}thiazole-4-carboxamide |
| A42 (Ex.42) | 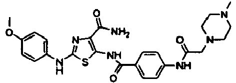 | 2-(4-methoxyphenylamino)-5-{4-[2-(4-methylpip erazin-1-yl)acetamido]benzamido}thiazole-4-carb oxamide |
| A43 (Ex.43) | 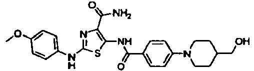 | 5-{4-[4-(hydroxymethyl)piperidin-1-yl]benzamid o}-2-(4-methoxyphenylamino)thiazole-4-carboxa mide |
| A44 (Ex.44) | 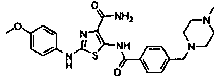 | 2-(4-methoxyphenylamino)-5-{4-[(4-methylpiper azin-1-yl)methyl]benzamido}thiazole-4-carboxa mide |
| A45 (Ex.45) | 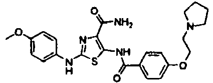 | 2-(4-methoxyphenylamino)-5-{4-[2-(pyrrolidin-1 -yl)ethoxy]benzamido}thiazole-4-carboxamide |
| A46 (Ex.46) | 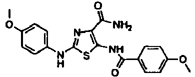 | 5-(4-methoxybenzamido)-2-(4-methoxyphenylam ino)thiazole-4-carboxamide |
| A47 (Ex.47) | 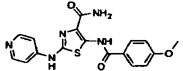 | 5-(4-methoxybenzamido)-2-(pyridin-4-ylamino)t hiazole-4-carboxamide |
| A48 (Ex.48) | 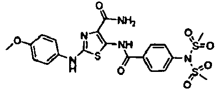 | 2-(4-methoxyphenylamino)-5-{4-[N-(methylsulfo nyl)methylsulfonamido]benzamido}thiazole-4-ca rboxamide |
| A49 (Ex.49) | 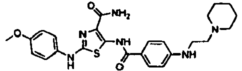 | 2-(4-methoxyphenylamino)-5-{4-[2-(piperidin-1-yl)ethylamino]benzamido}thiazole-4-carboxamid e |
| A50 (Ex.50) | 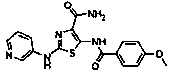 | 5-(4-methoxybenzamido)-2-(pyridin-3-ylamino)t hiazole-4-carboxamide |
| A51 (Ex.51) | 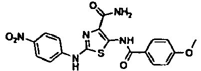 | 5-(4-methoxybenzamido)-2-(4-nitrophenylamino) thiazole-4-carboxamide |
| A52 (Ex.52) | 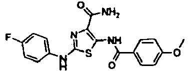 | 2-(4-fluorophenylamino)-5-(4-methoxybenzamid o)thiazole-4-carboxamide |
| A53 (Ex.53) | 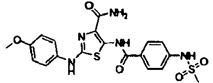 | 2-(4-methoxyphenylamino)-5-[4-(methylsulfona mido)benzamido]thiazole-4-carboxamide |
| A54 (Ex.54) | 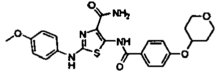 | 2-(4-methoxyphenylamino)-5-[4-(tetrahydro-2H-pyran-4-yloxy)benzamido]thiazole-4-carboxamid e |
| A55 (Ex.55) | 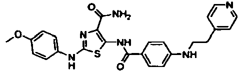 | 2-(4-methoxyphenylamino)-5-{4-[2-(pyridin-4-yl )ethylamino]benzamido}thiazole-4-carboxamide |
| A56 (Ex.56) | 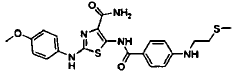 | 2-(4-methoxyphenylamino)-5-{4-[2-(methylthio) ethylamino]benzamido}thiazole-4-carboxamide |
| A57 (Ex.57) | 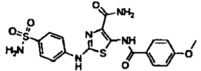 | 5-(4-methoxybenzamido)-2-(4-sulfamoylphenyla mino)thiazole-4-carboxamide |
| A58 (Ex.58) | 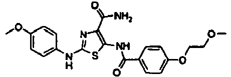 | 5-[4-(2-methoxyethoxy)benzamido]-2-(4-methox yphenylamino)thiazole-4-carboxamide |
| A59 (Ex.59) | 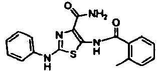 | 5-(2-methylbenzamido)-2-(phenylamino)thiazole-4-carboxamide |
| A60 (Ex.60) | 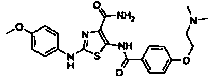 | 5-{4-[2-(dimethylamino)ethoxy]benzamido}-2-(4 -methoxyphenylamino)thiazole-4-carboxamide |
| A61 (Ex.61) | 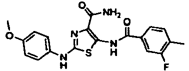 | 5-(3-fluoro-4-methylbenzamido)-2-(4-methoxyph enylamino)thiazole-4-carboxamide |
| A62 (Ex.62) | 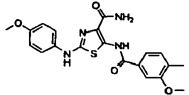 | 5-(3-methoxy-4-methylbenzamido)-2-(4-methoxy phenylamino)thiazole-4-carboxamide |
| A63 (Ex.63) | 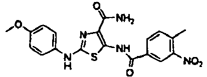 | 2-(4-methoxyphenylamino)-5-(4-methyl-3-nitrob enzamido)thiazole-4-carboxamide |
| A64 (Ex.64) | 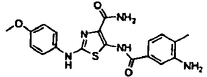 | 5-(3-amino-4-methylbenzamido)-2-(4-methoxyph enylamino)thiazole-4-carboxamide |
| A65 (Ex.65) | 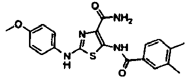 | 5-(3,4-dimethylbenzamido)-2-(4-methoxyphenyla mino)thiazole-4-carboxamide |
| A66 (Ex.66) | 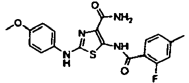 | 5-(2-fluoro-4-methylbenzamido)-2-(4-methoxyph enylamino)thiazole-4-carboxamide |
| A67 (Ex.67) | 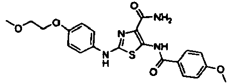 | 5-(4-methoxybenzamido)-2-[4-(2-methoxyethoxy )phenylamino]thiazole-4-carboxamide |
| A68 (Ex.68) | 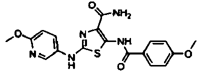 | 5-(4-methoxybenzamido)-2-(6-methoxypyridin-3 -ylamino)thiazole-4-carboxamide |
| A69 (Ex.69) | 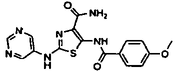 | 5-(4-methoxybenzamido)-2-(pyrimidin-5-ylamin o)thiazole-4-carboxamide |
| A70 (Ex.70) | 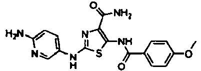 | 2-(6-aminopyridin-3-ylamino)-5-(4-methoxybenz amido)thiazole-4-carboxamide |
| A71 (Ex.71) | 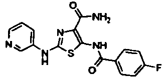 | 5-(4-fluorobenzamido)-2-(pyridin-3-ylamino)thia zole-4-carboxamide |
| A72 (Ex.72) | 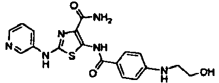 | 5-[4-(2-hydroxyethylamino)benzamido]-2-(pyridi n-3-ylamino)thiazole-4-carboxamide |
| A73 (Ex.73) | 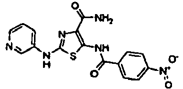 | 5-(4-nitrobenzamido)-2-(pyridin-3-ylamino)thiaz ole-4-carboxamide |
| A74 (Ex.74) | 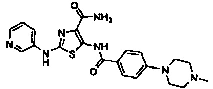 | 5-[4-(4-methylpiperazin-1-yl)benzamido]-2-(pyri din-3-ylamino)thiazole-4-carboxamide |
| A75 (Ex.75) | 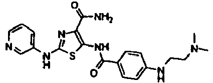 | 5-(4-[2-(dimethylamino)ethylamino]benzamido)-2-(pyridin-3-ylamino)thiazole-4-carboxamide |
| A76 (Ex.76) | 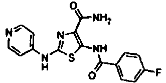 | 5-(4-fluorobenzamido)-2-(pyridin-4-ylamino)thia zole-4-carboxamide |
| A77 (Ex.77) | 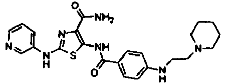 | 5-{4-[2-(piperidin-1-yl)ethylamino]benzamido}-2 -(pyridin-3-ylamino)thiazole-4-carboxamide |
| A78 (Ex.78) | 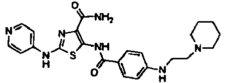 | 5-(4-[2-(piperidin-1-yl)ethylamino]benzamido)-2 -(pyridin-4-ylamino)thiazole-4-carboxamide |
| A79 (Ex.79) | 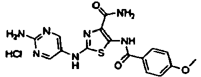 | 2-(2-aminopyrimidin-5-ylamino)-5-(4-methoxybe nzamido)thiazole-4-carboxamide hydrochloride |
| A80 (Ex.80) | 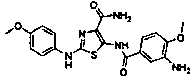 | 5-(3-am ino-4-methoxybenzam ido)-2-(4-methoxy phenylamino)thiazole-4-carboxamide |
| A81 (Ex.81) | 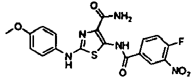 | 5-(4-fluoro-3-nitrobenzamido)-2-(4-methoxyphen ylamino)thiazole-4-carboxamide |
| A82 (Ex.82) | 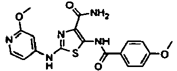 | 5-(4-methoxybenzamido)-2-(2-methoxypyridin-4 -ylamino)thiazole-4-carboxamide |
| A83 (Ex.83) | 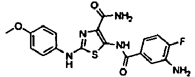 | 5-(3-amino-4-fluorobenzamido)-2-(4-methoxyph enylamino)thiazole-4-carboxamide |
| A84 (Ex.84) | 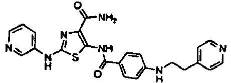 | 2-(pyridin-3-ylamino)-5-{4-[2-(pyridin-4-yl)ethyl amino]benzamido}thiazole-4-carboxamide |
| A85 (Ex.85) | 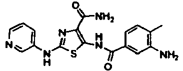 | 5-(3-amino-4-methylbenzamido)-2-(pyridin-3-yla mino)thiazole-4-carboxamide |
| A86 (Ex.86) | 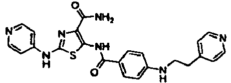 | 5-{-4-[2-(pyridin-4-yl)ethylamino]benzamido}-2-( pyridin-4-ylamino)thiazole-4-carboxamide |
| A87 (Ex.87) | 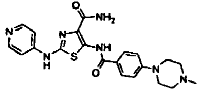 | 5-[4-(4-methylpiperazin-1-yl)benzamido]-2-(pyri din-4-ylamino)thiazole-4-carboxamide |
| A88 (Ex.88) | 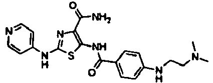 | 5-{4-[2-(dimethylamino)ethylamino]benzamido}-2-(pyridin-4-ylamino)thiazole-4-carboxamide |
| A89 (Ex.89) | 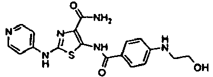 | 5-[4-(2-hydroxyethylamino)benzamido]-2-(pyridi n-4-ylamino)thiazole-4-carboxamide |
| A90 (Ex.90) | 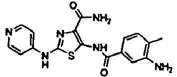 | 5-(3-amino-4-methylbenzamido)-2-(pyridin-4-yla mino)thiazole-4-carboxamide |
| A91 (Ex.91) | 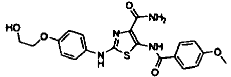 | 2-[4-(2-hydroxyethoxy)phenylamino]-5-(4-metho xybenzamido)thiazole-4-carboxamide |
| A92 (Ex.92) | 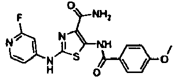 | 2-(2-fluoropyridin-4-ylamino)-5-(4-methoxybenz amido)thiazole-4-carboxamide |
| A93 (Ex.93) | 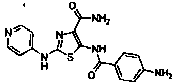 | 5-(4-aminobenzamido)-2-(pyridin-4-ylamino)thia zole-4-carboxamide |
| A94 (Ex.94) | 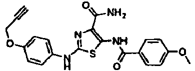 | 5-(4-methoxybenzamido)-2-[4-(prop-2-ynyloxy)p henylamino]thiazole-4-carboxamide |
| A95 (Ex.95) | 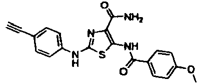 | 2-(4-ethynylphenylamino)-5-(4-methoxybenzami do)thiazole-4-carboxamide |
| A96 (Ex.96) | 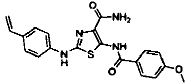 | 5-(4-methoxybenzamido)-2-(4-vinylphenylamino )thiazole-4-carboxamide |
| A97 (Ex.97) | 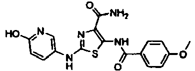 | 2-(6-hydroxypyridin-3-ylamino)-5-(4-methoxybe nzam ido)th iazole-4-carboxam ide |
| A98 (Ex.98) | 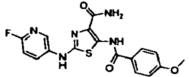 | 2-(6-fluoropyridin-3-ylamino)-5-(4-methoxybenz amido)thiazole-4-carboxamide |
| A99 (Ex.99) | 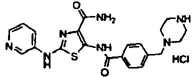 | 5-(4-(piperazin-1-ylmethyl)benzamido)-2-(pyridi n-3-ylamino)thiazole-4-carboxamide hydrochloride |
| A100 (Ex.100) | 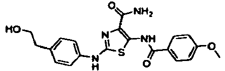 | 2-[4-(2-hydroxyethyl)phenylamino]-5-(4-methox ybenzamido)thiazole-4-carboxamide |
| A101 (Ex.101) | 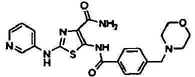 | 5-[4-(morpholinomethyl)benzamido]-2-(pyridin-3 -ylamino)thiazole-4-carboxamide |
| A 102 (Ex.102) | 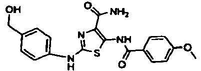 | 2-[4-(hydroxymethyl)pheny lam ino]-5-(4-methox ybenzamido)thiazole-4-carboxamide |
| A103 (Ex.103) | 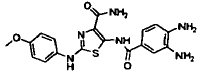 | 5-(3,4-diaminobenzamido)-2-(4-methoxyphenyla mino)thiazole-4-carboxamide |
| A104 (Ex.104) | 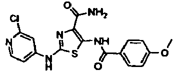 | 2-(2-chloropyridin-4-ylamino)-5-(4-methoxybenz amido)thiazole-4-carboxamide |
| A105 (Ex. 105) | 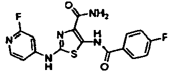 | 5-(4-fluorobenzamido)-2-(2-fluoropyridin-4-ylam ino)thiazole-4-carboxamide |
| A106 (Ex.106) | 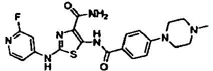 | 2-(2-fluoropyridin-4-ylamino)-5-[4-(4-methylpip erazin-1-yl)benzamido]thiazole-4-carboxamide |
| A107 (Ex.107) | 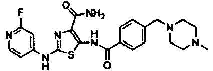 | 2-(2-fluoropyridin-4-ylamino)-5-{4-[(4-methylpi perazin-1-yl)methyl]benzamido}thiazole-4-carbo xamide |
| A108 (Ex.108) | 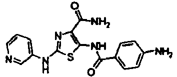 | 5-(4-aminobenzamido)-2-(pyridin-3-ylamino)thia zole-4-carboxamide |
| A109 (Ex.109) | 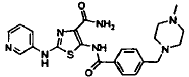 | 5-{4-[(4-methylpiperazin-1-yl)methyl]benzamido }-2-(pyridin-3-ylamino)thiazole-4-carboxamide |
| A110 (Ex.110) | 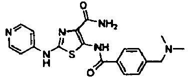 | 5-{4-[(dimethylamino)methyl]benzamido}-2-(pyr idin-4-ylamino)thiazole-4-carboxamide |
| A111 (Ex.111) | 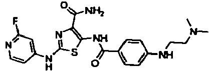 | 5-{4-[2-(dimethylamino)ethylamino]benzamido}-2-(2-fluoropyridin-4-ylamino)thiazole-4-carboxa mide |
| A112 (Ex.112) | 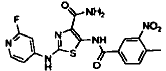 | 2-(2-fluoropyridin-4-ylamino)-5-(4-methyl-3-nitr obenzamido)thiazole-4-carboxamide |
| A113 (Ex.113) | 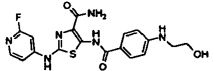 | 2-(2-fluoropyridin-4-ylamino)-5-[4-(2-hydroxyet hylamino)benzamido]thiazole-4-carboxamide |
| A114 (Ex.114) | 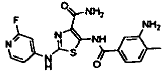 | 5-(3-amino-4-methylbenzamido)-2-(2-fluoropyrid in-4-ylamino)thiazole-4-carboxamide |
| A115 (Ex.115) | 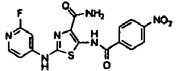 | 2-(2-fluoropyridin-4-ylamino)-5-(4-nitrobenzami do)thiazole-4-carboxamide |
| A 116 (Ex.116) | 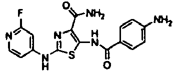 | 5-(4-aminobenzamido)-2-(2-fluoropyridin-4-ylam ino)thiazole-4-carboxamide |
| A117 (Ex.117) | 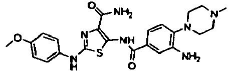 | 5-[3-amino-4-(4-methylpiperazin-1-yl)benzamido ]-2-(4-methoxyphenylamino)thiazole-4-carboxam ide |
| A118 (Ex.118) | 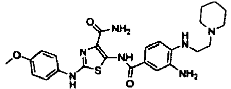 | 5-{3-amino-4-[2-(piperidin-1-yl)ethylamino]benz amido}-2-(4-methoxyphenylamino)thiazole-4-car boxamide |
| A119 (Ex.119) | 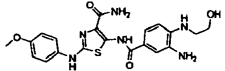 | 5-[3-amino-4-(2-hydroxyethylamino)benzamido]-2-(4-methoxyphenylamino)thiazole-4-carboxami de |
| A120 (Ex.120) | 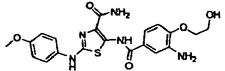 | 5-[3-amino-4-(2-hydroxyethoxy)benzamido]-2-(4 -methoxyphenylamino)thiazole-4-carboxamide |
| A121 (Ex.121) | 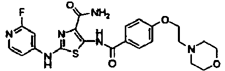 | 2-(2-fluoropyridin-4-ylamino)-5-[4-(2-morpholin oethoxy)benzamido]thiazole-4-carboxamide |
| A122 (Ex.122) | 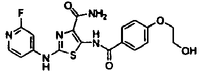 | 2-(2-fluoropyridin-4-ylamino)-5-[4-(2-hydroxyet hoxy)benzamido]thiazole-4-carboxamide |
| A123 (Ex.123) | 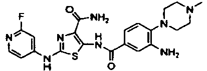 | 5-[3-amino-4-(4-methylpiperazin-1-yl)benzamido ]-2-(2-fluoropyridin-4-ylamino)thiazole-4-carbox amide |
| A124 (Ex.124) | 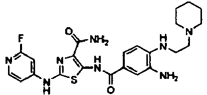 | 5-{3-amino-4-[2-(piperidin-1-yl)ethylamino]benz amido}-2-(2-fluoropyridin-4-ylamino)thiazole-4-carboxamide |
| A125 (Ex.125) | 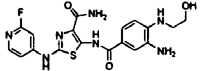 | 5-[3-amino-4-(2-hydroxyethylamino)benzamido]-2-(2-fluoropyridin-4-ylamino)thiazole-4-carboxa mide |
| A126 (Ex.126) | 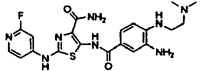 | 5-{3-amino-4-[2-(dimethylamino)ethylamino]ben zamido}-2-(2-fluoropyridin-4-ylamino)thiazole-4 -carboxamide |
| A127 (Ex.127) | 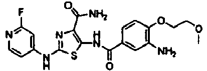 | 5-[3-amino-4-(2-methoxyethoxy)benzamido]-2-( 2-fluoropyridin-4-ylamino)thiazole-4-carboxamid e |
| A128 (Ex.128) | 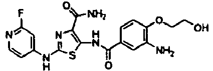 | 5-[3-amino-4-(2-hydroxyethoxy)benzamido]-2-(2 -fluoropyridin-4-ylamino)thiazole-4-carboxamide |
| A 129 (Ex.129) | 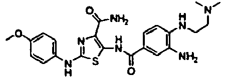 | 5-{3-amino-4-[2-(dimethylamino)ethylamino]ben zamido}-2-(4-methoxyphenylamino)thiazole-4-ca rboxamide |

The aminothiazole derivatives (I) and aminothiazole derivatives (I') show the TNIK inhibitory effects (Test Example 1) and do not show undesirable activity (Test Example 2).

The aminothiazole derivatives show the anti-tumor activity (Test Example 3) and low toxicity.

The aminothiazole derivatives may be used as a pharmaceutical composition (for example an anti-tumor agent) in the form of a conventional pharmaceutical preparation for an oral or parenteral administration such as intravenous drip injection.

The preparation for oral administration includes solid preparations such as tablets, granules, powders, capsules, and liquid preparations such as syrups. These preparations can be prepared by a conventional method. The solid preparations can be prepared by using conventional pharmaceutical carriers, such as lactose, starch such as cornstarch, crystalline cellulose such as microcrystalline cellulose, hydroxypropyl cellulose, calcium carboxymethylcellulose, talc, magnesium stearate, etc. Capsules can be prepared by capsulating the granules or powders thus prepared. Syrups can be prepared by dissolving or suspending the aminothiazole derivatives in an aqueous solution containing sucrose, carboxymethylcellulose, etc.

The preparation for parenteral administration includes injections such as intravenous drip injection. The injection preparation can also be prepared by a conventional method, and optionally may be incorporateed in isotonic agents (e.g. mannitol, sodium chloride, glucose, sorbitol, glycerol, xylitol, fructose, maltose, mannose), stabilizers (e.g. sodium sulfite, albumin), preservatives (e.g. benzyl alcohol, methyl p-hydroxybenzoate).

The aminothiazole derivatives are effective for the treatment of tumors, especially solid tumors such as colorectal cancer, pancreatic cancer, non-small cell lung cancer, prostate cancer or breast cancer.

The dose of the aminothiazole derivatives may vary according to the severity of the diseases, ages and body weights of the patients, dosage forms and the like, but is usually in the range of 1mg - 1,000 mg per day in an adult, which may be administered once or by dividing into two or three times by the oral or parenteral route.

### TEST EXAMPLE

### TEST EXAMPLE I

### Preparation of recombinant human TNIK (N-terminal segment):

A cDNA encoding the N-terminal segment (TNIK_N, residues 1-314) containing the kinase domain of human TNIK (NM_015028.1) was amplified from cDNA mixture synthesized from human tissue (Biochain) by PCR using the following primers: 5'-AATTTCAGGGCGCCATGGCGAGCGACTCCCCGGCTCGAAG-3' ([SEQ ID NO.1] : forward primer, the underlined nucleotides indicates the location of a *Ehe*I site); 5'-ATTCGAAAGCGGCCGCTCATCCTCGCTTCTTCTTTGTTCTAT-3' ([SEQ ID NO.2]: reverse primer, the underlined nucleotides indicates the location of a *Not*I site). The cDNA was subcloned into baculovirus transfer vector pFastBac_GSTb that includes protease cleavage site and glutathione S-transferase purification tag (GST-tag). The plasmid was purified and the insertion of the pFastBac_GSTb-TNIK_N was confirmed by DNA sequencing. Then *E.coli* DH10Bac competent cells were transformed with the plasmid to prepare the recombinant bacmid according to the instructions for the Bac-to-Bac™ baculovirus expression systems (Invitrogen). The *Sf*9 cells were transfected with the recombinant bacmid containing pFastBac_GSTb-TNIK_N using Cellfectin Reagent (Invitrogen) in SF-90011 serum free media (invitrogen). The viral supernatant was collected from the medium 72 h after transfection. The virus was amplified three times by infecting actively growing *Sf*9 or *Sf*21 cells in Grace's insect media (Invitrogen) supplemented with 10% FCS and an antibiotic-antimycotic reagent (Invitrogen) for 72 h at 27°C in T-flask or roller bottles. The titer of amplified TNIK_N virus was estimated at 2.36x10⁸ pfu/ml by using BacPAK™ Baculovirus Rapid Titer kit (Clontech).

Log-phase *Sf*21 cells (2 x 10⁶ cells/ml) in the Grace's insect media were infected with the recombinant baculovirus at MOI of 3.0 and incubated in roller bottles (250 ml media per bottle) for 72 h at 27°C, after which, the cells were collected by centrifugation, and the cell pellet washed with cold PBS and kept at -80°C until purification. The following purification procedures were carried out at 4°C. The frozen cells were thawed on ice and lysed in lysis buffer (50 mM Tris-HCl, pH 7.5, 150 mM NaCl, 1% Nonidet P-40, 5 mM DTT, 0.5 mM EDTA, 0.5 mM EGTA) supplemented with 1 mM phenylmethansulfonylfluoride, 2 µg/ml leupeptin, 2 µg/ml aprotinin, I mM NaF, 100 µM sodium orthovanadate, and I µM cantharidin by sonication. The suspended lysate was cleared by centrifugation at 9000g for 20 min and the supernatant was incubated for 1h with glutathione Sepharose beads (GE Healthcare). The beads were suspended in buffer-H (50 mM Tris-HCl, pH 7.5, 1 M NaCl, I mM DTT, 0.5 mM EDTA, 0.5 mM EGTA and 0.05% Brij35) and washed with buffer-H followed by buffer-L (50 mM Tris-HCl, pH 7.5, 150 mM NaCl, 1 mM DTT, 0.5 mM EDTA, 0.5 mM EGTA, 0.05% Brij35) in an Econo-pack column (BIO-RAD). The bound TNIK_N was eluted with elution buffer (50 mM Tris-HCl, pH 8.0, 150 mM NaCl, 1 mM DTT, 10% glycerol, 0.5 mM EDTA, 0.5 mM EGTA and 5 mM reduced glutathione). The eluted fractions were collected and determined the protein concentration by Bradford reagent (BIO-RAD). The TNIK_N fractions were pooled and desalted using 10DG column (BIORAD) equilibrated with the storage buffer (50 mM Tris-HCl, pH 7.5, 150 mM NaCl, 1 mM DTT, 10% glycerol, 0.05% Brij35). The purified TNIK_N was characterized by electrophoresis using 4-20% polyacrylamide gels and matrix-assisted laser desorption/ionization reflection time-of-flight (MALDI-TOF) mass spectrometry on a Voyager-DE RP MALDI/TOF (Applied Biosystems). TNIK_N was confirmed by the molecular weight and MASCOT Peptide Mass Fingerprint.

### Kinase assay:

The kinase assays were conducted in a 20 µl volume using 384-well plates (Greiner). The reaction mixture consists of compound or vehicle (1% DMSO), 0.08 ng/µl TNIK_N, I µM FITC-labeled substrate peptides, FITC-x-Lys-Tyr-Lys-Thr-Leu-Arg-Gln ([SEQ ID NO.3], x: ε-aminocaproic acid), 20 mM Hepes, pH 7.5, 0.01% Triton X-100, 5 mM MgCl₂, 25 µM ATP and 2 mM DTT. As blank, TNIK_N was excluded from the reaction mixture of vehicle (1% DMSO). The kinase reaction was carried out I h at room temperature and terminated by addition of 60 µl of the termination buffer (127 mM Hepes, pH 7.5, 26.7 mM EDTA, 0.01% Triton X-100, 1% DMSO and 0.13% Coating Reagent 3 (Caliper Life Sciences)). The amount of unphosphorylated and phosphorylated FITC-labeled substrate peptides was detected by Mobility Shift Micro Fluidic Technology (Caliper LC3000 System, Caliper Life Sciences). The kinase activity of TNIK_N was defined as P/(P+S) (P: peak height of the phosphorylated FITC-labeled substrate peptide; S: peak height of the FITC-labeled substrate peptide). Inhibition of the compounds was calculated as follows; inhibition (%) = (1-(A-C)/(B-C)) x 100 A: the mean P/(P+S) of compound wells; B: the mean P/(P+S) of vehicle wells; C: the mean P/(P+S) of blank wells. The IC50 values of the compound against the kinases were calculated from regression analysis of the log-concentration-inhibition curves.

### Result:

The test results are shown in Table 2.

**Table 2**

| NO. | Test Compound | IC50 (µM) |
|---|---|---|
| 1 | 5-(4-acetamidobenzamido)-2-(phenylamino) thiazole-4-carboxamide | 0.009 |
| 2 | 5-(3-methylbenzamido)-2-(phenylamino) thiazole-4-carboxamide | 0.040 |
| 3 | 5-(2-fluorobenzamido)-2-(phenylamino) thiazole-4-carboxamide | 0.019 |
| 4 | 5-(4-methoxybenzamido)-2-(phenylamino) thiazole-4-carboxamide | 0.010 |
| 5 | 5-(3-methoxybenzamido)-2-(phenylamino) thiazole-4-carboxamide | 7.7 |
| 6 | 2-(phenylamino)-5-(2-(thiophen-2-yl)acetamido) thiazole-4-carboxamide | 0.28 |
| 7 | 5-(3-methylbutanamido)-2-(phenylamino) thiazole-4-carboxamide | 0.18 |
| 8 | 5-(2-cyclopentylacetamido)-2-(phenylamino) thiazole-4-carboxamide | 0.13 |
| 9 | 2-(p-toluidino)-5-(2-fluorobenzamido) thiazole-4-carboxamide | 0.068 |
| 10 | 2-(p-toluidino)-5-(4-acetamidobenzamido) thiazole-4-carboxamide | 0.014 |
| 11 | 2-(p-toluidino)-5-(2-chlorobenzamido) thiazole-4-carboxamide | 1.7 |
| 12 | 2-(p-toluidino)-5-(3-bromobenzamido) thiazole-4-carboxamide | 1.7 |
| 13 | 2-(p-toluidino)-5-(2,6-difluorobenzamido) thiazole-4-carboxamide | 0.18 |
| 14 | 2-(p-toluidino)-5-(3,4-dimethoxybenzamido) thiazole-4-carboxamide | 0.057 |
| 15 | 2-(p-toluidino)-5-(thiophene-2-carboxamido) thiazole-4-carboxamide | 0.031 |
| 16 | 2-(p-toluidino)-5-(2-ethylbutanamido) thiazole-4-carboxamide | 4.1 |
| 17 | 2-(ethylamino)-5-(8-methyl-2-phenylquinoline -4-carboxamido)thiazole-4-carboxamide | 3.1 |
| A1 | 5-(4-methylbenzamido)-2-(phenylamino)thiazole-4-carbox amide | 0.006 |
| A2 | 5-(4-fluorobenzamido)-2-(phenylamino)thiazole-4-carboxa mide | 0.017 |
| A4 | 5-(4-acetamidobenzamido)-2-(4-fluorophenylamino)thiazol e-4-carboxamide | 0.018 |
| A9 | 2-(o-toluidino)-5-(4-acetamidobenzamido)thiazole-4-carbo xamide | 0.018 |
| A11 | 5-(4-acetamidobenzamido)-2-(4-methoxyphenylamino)thia zole-4-carboxam ide | 0.014 |
| A19 | 5-(4-acetamidobenzamido)-2-(4-aminophenylamino)thiazo le-4-carboxamide | 0.024 |
| A20 | 5-(4-acetamidobenzamido)-2-(pyridin-3-ylamino)thiazole-4-carboxamide | 0.009 |
| A22 | 2-(4-methoxyphenylamino)-5-[4-(4-methylpiperazin-1-yl)b enzamido]thiazole-4-carboxamide | 0.015 |
| A23 | 5-[4-(2-hydroxyethylamino)benzamido]-2-(4-methoxyphen ylamino)thiazole-4-carboxamide | 0.008 |
| A24 | 5-{4-[2-(dimethylamino)ethylamino]benzamido}-2-(4-met hoxyphenylamino)thiazole-4-carboxamide | 0.011 |
| A27 | 2-(4-methoxyphenylamino)-5-[4-(2-morpholinoethylamino )benzamido]thiazole-4-carboxamide | 0.009 |
| A29 | 2-(4-methoxyphenylamino)-5-{4-[2-(pyrrolidin-1-yl)ethyla mino]benzamido}thiazole-4-carboxamide | 0.008 |
| A31 | 5-[4-(2-methoxyethylamino)benzamido]-2-(4-methoxyphe nylamino)thiazole-4-carboxamide | 0.010 |
| A37 | 5-[4-(2-hydroxyacetamido)benzamido]-2-(4-methoxyphen ylamino)thiazole-4-carboxamide | 0.009 |
| A42 | 2-(4-methoxyphenylamino)-5-{4-[2-(4-methylpiperazin-1-yl)acetamido]benzamido}thiazole-4-carboxamide | 0.018 |
| A43 | 5-{4-[4-(hydroxymethyl)piperidin-1-yl]benzamido}-2-(4-methoxyphenylamino)thiazole-4-carboxamide | 0.013 |
| A44 | 2-(4-methoxyphenylamino)-5-{4-[(4-methylpiperazin-1-yl) methyl]benzamido}thiazole-4-carboxamide | 0.011 |
| A45 | 2-(4-methoxyphenylamino)-5-{4-[2-(pyrrolidin-1-yl)ethox y]benzamido}thiazole-4-carboxamide | 0.022 |
| A46 | 5-(4-methoxybenzamido)-2-(4-methoxyphenylamino)thiaz ole-4-carboxamide | 0.016 |
| A47 | 5-(4-methoxybenzamido)-2-(pyridin-4-ylamino)thiazole-4-carboxamide | 0.003 |
| A48 | 2-(4-methoxyphenylamino)-5-{4-[N-(methylsulfonyl)meth ylsulfonamido]benzamido}thiazole-4-carboxamide | 0.006 |
| A49 | 2-(4-methoxyphenylamino)-5-{4-[2-(piperidin-1-yl)ethyla mino]benzamido} thiazole-4-carboxamide | 0.005 |
| A55 | 2-(4-methoxyphenylamino)-5-{4-[2-(pyridin-4-yl)ethylami no]benzamido}thiazole-4-carboxamide | 0.010 |
| A69 | 5-(4-methoxybenzamido)-2-(pyrimidin-5-ylamino)thiazole -4-carboxamide | 0.007 |
| A72 | 5-[4-(2-hydroxyethylamino)benzamido]-2-(pyridin-3-ylami no)thiazole-4-carboxamide | 0.004 |
| A74 | 5-[4-(4-methylpiperazin-1-yl)benzamido]-2-(pyridin-3-yla m i no)th iazo le-4-carboxam ide | 0.007 |
| A76 | 5-(4-fluorobenzamido)-2-(pyridin-4-ylamino)thiazole-4-car boxamide | 0.007 |
| A84 | 2-(pyridin-3-ylamino)-5-{4-[2-(pyridin-4-yl)ethylamino]be nzamido}thiazole-4-carboxamide | 0.002 |
| A92 | 2-(2-fluoropyridin-4-ylamino)-5-(4-methoxybenzamido)thi azole-4-carboxamide | 0.003 |
| A102 | 2-[4-(hydroxymethyl)phenylamino]-5-(4-methoxybenzami do)thiazole-4-carboxamide | 0.006 |
| A103 | 5-(3,4-diaminobenzamido)-2-(4-methoxyphenylamino)thia zole-4-carboxamide | 0.010 |

### TEST EXAMPLE 2

### Selectivity Profiling Test:

The inhibitory effects of Compound I against 20 tyrosine kinases and 30 serine/threonine kinases were investigated using QuickScout^{™} TK and STK Screening Panel (Carna Biosciences, Kobe Japan). The IC50 values of Compound I are showed in Table 3. The results reveal that Compound I inhibits TNIK_N more potently (IC50 ; 9 nM) than the other 50 kinases.

**Table 3 Selectivity Profiling of Compound 1**

| Tyrosine kinases | IC50 (nM) | Serine/threonine kinases | IC50 (nM) |
|---|---|---|---|
| KDR | 59 | MLKI | 18 |
| EGFR | 69 | GSK3b | 120 |
| FLT3 | 95 | AurA | 130 |
| PDGFRa | 150 | CaMK4 | >300 |
| ABL | 290 | CDK2 | >300 |
| FGFR1 | >300 | CHK1 | >300 |
| EphA2 | >300 | CK1e | >300 |
| IGF1R | >300 | DAPKI | >300 |
| ITK | >300 | DYRK1B | >300 |
| JAK3 | >300 | Erk2 | >300 |
| CSK | >300 | AKT1 | >300 |
| LCK | >300 | IKKb | >300 |
| MET | >300 | IRAK4 | >300 |
| EphB4 | >300 | MAPKAPK2 | >300 |
| PYK2 | >300 | MSTI | >300 |
| SRC | >300 | NEK2 | >300 |
| SYK | >300 | p38a | >300 |
| TIE2 | >300 | p70S6K | >300 |
| TRKA | >300 | PAK4 | >300 |
| TYR03 | >300 | PDK1 | >300 |
| | | PIM1 | >300 |
| | | PKACa | >300 |
| | | PKCa | >300 |
| | | PKD2 | >300 |
| | | ROCK1 | >300 |
| | | SGK | >300 |
| | | JNK2 | >300 |
| | | MAP2K1 | >300 |
| | | AMPKa1/b1/g1 | >300 |
| | | RAF1 | >300 |

### TEST EXAMPLE 3

### TCF/Lymphoid enhancer factor (LEF) reporter gene assay:

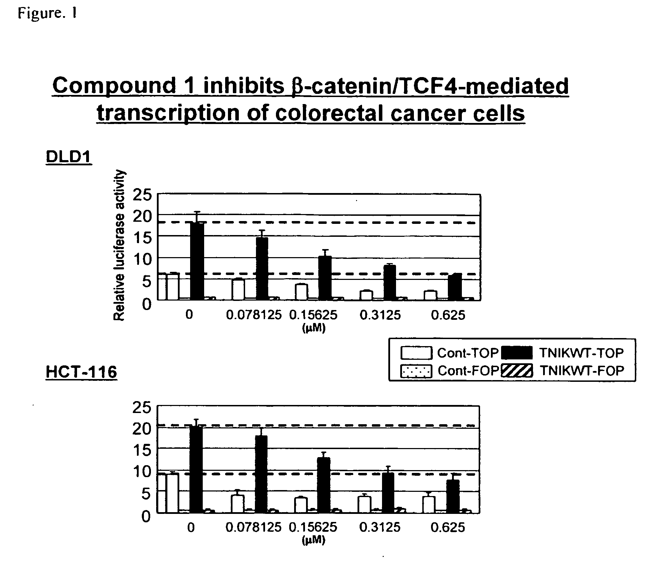

Human colorectal cancer cell lines DLD-1 and HCT-116 were obtained from Health Science Research Resources Bank and American Type Culture Collection, respectively. Full length human TNIK inserted into pCIneo-HA vector (Promega) was kindly gifted from Dr. Kenichi Kariya (Ryukyu University). DLD1 and HCT-116 cells were co-transfected in triplicate with canonical (TOP-FLASH) or mutant (FOP-FLASH) TCF/LEF luciferase reporter, phRL-TK (Promega) (an internal standard), and pCIneo-HA-TNIK or pCIneo-HA (control plasmid). Twenty-four hours after transfection, compound 1 or vehicle were added to the cells at final concentration of 0.078125, 0.15625, 0.3125, and 0.625 µM. After subsequent 24 h, reporter activity was assayed by using the Dual-Luciferase Reporter Assay System (Promega) according to the instruction manuals. The test results are shown in Fig. 1. Results were normalized to *Renilla* values of each sample. The reporter assay results represent the average and standard deviation of triplicate assays. Compound I inhibited β-catenin/TCF4-mediated transcription in DLD1 and HCT-116 in a concentration-dependent manner.

### EXAMPLE

The following examples are illustrative only, and not intended to limit the scope of the limit the present invention.

Abbreviations and symbols used in the following descriptions mean as follows:
CDCl₃: chloroform-d
D₂O: deuterium oxide
DCM: dichloromethane
DMA: dimethylacetamide
DMF: dimethyl formamide
DMSO: dimethyl sulfoxide
EtOH: ethanol
EtOAc: ethyl acetate
HCl: hydrochloric acid
K₂CO₃: potassium carbonate
MeOH: methanol
MgSO₄: magnesium sulfate
NaHCO₃: sodium bicarbonate
Na₂SO₄: sodium sulfate
NH₄Cl: ammonium chloride
NH₃: ammonia
N₂: nitrogen
POCl₃: phosphorous oxychloride
THF: tetrahydrofuran
TFA: trifluoroacetic acid
Xantphos: 4,5-Bis(diphenylphosphino)-9,9-dimethylxanthene
EDC: 1-ethyl-3-(3-dimethylaminopropyl) carbodiimide hydrochloride
HOBT: hydroxybenzotriazole
min.: minute(s)
h or hr(s): hour(s)
RT or rt: room temperature
sat.: saturated
aq.: aqueous
TLC: thin layer chromatography
HPLC: high performance liquid chromatography
Prep HPLC: preparative HPLC
LCMS: high performance liquid chromatography/mass spectrometry
MS: mass spectrometry
NMR: nuclear magnetic resonance

### Example 1-3, 5-16, 20, 26, 34, 36, 46, 52, 54, 61-63, 65, 66, 81 and 121

Each of the Examples shown in the following Table 4 were synthesized according to the procedure described in the following Example 4 using appropriate starting materials.

**Table 4.**

| Ex. No. | ¹H-NMR δ (ppm) | LCMS m/z [M+H]⁺ |
|---|---|---|
| 1 | (DMSO-d₆): 2.41 (s, 3H), 6.95 (t, 1H, J = 7.6 Hz), 7.30 (dd, 2H, J = 8.4, 7.6 Hz), 7.43 (d, 2H, J = 8.0 Hz), 7.65-7.75 (m, 3H), 7.75-7.9 (m, 3H), 10.06 (s, 1H), 12.59 (s, 1H). | 352.9 |
| 2 | (DMSO-d₆): 6.95 (t, 1H, J = 7.2 Hz), 7.30 (dd, 2H, J = 8.4, 7.2 Hz), 7.47 (t, 2H, J = 8.8 Hz), 7.7-7.8 (m, 3H), 7.87 (br, 1H), 7.97 (dd, 2H, J = 8.4, 5.2 Hz), 10.09 (s, 1H), 12.63 (s, 1H). | 356.9 |
| 3 | (DMSO-d₆): 6.95 (t, 1H, J = 7.6 Hz), 7.30 (dd, 2H, J = 8.8, 7.6 Hz), 7.71 (d, 2H, J = 8.8 Hz), 7.72-7.8 (m, 3H), 7.91 (d, 2H, J = 8.8 Hz), 10.10 (s, 1H), 12.67 (s, 1H). | 372.8 |
| 5 | (DMSO-d₆): 2.09 (s, 3H), 7.25 (d, 2H J = 8.6 Hz), 7.7-7.9 (m, 8H), 10.28 (s, 1H), 10.33 (s, 1H), 12.54 (s, 1H). | 480.0 |
| 6 | (DMSO-d₆): 2.09 (s, 3H), 2.31 (s, 3H), 6.76 (d, 1H, J = 7.4 Hz), 7.18 (t, 1H, J = 7.8 Hz), 7.35 (s, 1H), 7.6-7.7 (m, 2H), 7.7-7.9 (m, 5H), 9.95 (s, 1H), 10.33 (s, 1H), 12.53 (s, 1H). | 410.2 |
| 7 | (DMSO-d₆): 2.10 (s, 3H), 7.60 (d, 2H, J = 8.5 Hz), 7.7-7.9 (m, 6H), 7.95 (d, 2H, J = 8.6 Hz), 10.33 (s, 1H), 10.50 (s, 1H), 12.56 (s, 1H). | 464.4 |
| 8 | (DMSO-d₆): 2.09 (s, 3H), 2.84 (s, 6H), 6.73 (d, 2H, J = 8.4 Hz), 7.5-7.6 (m, 2H), 7.7-7.9 (m, 4H), 9.65 (s, 1H), 10.32 (s, 1H), 12.45 (s, 1H). | 439.0 |
| 9 | (DMSO-d₆): 2.09 (s, 3H), 2.28 (s, 3H), 6.97 (t, 1H, J = 7.3 Hz), 7.1-7.2 (m, 2H), 7.42 (s, 1H), 7.7-7.9 (m, 5H), 8.08 (d, 1H, J = 8.3 Hz), 9.04 (s, 1H), 10.31 (s, 1H), 12.46 (s, 1H). | 410.4 |
| 10 | (DMSO-d₆): 2.08 (s, 3H), 2.23 (s, 6H), 7.1-7.2 (m, 4H), 7.7-7.8 (m, 5H), 8.89 (s, 1H), 10.30 (s, 1H), 12.34 (s, 1H). | 424.2 |
| 11 | (DMSO-d₆): 2.09 (s, 3H), 3.72 (s, 3H), 6.87 (d, 2H, J = 8.7 Hz), 7.6-7.7 (m, 3H), 7.7-7.9 (m, 5H), 9.87 (s, 1H), 10.34 (s, 1H), 12.51 (s, 1H). | 426.4 |
| 12 | (DMSO-d₆): 6.95 (t, 1H, J = 7.2 Hz), 7.30 (dd, 2H, J = 8.4, 7.6 Hz), 7.63 (d, 2H, J = 8.0 Hz), 7.7-7.8 (m, 3H), 7.89 (br, 1H), 8.03 (d, 2H, J = 8.8 Hz), 10.11 (s, 1H), 12.68 (s, 1H). | 422.9 |
| 13 | (DMSO-d₆): 2.09 (s, 3H), 7.30 (d, 2H, J = 8.7 Hz), 7.7-7.9 (m, 8H), 10.22 (s, 1H), 10.34 (s, 1H), 12.55 (s, 1H). | 428.2 [M-H]⁺ |
| 14 | (DMSO-d₆): 2.09 (s, 3H), 2.23 (s, 3H), 2.25 (s, 3H), 7.00-7.1 (m, 2H), 7.37 (s, 1H), 7.7-7.9 (m, 6H), 8.99 (s, 1H), 10.33 (s, 1H), 12.44 (s, 1H). | 424.0 |
| 15 | (DMSO-d₆): 2.09 (s, 3H), 2.16 (s, 3H), 2.27 (s, 3H), 6.96 (d, 1H, J = 7.4 Hz), 7.09 (t, 1H, J = 7.7 Hz), 7.33 (s, 1H), 7.67 (d, 1H, J = 7.9 Hz), 7.7-7.9 (m, 5H), 9.08 (s, 1H), 10.34 (s, 1H), 12.42 (s, 1H). | 424.6 |
| 16 | (DMSO-d₆): 3.86 (s, 3H), 6.94 (t, 1H, J = 7.3 Hz), 7.16 (d, 2H, J = 8.4 Hz), 7.30 (t, 2H, J = 7.6 Hz), 7.68 (br, 1H), 7.73 (d, 2H, J = 8.0 Hz), 7.80 (br, 1H), 7.86 (d, 2H, J = 7.7 Hz), 10.03 (s, 1H), 12.5 (s, 1H). | 368.8 |
| 20 | (DMSO-d₆): 2.13 (s, 3H), 7.3-7.4 (m, 1H), 7.7-7.9 (m, 6H), 8.15 (br, 1H), 8.4-8.5 (m, 1H), 8.70 (br, 1H), 10.30 (s, 1H), 10.33 (s, 1H), 12.57 (S, 1H). | 395.3 [M-H]⁺ |
| 26 | (DMSO-d₆): 3.02 (s, 6H), 3.72 (s, 3H), 6.83 (d, 2H, J = 8.8 Hz), 6.87 (d, 2H, J = 8.8 Hz), 7.55 (s, 1H), 7.64 (d, 2H, J = 8.9 Hz), 7.7-7.8 (m, 3H), 9.79 (s, 1H), 12.33 (s, 1H). | 412.4 |
| 34 | (DMSO-d₆): 3.73 (s, 3H), 5.22 (s, 2H), 6.88 (d, 2H, J = 8.8 Hz), 7.24 (d, 2H, 8.8 Hz), 7.3-7.55 (m, 5H), 7.63 (br, 1H), 7.66 (d, 2H, J = 9.2 Hz), 7.80 (br, 1H), 7.86 (d, 2H, J = 8.8 Hz), 9.86 (s, 1H), 12.50 (s, 1H). | 474.9 |
| 36 | (DMSO-d₆): 2.35-2.6 (m, 4H), 2.72 (t, 2H, J = 5.6 Hz), 3.5-3.6 (m, 4H), 3.73 (s, 3H), 4.20 (t, 2H, J = 5.6 Hz), 6.88 (d, 2H, J = 8.8 Hz), 7.16 (d, 2H, J = 9.2 Hz), 7.63 (br, 1H), 7.66 (d, 2H, J = 9.2 Hz), 7.80 (br, 1H), 7.84 (d, 2H, J = 8.8 Hz), 9.86 (s, 1H), 12.49 (s, 1H). | 498.4 |
| 46 | (DMSO-d₆): 3.72 (s, 3H), 3.86 (s, 3H), 6.88 (d, 2H, J = 8.7 Hz), 7.15 (d, 2H, J = 8.6 Hz), 7.60 (s, 1H), 7.65 (d, 2H, J = 8.6 Hz), 7.78 (s, 1H), 7.85 (d, 2H, J = 8.5 Hz), 9.84 (s, 1H), 12.48 (s, 1H). | 399.2 |
| 52 | (DMSO-d₆,): 3.86 (s, 3H), 7.09 (t, 1H, J= 8.8 Hz), 7.14 (d, 1H, J = 8.76 Hz), 7.71-7.81 (m, 4H), 7.87 (d, 1H, J = 8.72 Hz), 10.08 (s, 1H), 12.51 (s, 1H). | 387.1 |
| 54 | (DMSO-d₆): 1.5-1.7 (m, 2H), 1.85-2.1 (m, 2H), 3.4-3.6 (m, 2H), 3.73 (s, 3H), 3.8-4.0 (m, 2H), 4.73 (t, 1H, J = 4.0 Hz), 6.88 (d, 2H, J = 9.2 Hz), 7.19 (d, 2H, J = 8.8 Hz), 7.62 (br, 1H), 7.65 (d, 2H, J = 8.8 Hz), 7.79 (br, 1H), 7.84 (d, 2H, J = 8.8 Hz), 9.85 (s, 1H), 12.48 (s, 1H). | 468.9 |
| 61 | (DMSO-d₆): 2.33 (s, 3H), 3.72 (s, 3H), 6.88 (d, 2H, J = 8.8 Hz), 7.5-7.7 (m, 6H), 7.85 (s, 1H), 9.90 (s, 1H), 12.59 (s, 1H). | 401.4 |
| 62 | (DMSO-d₆): 2.23 (s, 3H), 3.72 (s, 3H), 3.88 (s, 3H), 6.88 (d, 2H, J = 8.5 Hz), 7.3-7.4 (m, 2H), 7.43 (s, 1H), 7.6-7.7 (m,3H), 7.82 (s, 1H), 9.88 (s, 1H), 12.59 (s, 1H). | 413.3 |
| 63 | (DMSO-d₆): 2.62 (s, 3H), 3.72 (s, 3H), 6.88 (d, 2H, J = 8.9 Hz), 7.6-7.7 (m, 3H), 7.75 (d, 1H, J = 8.0 Hz), 7.86 (s, 1H), 8.07 (d, 1H, J = 8.2 Hz), 8.43 (s, 1H), 9.90 (s, 1H), 12.72 (s, 1H). | 428.4 |
| 65 | (DMSO-d₆): 2.32 (s, 6H), 3.72 (s, 3H), 6.88 (d, 2H, J = 8.7 Hz), 7.37 (d, 1H, J = 7.8 Hz), 7.6-7.7 (m, 5H), 7.80 (s, 1H), 9.86 (s, 1H), 12.52 (s, 1H). | 397.4 |
| 66 | (DMSO-d₆): 2.40 (s, 3H), 3.72 (s, 3H), 6.88 (d, 2H, J = 8.8 Hz), 7.23 (d, 1H, J = 8.04 Hz), 7.30 (d, 1H, J = 13.0 Hz), 7.55 (s, 1H), 7.64 (d, 2H, J = 8.7 Hz), 7.72 (s, 1H), 7.92 (t, 1H, J = 8.1 Hz), 9.86 (s, 1H), 12.50 (d, 1H, J = 10.1 Hz). | 401.2 |
| 81 | (DMSO-d₆): 3.73 (s, 3H), 6.89 (d, 2H, J = 8.8 Hz), 7.66 (d, 2H, J = 8.8 Hz), 7.72 (br, 1H), 7.85 (dd, 1H, J = 11.2, 8.8 Hz), 7.91 (br, I H), 8.2-8.3 (m, 1H), 8.59 (dd, 1H, J = 6.8, 2.4 Hz), 9.94 (s, 1H), 12.76 (s, 1H). | 430.0 [M-H]⁺ |
| 121 | (DMSO-d₆): 2.4-2.55 (m, 4H), 2.73 (t, 2H, J = 5.6 Hz), 3.5-3.65 (m, 4H), 4.21 (t, 2H, J = 5.6 Hz), 7.17 (d, 2H, J = 8.8 Hz), 7.44 (d, 1H, J = 5.6 Hz), 7.53 (d, 1H, J = 1.2 Hz), 7.8-7.9 (m, 3H), 8.00 (d, 1H, J = 5.6 Hz), 8.02 (br, 1H), 10.82 (s, 1H), 12.64 (s, 1H). | 487.0 |

### Example 4

### 5-(4-acetamidobenzamido)-2-(4-fluorophenylamino)thiazole-4-carboxamide

### (a) 5-amino-2-(4-fluorophenylamino)thiazole-4-carboxamide:

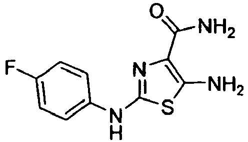

To a suspension of 2-amino-2-cyanoacetamide (0.25 g, 2.5 mmol) in EtOAc (7 mL) was added 4-fluorophenyl isothiocyanate (0.386 g, 2.5 mmol), and the mixture was refluxed for 30 min. The solvent was evaporated and the residue was purified by silica gel column chromatography eluted with 2% MeOH in DCM to give 0.4 g (52% yield) of the titled compound.
¹H-NMR (400 MHz, DMSO-d₆) δ (ppm) 6.65 (s, 2H), 6.89 (br, 2H), 7.04 (t, 2H J = 8.7 Hz), 7.6-7.7 (m, 2H), 9.56 (s, 1H). LCMS m/z [M+H]⁺ 253.0.

### (b) 5-(4-acetamidobenzamido)-2-(4-fluorophenylamino)thiazole-4-carboxamide

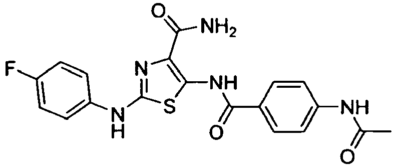

To a mixture of 4-acetamidobenzoic acid (0.106 g, 0.59 mmol) and catalytic amount of DMF in dry THF (5 mL) was added dropwise oxalyl chloride (0.06 mL, 0.79 mmol) at 0 °C, and the mixture was stirred for 2 h at rt. The solvent was evaporated, and the residual oxalyl chloride was removed with azeotropic distillation using toluene under nitrogen atmosphere. The resulting acid chloride was then dissolved in pyridine (5 mL) and cooled to 0 °C. To this solution, a solution of 5-amino-2-(4-fluorophenylamino)thiazole-4-carboxamide (0.1 g, 0.39 mmol) in pyridine (5 mL) was added at 0 °C, and the mixture was stirred for 12 h at rt. The solvent was evaporated, and the residue was suspended into 1M HCl, and the resulting solids were collected. The solids were washed with water (10 mL), ether (20 mL) and dried. The crude solids were purified by silica gel column chromatography eluted with 3% MeOH in DCM to give 38 mg (10% yield) of the titled compound.
¹H-NMR (400 MHz, DMSO-d₆) δ (ppm) 2.09 (s, 3H), 7.01 (t, 2H, J = 8.7 Hz), 7.7-7.9 (m, 8H), 10.09 (s, I H), 10.32 (s, I H), 12.5 (s, 1H). LCMS m/z [M+H]⁺ 414.4.

### Example 17

### 5-(4-acetamidobenzamido)-2-(4-hydroxyphenylamino)thiazole-4-carboxamide:

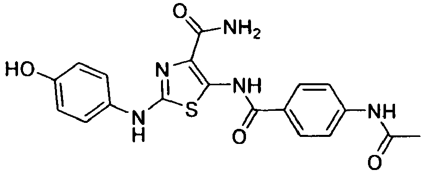

To a solution of 5-(4-acetamidobenzamido)-2-(4-methoxyphenylamino)thiazole-4-carboxamide (100 mg, 0.23 mmol) in 1,2-dichloroethane (10 mL) was added dropwise BBr₃ (587.5 mg, 2.35 mmol) at 0 °C under nitrogen atmosphere, and the mixture was stirred at rt for 5h. The reaction mixture was quenched with 1M HCl (5ml), and the organic layer was separated and concentrated. The resulting solids were collected, and washed successively with hexane and ether, and dried to give 70 mg (72% yield) of the titled compound. ¹H-NMR (400 MHz, DMSO-d₆) δ (ppm) 2.09 (s, 3H), 6.71 (d, 2H, J = 8.6 Hz), 7.51 (d, 2H, J = 8.6 Hz), 7.58 (br, 1H), 7.7-7.9 (m, 5H), 9.71 (s, 1H), 10.33 (s, 1H), 12.49 (s, I H). LCMS m/z [M+H]⁺ 412.1.

### Example 18

### tert-butyl-4-[5-(4-acetamidobenzamido)-4-carbamoylthiazol-2-ylamino]phenylcarb amate:

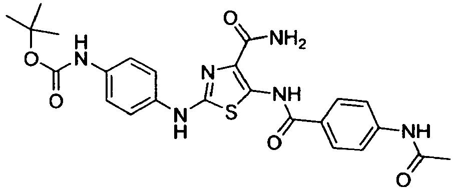

### (a) tert-butyl-4-isothiocyanatophenylcarbamate

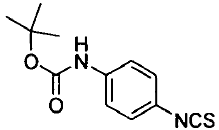

To a mixture of *tert*-butyl-4-aminophenylcarbamate (0.5 g, 2.4 mmol) and triethylamine (0.98 mL, 7.2 mmol) in THF (45 mL) was added dropwise thiophosgene (0.2 mL, 2.64 mmol) at 0 °C, and the mixture was stirred at rt for 30 min. The reaction mixture was quenched with water and extracted with ether (2 x 30 mL). The organic layer was dried over Na₂SO₄ and concentrated to give 0.5 g (83% yield) of the titled compound, which was used for next step without further purification.
¹H-NMR (400 MHz, CDCl₃) δ (ppm) 1.50 (s, 9H), 6.50 (s, 1H), 7.14 (d, 2H, J = 8.6 Hz), 7.34 (d, 2H, J = 8.4 Hz). LCMS m/z [M+H]⁺ 251.2.

### (b) tert-butyl-4-(5-amino-4-carbamoylthiazol-2-ylamino)phenylcarbamate

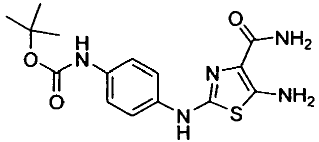

To a suspension of 2-amino-2-cyanoacetamide (0.178 g, 1.8 mmol) in EtOAc (10 mL) was added *tert*-butyl-4-isothiocyanatophenylcarbamate (0.5 g, 2.0 mmol), and the mixture was refluxed for 30 min. The solvent was evaporated, and the residue was purified by silica gel column chromatography eluted with 2% MeOH in DCM to give 0.4 g (57% yield) of the titled compound.
¹H-NMR (400 MHz, DMSO-d₆) δ (ppm) 1.46 (s, 9H), 6.62 (s, 2H), 6.88 (s, 2H), 7.3-7.4 (m, 2H), 7.46 (d, 2H, J = 8.8 Hz), 9.12 (s, 1H), 9.39 (s, 1H). LCMS m/z [M+H]⁺ 350.3.

### (c)tert-butyl-4-[5-(4-acetamidobenzamido)-4-carbamoylthiazol-2-ylamino]phenylca rbamate

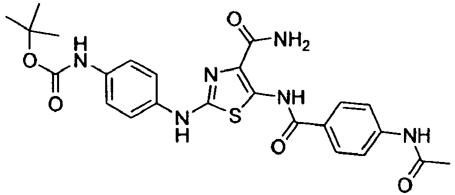

To a mixture of 4-acetamidobenzoic acid (0.256 g, 1.43 mmol) and catalytic amount of THF in dry THF (15 mL) was added dropwise oxalyl chloride (0.25 mL, 2.86 mmol) at 0 °C, and the mixture was stirred for 2 h at rt. The solvent was evaporated, and the residual oxalyl chloride was removed with azeotropic distillation using toluene under nitrogen atmosphere. The resulting acid chloride was then dissolved in pyridine (10 mL) and cooled to 0 °C. To this solution, a solution of *tert*-butyl-4-(5-amino-4-carbamoylthiazol-2-ylamino)phenylcarbamate (0.4 g, 1.14 mmol) in pyridine (5 mL) was added at 0 °C, and the mixture was stirred for 12 h at rt. The solvent was evaporated, and the residue was suspended into 1M HCl, and the resulting solids were collected. The solids were washed with water (10 mL), ether (20 mL) and dried. The crude solids were purified by silica gel column chromatography eluted with 2-5% MeOH in DCM to give 125 mg (21% yield) of the titled compound. ¹H-NMR (400 MHz, DMSO-d₆) δ (ppm) 1.47 (s, 9H), 2.09 (s, 3H), 7.3-7.4 (m, 2H), 7.62 (d, 2H, J = 8.5 Hz), 7.72 (s, 1H), 7.8-7.9 (m, 5H), 9.20 (br, 1H), 9.92 (s, 1H), 10.33 (s, 1H), 12.54 (s, I H). LCMS m/z [M+H]⁺ 511.3.

### Example 68-69, 71, 73, 76, 82, 92, 95-98, 100, 102 and 104

The compounds shown in the following Table 5 were prepared by following the procedure described for above Example 18 using appropriate starting materials.

**Table 5**

| Ex. No. | ¹H-NMR δ (ppm) | LCMS m/z [M+H]⁺ |
|---|---|---|
| 68 | (DMSO-d₆): 3.82 (s, 3H), 3.86 (s, 3H), 6.77 (d, 1H, J = 8.8 Hz), 7,16 (d, 2H, J = 8.8 Hz), 7.78 (br, 1H), 7.86 (d, 2H, J = 8.8 Hz), 8.19 (dd, 1H, J = 8.8, 2.8 Hz), 8.51 (d, 1H, J = 2.8 Hz), 10.03 (s, 1H), 12.55 (s, 1H). | 400.3 |
| 69 | (DMSO-d₆): 3.87 (s, 3H), 7.17 (d, 2H, J = 8.8 Hz), 7.79 (br, I H), 7.88 (d, 2H, J = 8.8 Hz), 8.07 (br, 1H), 8.80 (s, 1H), 9.2 (s, 2H), 10.49 (s, 1H), 12.65 (s, 1H). | 371.0 |
| 71 | (DMSO-d₆): 7.47 (t, 2H, J = 8.6 Hz), 7.8-8.0 (m, 4H), 8.12 (s, 1H), 8.42 (d, 1H, J = 4.8 Hz), 8.78 (d, 1H, J = 8.3 Hz), 9.43 (s, 1H), 11.66 (s, 1H), 12.66 (s, 1H). | 358.2 |
| 73 | (DMSO-d₆): 7.8-7.9 (m, 1H), 8.02 (s, 1H), 8.15 (d, 2H, J = 8.7 Hz), 8.4-8.5 (m, 3H), 8.7-8.8 (m, 1H), 9.34 (s, 1H), 11.36 (s, 1H), 12.84 (s, 1H). | 385.2 |
| 76 | (DMSO-d₆): 7.47 (t, 2H, J = 8.6 Hz), 7.65-7.75 (m, 2H), 7.9-8.0 (m, 4H), 8.36 (s, 2H), 10.57 (s, 1H), 12.86 (s, 1H). | 358.1 |
| 82 | (DMSO-d₆): 3.87 (s, 3H), 3.91 (s, 3H), 7.1-7.2 (m, 3H), 7.40 (d, 1H, J = 5.2 Hz), 7.80 (br, 1H), 7.83-7.95 (m, 3H), 8.00 (d, 1H, J = 6.4 Hz), 10.85 (br, 1H), 12.58 (s, 1H). | 400.3 |
| 92 | (DMSO-d₆): 3.87 (s, 3H), 7.17 (d, 2H, J = 8.8 Hz), 7.44 (d,1H, J = 5.6 Hz), 7.54 (d, 1H, J = 1.6 Hz), 7.85 (br, 1H), 7.88 (d, 2H, J = 8.8 Hz), 8.00 (d, 1H, J = 6.0 Hz), 8.03 (br, 1H), 10.82 (s, 1H), 12.64 (s, 1H). | 388.2 |
| 95 | (DMSO-d₆): 3.86 (s, 3H), 4.01 (s, 1H), 7.15 (d, 2H, J = 7.7 Hz), 7.38 (d, 2H, J = 7.7 Hz), 7.7-7.8 (m, 3H), 7.8-7.9 (m, 3H), 10.28 (s, 1H), 12.54 (s, 1H). | 393.4 |
| 96 | (DMSO-d₆): 3.86 (s, 3H), 5.12 (d, 1H, J = 10.8 Hz), 5.67 (d, 1H, J = 17.6 Hz), 6.68 (dd, 1H, J=17.1, 11.2 Hz), 7.16 (d, 2H, J = 8.8 Hz), 7.40 (d, 2H, J = 7.8 Hz), 7.72 (m, 3H), 7.8 - 7.9 (m, 3H), 10. 15 (s, 1H), 12.54 (s, 1H). | 395.2 |
| 97 | (DMSO-d₆): 3.86 (s, 3H), 6.44 (d, 1H, J = 9.6 Hz), 7.15 (d, 2H, J = 9.2 Hz), 7.60 (br, 1H), 7.64 (dd, 1H, J = 9.6, 2.8 Hz), 7.82 (br, 1H), 7.85 (d, 2H, J = 5.2 Hz), 8.14 (d, 1H, J = 2.8 Hz), 9.71 (s, 1H), 12.44 (s, 1H). | 386.0 |
| 98 | (DMSO-d₆): 3.87 (s, 3H), 7.0-7.1 (m, 1H), 7.16 (d, 2H, J = 8.8 Hz), 7.75-7.95 (m, 4H), 8.45-8.55 (m, 2H), 10.36 (s, 1H), 12.57 (s, 1H). | 388.2 |
| 100 | (DMSO-d₆): 2.6-2.7 (m, 2H), 3.5-3.6 (m, 2H), 3.86 (s., 3H), 4.60 (br, 1H), 7.1-7.2 (m, 4H), 7.6-7.7 (m, 3H), 7.75-7.90 (m, 3H), 9.95 (s, 1H), 12.50 (s, 1H). | 413.2 |
| 102 | (DMSO-d₆): 3.86 (s., 3H), 4.23 (s, 2H), 5.05 (br, 1H), 7.15 (d, 2H, J = 7.7 Hz), 7.24 (d, 2H, J = 8.0 Hz), 7.65-7.70 (m, 3H), 7.80-7.85 (m, 1H), 7.86 (d, 2H, J = 8.5 Hz), 10.01 (s, 1H), 12.52 (s, 1H). | 399.3 |
| 104 | (DMSO-d₆): 3.87 (s, 3H), 7.17 (d, 2H, J = 9.2 Hz), 7.51 (d, 1H, J = 1.6 Hz), 7.8-8.0 (m, 5H), 8.17 (d, 1H, J = 5.6 Hz), 10.82 (s, 1H), 12.60 (s, 1H). | 401.9 [M-H]⁺ |

### Example 19

### 5-(4-acetamidobcnzamido)-2-(4-aminophenylamino)thiazole-4-carboxamide:

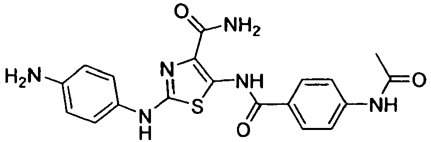

*tert*-butyl-4-[5-(4-acetamidobenzamido)-4-carbamoylthiazol-2-ylamino]phenylcarbamat e (0.10 g, 19 mmol) was dissolved in 4M HCl in 1,4-dioxane (10 mL) at 0 °C under argon atmosphere, and the mixture was stirred at 0 °C for 3 h. The solvent was evaporated, and the residual acid was removed with azeotropic distillation using toluene. The resulting solids were dried to give 78 mg (98% yield) of the titled compound.
¹H-NMR (400 MHz, DMSO-d₆) δ (ppm) 2.07 (s, 3H), 7.29 (d, 2H, J = 8.7 Hz), 7.7-7.9 (m, 8H), 9.93 (br, 2H), 10.36 (s, 1H), 10.38 (s, 1H), 12.56 (s, 1H). LCMS m/z [M+H]⁺ 411.1.

### Example 21

### 5-(4-fluorobenzamido)-2-(4-methoxyphenylamino)thiazole-4-carboxamide:

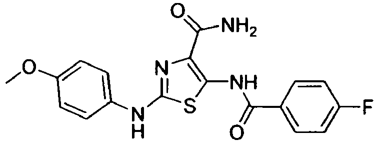

### (a) 5-amino-2-(4-methoxyphenylamino)thiazole-4-carboxamide

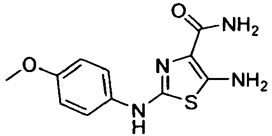

A mixture of 4-methoxyphenylisothiocyanate (1.91 g, 11.53 mmol) and 2-amino-2-cyanoacetamide (1.20 g, 12.11 mmol) in EtOAc (16 mL) was heated at 80° for 50 min. The reaction mixture was cooled to rt, and the resulting solids were filtered off. The filtrate was concentrated, and the residue was purified by silica gel chromatography (eluent: 2% MeOH in CHCl₃ to 5% MeOH in CHCl₃) to afford the titled compound (2.63 g, 86 % yield).
¹H-NMR (400 MHz, DMSO-d₆) δ (ppm) 3.70 (s, 3H), 6.61 (s, 2H), 6.7-7.0 (m, 4H), 7.51 (d, 2H, J = 9.2 Hz), 9.34 (s, 1H).

### (b) 5-(4-fluorobenzamido)-2-(4-methoxyphenylamino)thiazole-4-carboxamide

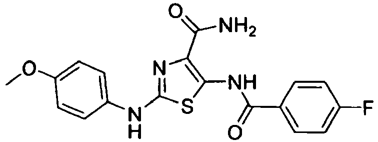

To a solution of 5-amino-2-(4-methoxyphenylamino)thiazole-4-carboxamide (920 mg, 3.48 mmol) in pyridine (15 mL) was added 4-fluorobenzoyl chloride (0.411 mL, 3.48 mmol) at 0°C, the mixture was allowed to warm to rt and stirred for I h at rt. The reaction mixture was diluted with EtOAc, and the organic layer was washed successively with water (x 2) and brine. The organic layer was dried over Na₂SO₄ and evaporated. The resulting solids were collected and washed with 50% hexane in EtOAc to afford the titled compound (1.04 g, 77% yield).
¹H-NMR (400 MHz, DMSO-d₆) δ (ppm) 3.73 (s, 3H), 6.88 (d, 2H, J = 9.2 Hz), 7.46 (t, 2H, J = 8.8 Hz), 7.6-7.75 (m, 3H), 7.84 (br, 1H), 7.96 (dd, 2H, J = 8.8, 5.2 Hz), 9.89 (s, 1H), 12.58 (s, I H). LCMS m/z [M+H]⁺ 386.9.

### Example 59, 105, 112 and 115

The compounds shown in the following Table 6 were prepared by following the procedure described for above Example 21 using appropriate starting materials.

**Table 6**

| Ex. No. | ¹H-NMR δ (ppm) | LCMS m/z [M+H]⁺ |
|---|---|---|
| 59 | (DMSO-d₆): 2.48 (s, 3H), 6.95 (t, 1H, J = 7.2 Hz), 7.30 (dd, 2H, J = 8.4, 7.6 Hz), 7.35-7.45 (m, 2H), 7.49 (td, 1H, J = 7.6, 1.2 Hz), 7.62 (dd, 1H, J = 7.6, 1.6 Hz), 7.68 (br, 1H), 7.74 (d, 2H, J = 7.6 Hz), 7.80 (br, 1H), 10.07 (s, 1H), 12.03 (s, 1H). | 353.0 |
| 105 | (DMSO-d₆): 7.4-7.52 (m, 3H), 7.54 (d, 1H, J = 1.6 Hz), 7.89 (br, 1H), 7.95-8.05 (m, 3H), 8.07 (br, 1H), 10.86 (s, 1H), 12.73 (s, 1H). | 375.8 |
| 112 | (DMSO-d₆): 2.63 (s, 3H), 7.44 (d, 1H, J = 5.6 Hz), 7.54 (d, 1H, J = 2.0 Hz), 7.77 (d, 1H, J = 8.4 Hz), 7.93 (br, 1H), 8.00 (d, 1H, J = 5.6 Hz), 8.05-8.15 (m, 2H), 8.46 (d, 1H, J = 2.0 Hz), 10.88 (s, 1H), 12.88 (s, 1H). | 416.9 |
| 115 | (DMSO-d₆): 7.45 (d, 1H, J=5.8 Hz), 7.56 (s, 1H), 7.9-8.0 (m, 1H), 8.01 (d, 1H, J=5.8 Hz), 8.10-8.1 (m, 1H), 8.1 (d, 2H, J=8.8 Hz), 8.45 (d, 2H, J=8.8 Hz), 10.90 (s, 1H), 12.91 (s, 1H) | 402.9 |

### Example 22

### 2-(4-methoxyphenylamino)-5-[4-(4-methylpiperazin-1-yl)benzamido]thiazole-4-car boxamide:

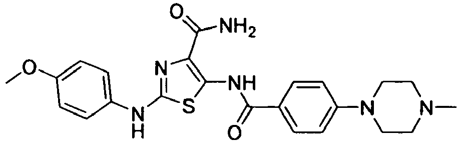

A mixture of 5-(4-fluorobenzamido)-2-(4-methoxyphenylamino)thiazole-4-carboxamide (50 mg, 0.13 mmol) and 1-methylpiperazine (0.072 mL, 0.65 mmol) in N-methylpyrrolidone (0.6 mL) was treated using a microwave synthesizer for 40 min (CEM corp, 180°C). The reaction mixture was concentrated and the residue was purified by silica gel chromatography (eluent: CHCl₃ to 12% MeOH in CHCl₃) to afford the titled compound (38 mg, 63 % yield).
¹H-NMR (400 MHz, DMSO-d₆) δ (ppm) 2.23 (s, 3H), 2.3-2.7 (m, 4H), 3.2-3.5 (m, 4H), 3.72 (s, 3H), 6.88 (d, 2H, J = 9.2 Hz), 7.08 (d, 2H, J = 9.2 Hz), 7.58 (br, 1H), 7.65 (d, 2H, J = 9.2 Hz), 7.73 (d, 2H, J = 8.8 Hz), 7.76 (br, 1H), 9.82 (s, 1H), 12.39 (s, 1H). LCMS m/z [M+H]⁺ 467.0.

### Example 23-24, 27-32, 43, 49, 55-56, 72, 74-75, 77-78, 84, 86-89, 106, 111 and 113

The compounds shown in the following Table 7 were prepared by following the procedure described for above Example 22 using appropriate starting materials.

**Table 7**

| Ex. No. | ¹H-NMR δ (ppm) | LCMS m/z [M+H]⁺ |
|---|---|---|
| 23 | (DMSO-d₆): 3.1-3.3 (m, 2H), 3.57 (q, 2H, J = 5.6 Hz), 3.72 (s, 3H), 4.76 (t, 1H, J = 5.6 Hz), 6.57 (t, 1H, J = 5.6 Hz), 6.71 (d, 2H, J = 8.8 Hz), 6.87 (d, 2H, J = 9.2 Hz), 7.54 (br, 1H), 7.6-7.7 (m, 4H), 7.72 (br, 1H), 9.79 (s, 1H), 12.28 (s, 1H). | 426.0 [M-H]⁺ |
| 24 | (DMSO-d₆): 2.19 (s, 6H), 2.45 (t, 2H, J = 6.4 Hz), 3.19 (q, 2H, J = 6.4 Hz), 3.72 (s, 3H), 6.43 (t, 1H, J = 5.2 Hz), 6.72 (d, 2H, J = 8.8 Hz), 6.88 (d, 2H, J = 8.8 Hz), 7.54 (br, 1H), 7.6-7.7 (m, 4H), 7.72 (br, 1H), 9.79 (s, 1H), 12.28 (s, 1H). | 455.0 |
| 27 | (DMSO-d₆): 2.3-2.6 (m, 6H), 3.23 (q, 2H, J = 6.4 Hz), 3.5-3.7 (m, 4H), 3.72 (s, 3H), 6.46 (t, 1H, J = 5.6 Hz), 6.71 (d, 2H, J = 9.2 Hz), 6.88 (d, 2H, J = 9.2 Hz), 7.54 (br, 1H), 7.6-7.7 (m, 4H), 7.72 (br, 1H), 9.79 (s, 1H), 12.28 (s, 1H). | 497.0 |
| 28 | (DMSO-d₆): 3.2-3.4 (m, 4H), 3.6-3.8 (m, 4H), 6.88 (d, 2H, J = 9.2 Hz), 7.10 (d, 2H, J = 9.2 Hz), 7.59 (br, 1H), 7.65 (d, 2H, J = 9.2 Hz), 7.7-7.8 (m, 3H), 9.83 (s, 1H), 12.41 (s, 1H). | 454.0 |
| 29 | (DMSO-d₆): 1.6-1.8 (m, 4H), 2.4-2.8 (m, 6H), 3.15-3.4 (m, 2H), 3.72 (s, 3H), 6.52 (t, 1H, J = 5.2 Hz), 6.71 (d, 2H, J = 8.8 Hz), 6.88 (d, 2H, J = 9.2 Hz), 7.54 (br, 1H), 7.6-7.7 (m, 4H), 7.72 (br, 1H), 9.79 (s, 1H), 12.28 (s, 1H). | 481.4 |
| 30 | (DMSO-d₆): 2.2-2.8 (m, 10H), 3.54 (q, 2H, J = 6.0 Hz), 3.72 (s, 3H), 4.45 (t, 1H, J = 5.6 Hz), 6.88 (d, 2H, J = 9.2 Hz), 7.08 (d, 2H, J = 9.2 Hz), 7.58 (br, 1H), 7.65 (d, 2H, J = 9.2 Hz), 7.73 (d, 2H, J = 9.2 Hz), 7.76 (br, 1H), 9.82 (s, 1H), 12.3 9 (s, 1H). | 497.0 |
| 31 | (DMSO-d₆): 3.2-3.4 (m, 5H), 3.50 (t, 2H, J = 5.6 Hz), 3.72 (s, 3H), 6.63 (t, 1H, J = 5.6 Hz), 6.72 (d, 2H, J = 8.8 Hz), 6.88 (d, 2H, J = 8.8 Hz), 7.54 (br, 1H), 7.6-7.7 (m, 4H), 7.72 (br, 1H), 9.79 (s, 1H), 12.28 (s, 1H). | 441.9 |
| 32 | (DMSO-d₆): 2.6-2.8 (m, 2H), 3.0-3.5 (m, 4H), 3.72 (s, 3H), 6.58 (br, 1H), 6.69 (d, 2H, J = 8.4 Hz), 6.87 (d, 2H, J = 9.2 Hz), 7.4-7.9 (m, 6H), 9.79 (br, 1H), 12.26 (br, 1H). | 426.9 |
| 43 | (DMSO-d₆): 1.1-1.3 (m, 2H), 1.5-1.8 (m, 3H), 2.75-2.95 (m, 2H), 3.28 (t, 2H, J = 5.6 Hz), 3.38 (s, 3H), 3.9-4.1 (m, 2H), 4.49 (t, 2H, J = 5.6 Hz), 6.88 (d, 2H, J = 9.2 Hz), 7.06 (d, 2H, J = 9.2 Hz), 7.58 (br, 1H), 7.65 (d, 2H, J = 8.8 Hz), 7.71 (d, 2H, J = 8.8 Hz), 7.53 (br, 1H), 9.82 (s, 1H), 12.36 (s, 1H). | 482.0 |
| 49 | (DMSO-d₆): 1.2-1.7 (m, 6H), 2.2-2.7 (m, 6H), 3.1-3.5 (m, 2H), 3.72 (s, 3H), 6.44 (br, 1H), 6.71 (d, 2H, J = 8.4 Hz), 6.87 (d, 2H, J = 9.2 Hz), 7.56 (br, 1H), 7.6-7.7 (br, 1H), 7.6-7.7 (m, 4H), 7.73 (br, 1H), 9.80 (s, 1H), 12.29 (s, 1H). | 495.4 |
| 55 | (DMSO-d₆): 2.89 (dd, 2H, J = 7.6, 6.8 Hz), 3.3-3.5 (m, 2H), 3.72 (s, 3H), 6.65-6.8 (m, 3H), 6.87 (d, 2H, J = 9.2 Hz), 7.32 (d, 2H, J = 6.0 Hz), 7.55 (br, 1H), 7.6-7.7 (m, 4H), 7.72 (br, 1H), 8.48 (d, 2H, J = 6.0 Hz), 9.79 (s, 1H), 12.29 (s, 1H). | 489.4 |
| 56 | (DMSO-d₆): 2.12 (s, 3H), 2.67 (dd, 1H, J = 7.6, 6.4 Hz), 3.2-3.5 (m, 2H), 3.72 (s, 3H), 6.65-6.8 (m, 3H), 6.87 (d, 2H, J = 9.2 Hz), 7.56 (br, 1H), 7.6-7.7 (m, 4H), 7.34 (br, 1H), 9.81 (s, 1H), 12.30 (s, 1H). | 457.9 |
| 72 | (DMSO-d₆): 3.20 (t, 2H, J = 5.5 Hz), 3.5-3.6 (m, 2H), 4.76 (t, 1H, J = 5.3 Hz), 6.5-6.6 (m, 1H), 6.71 (d, 2H, J = 8.4 Hz), 7.2-7.4 (m, 1H), 7.64 (d, 2H, J = 8.5 Hz), 7.75 (d, 2H, J = 9.0 Hz), 8.14 (d, 1H, J = 4.0 Hz), 8.47 (d, 1H, 9.2 Hz), 8.68 (s, 1H), 10.23 (s, 1H), 12.36 (s, 1H). | 399.2 |
| 74 | (DMSO-d₆): 2.23 (s, 3H), 2.4-2.5 (m, 4H), 3.3-3.4 (m, 4H), 7.09 (d, 2H, J = 9.0 Hz), 7.3-7.4 (m, 1H), 7.73 (d, 2H, J = 8.8 Hz), 7.75-7.85 (m, 2H), 8.15 (d, 1H, J = 3.4 Hz), 8.45-8.50 (m, 1H), 8.68 (d, 1H, J = 2.6 Hz), 10.27 (s, 1H), 12.47 (s, 1H). | 438.4 |
| 75 | (DMSO-d₆): 2.65 (s, 6H), 2.95-3.05 (m, 2H), 3.5-3.6 (m, 2H), 6.7-6.8 (m, 3H), 7.25-7.35 (m, 1H), 7.68 (d, 2H, J = 8.2 Hz), 7.7-7.8 (m, 2H), 8.1-8.2 (m, 1H), 8.4-8.5 (m, 1H), 8.70 (s, 1H), 10.34 (s, 1H), 12.40 (s, 1H). | 426.4 |
| 77 | (DMSO-d₆): 1.35-1.45 (m, 2H), 1.45-1.60 (m, 4H), 2.3-2.5 (m, 6H), 3.15-3.30 (m, 2H), 6.4-6.5 (m, 1H), 6.72 (d, 2H, J = 8.3 Hz), 7.25-7.35 (m, 1H), 7.65 (d, 2H, J = 8.1 Hz), 7.75-7.80 (m, 2H), 8.14 (d, 1H, J = 4.1 Hz), 8.47 (d, 1H, J = 8.2 Hz), 8.68 (s, 1H), 10.24 (s, 1H), 12.37 (s, 1H). | 466.4 |
| 78 | (DMSO-d₆): 1.35-1.45 (m, 2H), 1.45-1.60 (m, 4H), 2.3-2.5 (m, 6H), 3.2-3.3 (m, 2H), 6.4-6.5 (m, 1H), 6.71 (d, 2H, J = 8.4 Hz), 7.65 (d, 2H, J = 8.5 Hz), 7.69 (d, 2H, J = 5.4 Hz), 7.80 (d, 2H, J = 9.0 Hz), 8.34 (d, 2H, J = 5.5 Hz), 10.46 (s, 1H), 12.38 (s, 1H). | 466.2 |
| 84 | (DMSO-d₆): 2.85-2.95 (m, 2H), 3.3-3.4 (m, 2H), 6.7-6.8 (m, 3H), 7.25-7.35 (m, 3H), 7.65 (d, 2H, J = 8.6 Hz), 7.7-7.8 (m, 2H), 8.14 (d, 1H, J = 4.6 Hz), 8.4-8.5 (m 3H), 8.88 (s, 1H), 10.25 (s, 1H), 12.38 (s, 1H). | 460.2 |
| 86 | (DMSO-d₆): 2.8-2.9 (m, 2H), 3.4-3.5 (m, 2H), 6.7-6.8 (m, 3H), 7.32 (d, 2H, J = 4.9 Hz), 7.65 (d, 2H, J = 8.6 Hz), 7.69 (d, 2H, J = 5.2 Hz), 7.75-7.85 (m, 2H), 8.34 (d, 2H, J = 5.5 Hz), 8.48 (d, 2H, J = 5.2 Hz), 10.46 (s, 1H), 12.38 (s, 1H). | 460.2 |
| 87 | (DMSO-d₆): 2.27 (s, 3H), 2.4-2.5 (m, 4H), 3.3-3.4 (m, 4H), 7.10 (d, 2H, J = 8.8 Hz), 7.70 (d, 2H, J = 5.9 Hz), 7.74 (d, 2H, J = 9.6 Hz), 7.85 (d, 2H, J = 9.6 Hz), 8.34 (d, 2H, J = 6.0 Hz), 10.52 (s, 1H), 12.49 (s, 1H). | 438.2 |
| 88 | (DMSO-d₆): 2.25 (s, 6H), 2.5-2.6 (m, 2H), 3.2-3.3 (m, 2H), 6.45-6.50 (m, 1H), 6.73 (d, 2H, J = 8.1 Hz), 7.65 (d, 2H, J = 8.3 Hz), 7.65-7.75 (m, 2H), 7.75-7.85 (m, 2H), 8.3-8.4 (m, 2H), 10.46 (s, 1H), 12.38 (s, 1H). | 426.4 |
| 89 | (DMSO-d₆): 3.1-3.2 (m, 2H), 3.5-3.6 (m, 2H), 4.7-4.8 (m,1H), 6.6-6.7 (m, 1H), 6.71 (d, 2H, J = 8.3 Hz), 7.64 (d, 2H, J = 8.7 Hz), 7.65-7.70 (m, 2H), 7.81 (d, 2H, J = 9.6 Hz), 8.34 (d, 2H, J = 5.1 Hz), 10.47 (s, 1H), 12.38 (s, 1H). | 399.2 |
| 106 | (DMSO-d₆): 2.23 (s, 3H), 2.3-2.5 (m, 4H), 3.2-3.4 (m, 4H), 7.09 (d, 2H, J = 9.2 Hz), 7.44 (d, 1H, J = 5.6 Hz), 7.53 (d, 1H, J = 1.6 Hz), 7.75 (d, 2H, J = 9.2 Hz), 7.81 (br, 1H), 7.95-8.05 (m, 2H), 10.79 (s, 1H), 12.54 (s, 1H). | 455.9 |
| 111 | (DMSO-d₆): 2.19 (s, 6H), 2.45 (t, 2H, J = 6.4 Hz), 3.20 (q, 2H, J = 6.4 Hz), 6.47 (t, 1H, J = 5.2 Hz), 6.72 (d, 2H, J = 8.8 Hz), 7.43 (d, 1H, J = 5.6 Hz), 7.52 (d, 1H, J = 1.6 Hz), 7.65 (d, 2H, J = 8.8 Hz), 7.77 (br, 1H), 7.95 (br, 1H), 7.99 (d, 1H, J = 5.6 Hz), 10.78 (s, 1H), 12.43 (s, 1H). | 443.9 |
| 113 | (DMSO-d₆): 3.1-3.25 (m, 2H), 3.5-3.65 (m, 2H), 4.76 (t, 1H, J = 5.2 Hz), 6.62 (t, 1H, J = 5.6 Hz), 6.72 (d, 2H, J = 8.8 Hz), 7.43 (d, 1H, J = 6.0 Hz), 7.52 (d, 1H, J = 1.6 Hz), 7.65 (d, 2H, J = 8.8 Hz), 7.77 (br, 1H), 7.95 (br, 1H), 7.99 (d, 1H, J = 6.0 Hz), 10.77 (s, 1H), 12.43 (s, 1H). | 416.9 |

### Example 25

### 5-(4-acetamidobenzamido)-2-(4-acetamidophenylamino)thiazole-4-carboxamide:

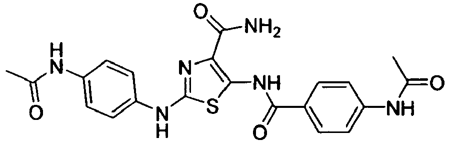

### To a solution of

5-(4-acetamidobenzamido)-2-(4-aminophenylamino)thiazole-4-carboxamide (50 mg, 0.12 mmol) in pyridine (2 mL) was added acetyl chloride (14.4 mg, 0.182 mmol) at 0 °C under nitrogen atmosphere, and the mixture was stirred at rt for 2h. The reaction mixture was concentrated, and the residue was suspended into 1M HCl. The solids were collected by filtration and washed successively with hexane, methanol, and dried to give 52 mg (76% yield) of the titled compound.
¹H-NMR (400 MHz, DMSO-d₆) δ (ppm) 2.01 (s, 3H), 2.09 (s, 3H), 7.51 (d, 2H, J = 8.4 Hz), 7.66 (d, 2H, J = 8.4 Hz), 7.73 (s, 1H), 7.8-7.9 (m, 5H), 9.81 (s, 1H), 9.98 (s, 1H), 10.34 (s, 1 H), 12.54 (s, 1 H). LCMS m/z [M+H]⁺ 453.4.

### Example 33

### 5-(4-aminobenzamido)-2-(4-methoxyphenylamino)thiazole-4-carboxamide:

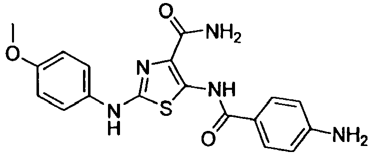

### (a) 2-(4-methoxyphenylamino)-5-(4-nitrobenzamido)thiazole-4-carboxamide:

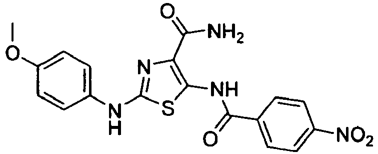

To a solution of 4-nitrobenzoyl chloride (0.7 g, 3.78 mmol) in pyridine (6 mL) was added a solution of 5-amino-2-(4-methoxyphenylamino)thiazole-4-carboxamide (1 g, 3.7 mmol) in pyridine (6 mL) at 0 °C, and the mixture was stirred at rt for 12 h. The solvent was evaporated, and the residue was diluted with water and extracted with ethyl acetate. The organic layer was washed successively with 1M HCl (2 x 100 mL), water (2 x 50 mL), sat. NaHCO₃ (2x50mL), water (50 mL) and brine (50 mL). The organic layer was dried over Na₂SO₄ and concentrated to give 0.41 g (27% yield) of the title compound, which was used for next step without further purification.
¹H-NMR (400 MHz, DMSO-d₆) δ (ppm) 3.70 (s, 3H), 6.88 (d, 2H, J = 8.7 Hz), 7.66 (d, 2H, J = 8.7 Hz), 7.70 (s, 1H), 7.88 (s, I H), 8.13 (d, 2H, J = 8.4 Hz), 8.43 (d, 2H, J = 8.4 Hz), 9.93 (s, 1H), 12.76 (s, 1H). LCMS m/z (M+H]⁺ 414.2.

### (b) 5-(4-aminobenzamido)-2-(4-methoxyphenylamino)thiazole-4-carboxamide:

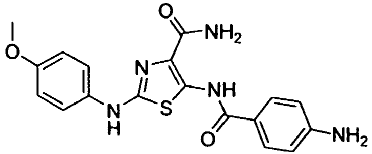

To a solution of 2-(4-methoxyphenylamino)-5-(4-nitrobenzamido)thiazole-4-carboxamide (0.1g, 0.24 mmol) in THF-EtOH (1:1, 30 mL) was added stannous chloride dihydrate (0.27 g, 1.2 mmol) at rt, and the mixture was refluxed for 5h. The reaction mixture was concentrated, and the residue was diluted with EtOAc. 1M NaOH was added to the solution until the solution was basic (pH = 8-9). The organic layer was separated, and the aqueous layer was extracted with EtOAc (2 x 50 mL). The combined organic layers were filtered through a bed of Celite. The filtrate was washed with water, and dried over Na₂SO₄ and concentrated. The resulting solids were collected and washed with hexane to give 39 mg (42% yield) of the titled compound.
¹H-NMR (400 MHz, DMSO-d₆) δ (ppm) 3.72 (s, 3H), 6.03 (s, 2H), 6.65 (d, 2H, J = 8.3 Hz), 6.87 (d, 2H, J = 8.6 Hz), 7.53 (s, 1H), 7.58 (d, 2H, J = 8.3 Hz), 7.54 (d, 2H, J = 8.5 Hz), 7.70 (s, 1H), 9.78 (s, 2H), 12.25 (s, 1H). LCMS m/z [M+H]⁺ 384.0.

### Example 35

### 5-(4-hydroxybenzamido)-2-(4-methoxyphenylamino)thiazole-4-carboxamide:

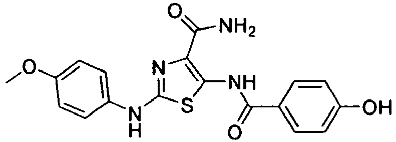

### (a) 4-[4-carbamoyl-2-(4-methoxyphenylamino)thiazol-5-ylcarbamoyl]phenyl acetate:

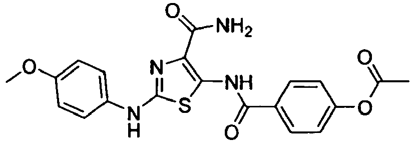

To a suspension of acetoxybenzoic acid (350 mg, 1.97 mmol) in CH₂Cl₂ (36 mL) was added oxalyl chloride (0.696 mL, 7.95 mmol) and catalytic amount of DMF at 0°C, and the mixture was stirred for 5 hr at rt. The solvent was evaporated, and the residual oxalyl chloride was removed with azeotropic distillation using toluene under nitrogen atmosphere. The resulting acid chloride was added to a solution of 5-amino-2-(4-methoxyphenylamino)thiazole-4-carboxamide (350 mg, 1.32 mmol) in pyridine (10 mL) at 0°C, and the mixture was stirred for 2 hr at rt. The reaction mixture was quenched by adding of ice-water, and extracted with EtOAc. The organic layer was washed with water twice, dried over Na₂SO₄ and concentrated. The residue was triturated with 50% EtOAc in Et₂O, and the resulting solids were collected by filtration and washed with 50% EtOAc in Et₂O to afford the titled compound (382 mg, 68 % yield).
¹H-NMR (400 MHz, DMSO-d₆) δ (ppm) 2.32 (s, 3H), 3.73 (s, 3H), 6.89 (d, 2H, J = 8.8 Hz), 7.39 (d, 2H, J = 8.8 Hz), 7.6-7.7 (m, 3H), 7.84 (br, 1H), 7.94 (d, 2H, J = 8.8 Hz), 9.90 (s, 1H), 12.59 (s, 1 H).

### (b) 5-(4-hydroxybenzamido)-2-(4-methoxyphenylamino)thiazole-4-carboxamide:

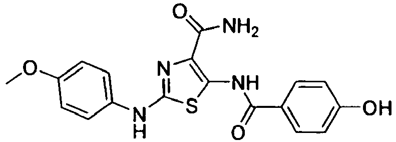

To a suspension of 4-[4-carbamoyl-2-(4-methoxyphenylamino)thiazcl-5-ylcarbamoyl]phenyl acetate (350 mg, 0.821 mmol) in dry MeOH (45 mL) was added K₂CO₃ (113 mg, 0.821 mmol), and the mixture was stirred at 50°C for 20 min. The reaction mixture was cooled to 0°C, and then diluted with water. The solution was acidified with 2M HCl (0.5 mL). The resulting solids were collected by filtration and washed with water to afford the titled compound (250 mg, 79 % yield).
¹H-NMR (400 MHz, DMSO-d₆) δ (ppm) 3.72 (s, 3H), 6.88 (d, 2H, J = 9.2 Hz), 6.95 (d, 2H, J = 8.8 Hz), 7.60 (br, 1H), 7.65 (d, 2H, J = 9.2 Hz), 7.7-7.8 (m, 3H), 9.84 (s, 1H), 10.40 (br, 1H), 12.42 (s, I H). LCMS m/z [M+H]⁺ 384.8.

### Example 37

### 5-[4-(2-hydroxyacetamido)benzamido]-2-(4-methoxyphenylamino)thiazole-4-carbo xamide:

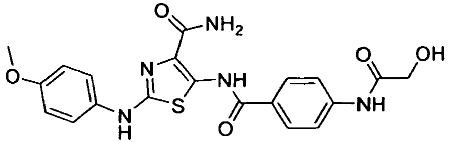

To a mixture of 5-(4-aminobenzamido)-2-(4-methoxyphenylamino)thiazole-4-carboxamide (0.05g, 0.13 mmol) and triethylamine (0.018 mL) in THF (10 mL) was added dropwise acetoxy acetylchloride (0.016 mL, 0.16 mmol) at 0 °C, and the mixture was stirred at rt for 2 h. The reaction mixture was diluted with water and extracted with EtOAc (3 x 20 mL). The combined organic layers were dried over Na₂SO₄ and concentrated. The resulting solids were dissolved in MeOH (5 mL), and K₂CO₃ (30 mg, 0.22 mmol) and catalytic amount of water was added to this solution. The mixture was stirred at rt for 1h. The solvent was evaporated, and the residue was purified by silica gel column chromatography eluted with 2% MeOH in DCM to give 7 mg (9% yield) of the titled compound.
¹H-NMR (400 MHz, DMSO-d₆) δ (ppm) 3.72 (s, 3H), 4.04 (d, 2H, J = 5.8 Hz), 5.7-5.8 (m, 1H), 6.88 (d, 2H, J = 8.6 Hz), 7.6-7.7 (m, 3H), 7.80 (s, 1H), 7.85 (d, 2H, J = 8.4 Hz), 7.94 (d, 2H, J = 8.4 Hz), 9.86 (s, 1H), 10.07 (s, 1H), 12.5 (s, 1H). LCMS m/z [M+H]⁺ 442.3.

### Example 38

### 5-{4-[2-(dimethylamino)acetamido]benzamido}-2-(4-methoxyphenylamino)thiazole -4-carboxamide:

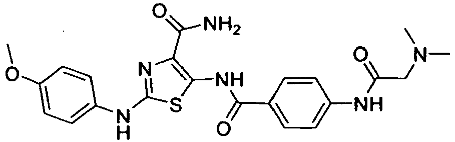

### (a) 5-[4-(2-bromoacetamido)benzamido]-2-(4-methoxyphenylamino)thiazole-4-carbox amide:

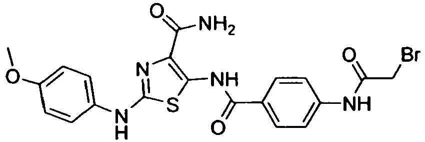

To a mixture of 5-(4-aminobenzamido)-2-(4-methoxyphenylamino)thiazole-4-carboxamide (0.3 g, 0.78 mmol) and triethylamine (0.213 mL, 1.56 mmol) in THF (30 mL) was added dropwise bromoacetylchloride (135 mg, 0.86 mmol) at 0 °C, and the mixture was stirred at rt for 4h. The reaction mixture was diluted with water and extracted with EtOAc. The organic layer was dried over Na₂SO₄ and concentrated. The resulting solids were washed with MeOH, and dried to give 0.3 g (75% yield) of the titled compound.
¹H-NMR (400 MHz, DMSO-d₆) δ (ppm) 3.72 (s, 3H), 4.09 (s, 2H), 6.88 (d, 2H, J = 8.6 Hz), 7.6-7.7 (m, 3H), 7.8-7.9 (m, 5H), 9.86 (s, I H), 10.75 (s, 1H), 12.52 (s, 1H). LCMS m/z [M+H]⁺ 506.2.

### (b) 5-{4-[2-(dimethylamino)acetamido]benzamido}-2-(4-methoxyphenylamino)thiazole -4-carboxamide:

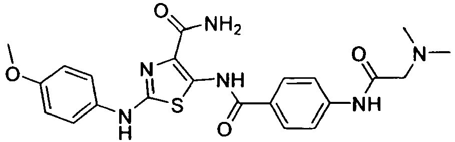

To a solution of dimethylamine (11% in MeOH, 0.07 mL, 0.138 mmol) in THF (10 mL) was added NaHCO₃ (11.5 mg, 0.138 mmol), and the mixture was stirred for 15 min at rt. A solution of 5-[4-(2-bromoacetamido)benzamido]-2-(4-methoxyphenylamino)thiazole-4-carboxamid e (70 mg, 0.138 mmol) in THF was added slowly to this solution at 0 °C, and the mixture was stirred at rt overnight. The solvent was evaporated, and water (10 mL) was added to the residue. The resulting solids were collected by filtration and purified by silica gel column chromatography eluted with 60% EtOAc in hexanes to give 15 mg (23% yield) of the titled compound.
¹H-NMR (400 MHz, DMSO-d₆) δ (ppm) 2.28 (s, 6H), 3.12 (s, 2H), 3.72 (s, 3H), 6.88 (d, 2H, J = 8.6 Hz), 7.6-7.7 (m, 3H), 7.80 (s, 1H), 7.83 (d, 2H, J = 8.5 Hz), 7.90 (d, 2H, J = 8.4 Hz), 9.86 (s, 1H), 10.11 (s, 1H), 12.51 (s, 1H). LCMS m/z [M+H]⁺ 469.3.

### Example 39-42

The compounds shown in the following Table 8 were prepared by following the procedure described for above Example 38 using appropriate starting materials.

**Table 8**

| Ex. No. | ¹H-NMR δ (ppm) | LCMS m/z [M+H]⁺ |
|---|---|---|
| 39 | (DMSO-d₆): 1.7-1.8 (m, 4H), 2.5-2.7 (m, 4H), 3.3-3.4 (m, 2H), 3.73 (s, 3H), 6.8-6.9 (m, 2H), 7.6-7.7 (m, 3H), 7.8-7.9 (m, 5H), 9.86 (s, 1H), 10.10 (s, 1H), 12.51 (s, 1H). | 495.1 |
| 40 | (DMSO-d₆): 3.18 (s, 2H), 3.2-3.4 (m, 4H), 3.6-3.6 (m, 4H), 3.72 (s, 3H), 6.88 (d, 2H, J = 8.8 Hz), 7.62 (s, 1H), 7.65 (d, 2H, J = 8.7 Hz), 7.8-7.9 (m, 5H), 9.85 (s, 1H), 10.12 (s, 1H), 12.51 (s, 1H). | 511.5 |
| 41 | (DMSO-d₆): 1.4-1.5 (m, 2H), 1.5-1.6 (m, 4H), 2.4-2.5 (m, 4H), 3.11 (s, 2H), 3.72 (s, 3H), 6.87 (d, 2H, J = 8.8 Hz), 7.6-7.7 (m, 3H), 7.8-7.9 (m, 5H), 9.85 (s, 1H), 10.04 (s, 1H), 12.51 (s, 1H). | 509.4 |
| 42 | (CD₃OD): 2.31 (s, 3H), 2.5-2.7 (m, 8H), 3.22 (s, 2H), 3.77 (s, 3H), 6.90 (d, 2H, J = 8.7 Hz), 7.51 (d, 2H, J = 8.8 Hz), 7.81 (d, 2H, J = 8.6 Hz), 7.92 (d, 2H, J = 8.4 Hz). | 524.2 |

### Example 44

### 2-(4-methoxyphenylamino)-5-{4-[(4-methylpiperazin-1-yl)methyl]benzamido}thiaz ole-4-carboxamide:

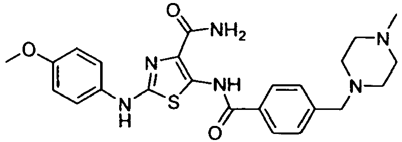

To a mixture of 5-amino-2-(4-methoxyphenylamino)thiazole-4-carboxamide (50 mg, 0.189 mmol) and N,N-diisopropylethylamine (27 mg, 0.208 mmol) in DMA (2 mL) was added 4-chloromethylbenzoyl chloride (39 mg, 0.208 mmol) at 0°C, and the mixture was stirred at rt. After 2h, 1-methylpiperazine (95 mg, 0.946 mmol) was added to this mixture, and the stirring was continued for 3 h at rt. The reaction mixture was diluted with EtOAc, and the organic layer was washed with water twice, dried over Na₂SO₄ and concentrated. The residue was purified with silica gel chromatography (eluent: CHCl₃ to 12% MeOH in CHCl₃) to afford the titled compound (34 mg, 37 % yield).
¹H-NMR (400 MHz, DMSO-d₆) δ (ppm) 2.17 (s, 3H), 2.2-2.6 (m, 8H), 3.55 (s, 2H), 3.73 (s, 3H), 6.88 (d, 2H, J = 9.2 Hz), 7.53 (d, 2H, J = 8.0 Hz), 7.6-7.7 (m, 3H), 7.83 (br, 1H), 7.85 (d, 2H, J = 8.4 Hz), 9.88 (s, 1H), 12.56 (s, I H), LCMS m/z [M+H]⁺ 481.4.

### Example 99, 101, 107, 109 and 110

The compounds shown in the following Table 9 were prepared by following the procedure described for above Example 44 using appropriate starting materials.

**Table 9**

| Ex. No. | ¹H-NMR δ (ppm) | LCMS m/z [M+H]⁺ |
|---|---|---|
| 99 | (DMSO-d₆): 2.85-2.95 (m, 4H), 3.15-3.26 (m, 4H), 3.30 (br., 1H), 4.31 (br, 2H), 7.46 (br, 2H), 7.8-7.9 (m, 2H), 7.9-8.0 (m, 2H), 8.12 (br, 1H), 8.41 (br, 1H), 8.73 (d, 1H, J = 7.9 Hz), 9.2-9.5 (m, 2H), 11.48 (s, 1H), 12.70 (s, 1H). | 438.4 |
| 101 | (DMSO-d₆): 2.3-2.45 (m, 4H), 3.5-3.65 (m, 6H), 7.31 (dd, 1H, J = 8.4, 4.8 Hz), 7.56 (d, 2H, J = 8,4 Hz), 7.8-7.95 (m, 4H), 8.16 (dd, 1H, J = 8.4, 1.6 Hz), 8.45-8.55 (m, 1H), 8.69 (d, 1H, J = 2.8 Hz), 10.32 (s, 1H), 12.65 (s, 1H). | 438.9 |
| 107 | (DMSO-d₆): 2.16 (s, 3H), 2.23-2.47 (m, 8H), 3.56 (s, 2H), 7.44 (d, 1H, J = 5.2 Hz), 7.5-7.6 (m, 3H), 7.8-7.9 (m, 3H), 8.00 (d, 1H, J = 5.2 Hz), 8.05 (br, 1H), 10.84 (s, 1H), 12.71 (s, 1H). | 470.0 |
| 109 | (Trifluoroacetic acid-d): 3.12 (s, 3H), 3.7-4.1 (m, 8H), 4.66 (br, 2H), 7.75-7.85 (m, 2H), 7.9-8.0 (m, 1H), 8.0-8.1 (m, 2H), 8.35-8.45 (m, 1H), 8.45-8.55 (m, 1H), 9.77 (br, 1H) | 452.2 |
| 110 | (DMSO-d₆): 2.32 (s, 6H), 3.73 (s., 2H), 7.60 (d, 2H, J=7.8 Hz), 7.73 (d, 2H, J=5.9 Hz), 7.85-8.00 (m, 3 H), 8.14 (s, 1H), 8.36 (d, 2H, J=4.9 Hz), 10.66 (br, 1H), 12.69 (br, 1H) | 397.2 |

### Example 45

### 2-(4-methoxyphenylamino)-5-{4-[2-(pyrrolidin-1-yl)ethoxy]benzamido}thiazole-4-c arboxamide:

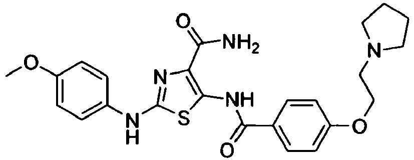

To a mixture of 5-(4-hydroxybenzamido)-2-(4-methoxyphenylamino)thiazole-4-carboxamide (50 mg, 013 mmol) and potassium carbonate (47 mg, 0.34 mmol) in DMF (2 mL) was added 2-chloroethylpyrrolidine hydrochloride (29 mg, 0.17 mmol) at rt , and the mixture was stirred at 80°C for 2 hr. The reaction mixture was diluted with EtOAc, and washed with water twice, dried over Na₂SO₄ and concentrated. The residue was purified by silica gel chromatography (eluent: CHCl₃ to 12% MeOH in CHCl₃) to afford the titled compound (16 mg, 26 % yield).
¹H-NMR (400 MHz, DMSO-d₆) δ (ppm) 1.5-1.8 (m, 4H), 2.3-2.6 (m, 4H), 2.7-3.0 (m, 2H), 3.73 (s, 3H), 4.18 (t, 2H, J = 5.6 Hz), 6.88 (d, 2H, J = 9.2 Hz), 7.17 (d, 2H, J = 8.8 Hz), 7.63 (br, I H), 7.66 (d, 2H, J = 8.8 Hz), 7.80 (br, I H), 7.84 (d, 2H, J = 8.8 Hz), 9.86 (s, I H), 12.49 (s, 1H), LCMS m/z [M+H]⁺ 482.4.

### Example 58, 60 and 122

The compounds shown in the following Table 10 were prepared by following the procedure described for above Example 45 using appropriate starting materials.

**Table 10**

| Ex. No. | ¹H-NMR δ (ppm) | LCMS m/z [M+H]⁺ |
|---|---|---|
| 58 | (DMSO-d₆): 3.33 (s, 3H), 3.6-3.71 (m, 2H), 3.73 (s, 3H), 4.15-4.25 (m, 2H), 6.88 (d, 2H, J = 8.8 Hz), 7.16 (d, 2H, J = 8.8 Hz), 7.63 (br, 1H), 7.65 (d, 2H, J = 8.8 Hz), 7.81 (br, 1H), 7.84 (d, 2H, J = 8.8 Hz), 9.87 (s, 1H), 12.50 (s, 1H). | 443.0 |
| 60 | (DMSO-d₆): δ 2.23 (s, 6H), 2.66 (t, 2H, J = 5.6 Hz), 3.73 (s, 3H), 4.16 (t, 2H, J = 5.6 Hz), 6.88 (d, 2H, J = 9.2 Hz), 7.16 (d, 2H, J = 9.2 Hz), 7.62 (br, 1H), 7.66 (d, 2H, J = 9.2 Hz), 7.80 (br, 1H), 7.84 (d, 2H, J = 8.4 Hz), 9.86 (s, 1 H), 12.49 (s, 1H). | 456.3 |
| 122 | (DMSO-d₆): 3.75 (q, 2H, J = 5.2 Hz), 4.11 (t, 2H, J = 5.2 Hz), 4.92 (t, 1H, J = 5.6 Hz), 7.17 (d, 2H, J = 8.8 Hz), 7.44 (d, 1H, J = 5.6 Hz), 7.53 (s, 1H), 7.8-7.9 (m, 3 H), 8.00 (d, 1H, J = 5.6 Hz), 8.02 (br, 1H), 10.82 (s, 1H), 12.64 (s, 1H). | 418.2 |

### Example 47

### 5-(4-methoxybenzamido)-2-(pyridin-4-ylamino)thiazole-4-carboxamide:

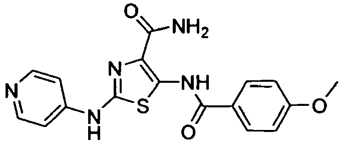

### (a) ethyl 5-amino-2-bromothiazole-4-carboxylate:

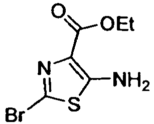

N-Bromosuccinimide (0.54 g, 3.03 mmol) was added to a solution of 5-aminothiazole-4-carboxylic acid ethyl ester (0.44 g, 2.53 mmol), prepared according to the procedure described by Golankiewicz et al. (Tetrahedron, 41 (24), 5989-5994 (1985)) in acetonitrile (10 mL), and the mixture was stirred for 30 min. The reaction mixture was diluted with EtOAc (50 mL) and washed with 5% K₂CO₃ aq. solution (25 mL) followed by brine (25mL). The organic layer was dried over Na₂SO₄ and concentrated. The residue was purified by silica gel column chromatography eluted with 15% EtOAc in hexane to give 0.37 g (58% yield) of the titled compound.
¹H-NMR (400 MHz, CDCl₃) δ (ppm) 1.38 (t, 3H, J = 7.1 Hz), 4.37 (q, 2H, J = 7.1 Hz), 6.02 (s, 2H). LCMS m/z [M+H]⁺ 253.1.

### (b) ethyl 2-bromo-5-(4-methoxybenzamido)thiazole-4-carboxylate:

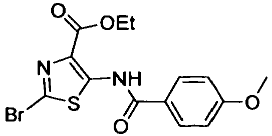

To a solution of p-anisoyl chloride (1.87 g, 11 mmol) in pyridine (30 mL) was added dropwise a solution of ethyl 5-amino-2-bromothiazole-4-carboxylate (1.5 g, 5.5 mmol) in pyridine (52 mL) over a period of 10 min at 0 °C, the mixture was stirred for 72 h at rt. The reaction mixture was diluted with 1M HCl and extracted with EtOAc (4 x 200 mL). The combined organic extracts were washed with brine, dried over Na₂SO₄ and concentrated. The residue was purified by silica gel column chromatography eluted with 10 % EtOAc in hexane to give 1.35 g (58% yield) of the titled compound.
¹H-NMR (400 MHz, CDCl₃) δ (ppm) 1.45 (t, 3H, J = 7.1 Hz), 3.89 (s, 3H), 4.48 (q, 2H, J = 7.1 Hz), 7.02 (d, 2H, J = 8.6 Hz), 7.96 (d, 2H, J = 8.6 Hz), 11.72 (s, 1H). LCMS m/z [M+H]⁺ 385.2.

### (c) ethyl 5-(4-methoxybenzamido)-2-(pyridin-4-ylamino)thiazole-4-carboxylate:

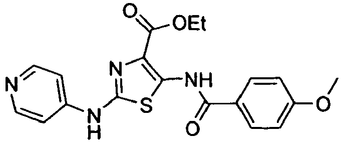

To the solution of ethyl 2-bromo-5-(4-methoxybenzamido)thiazole-4-carboxylate (0.2 g, 0.519 mmol) in 1,4-dioxane (18 mL) was added Xantphos (0.060 g, 0.1 mmol) and Pd₂(dba)₃ (0.047g, 0.05 mmol) under argon gas. Cesium carbonate (0.337g, 1.03 mmol) and 4-aminopyridine (0.048 g, 0.519 mmol) was then added to this solution, and the mixture was refluxed for 5h. The reaction mixture was filtered through a bed of Celite, and the celite was washed with EtOAc (3 x 5 mL). The filtrate was concentrated, and the residue was purified by silica gel column chromatography eluted with 40 % EtOAc in Hexane to give 54 mg (26% yield) of the titled compound.
¹H-NMR (400 MHz, DMSO-d₆) δ (ppm) 1.38 (t, 3H, J = 7.2 Hz), 3.87 (s, 3H), 4.42 (q, 2H, J = 7.0 Hz), 7.17 (d, 2H, J = 8.7 Hz), 7.58 (d, 2H, J = 5.6 Hz), 7.92 (d, 2H, J = 8.6 Hz), 8.39 (d, 2H, J = 5.2 Hz), 10.60 (s, 1H), 11.39 (s, 1H). LCMS m/z [M+H]⁺ 399.1.

### (d) 5-(4-methoxybenzamido)-2-(pyridin-4-ylamino)thiazole-4-carboxamide:

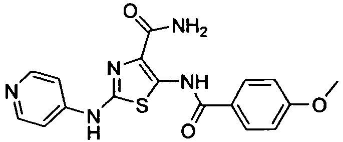

To a solution of ethyl 5-(4-methoxybenzamido)-2-(pyridin-4-ylamino)thiazole-4-carboxylate (0.05 g, 0.12 mmol) in THF (3 mL) was added 7M NH₃ in MeOH (7mL), and the solution was heated at 80 °C in a sealed tube for 5 h. The solvent was evaporated, and the resulting solid was collected. The solids were washed with ether and dried to give 21 mg (45% yield) of the titled compound.
¹H-NMR (400 MHz, DMSO-d₆) δ (ppm) 3.86 (s, 3H), 7.16 (d, 2H, J = 8.3 Hz), 7.6-7.8 (m, 2H), 7.8-8.0 (m, 4H), 8.3-8.4 (m, 2H), 10.50 (s, 1H), 12.58 (s, 1H). LCMS m/z [M+H]⁺ 370.4.

### Example 50-51 and 57

The compounds shown in the following Table 11 were prepared by following the procedure described for above Example 47 using appropriate starting materials.

**Table 11**

| Ex. No. | ¹H-NMR δ (ppm) | LCMS m/z [M+H]⁺ |
|---|---|---|
| 50 | (DMSO-d₆): 3.86 (s, 3H), 7.16 (d, 2H, J = 8.28 Hz), 7.31 (dd, 1H, J = 4.68 and 7.8 Hz), 7.8-7.9 (m, 4H), 8.15 (d, 1H, J = 3.92 Hz), 8.47 (d, 1H, J = 8.0 Hz), 8.68 (s, 1H), 10.29 (s, 1H), 12.56 (s, 1H). | 370.3 |
| 51 | (DMSO-d₆): 3.86 (s, 3H), 7.16 (d, 2H, J = 8.4 Hz), 7.38 (m, 1H), 7.8-8.0 (m, 4H), 7.98 (d, 2H, J = 9.0 Hz), 8.17 (d, 2H, J = 8.9 Hz), 10.88 (s, 1H), 12.58 (s, 1H). | 414.1 |
| 57 | (DMSO-d₆): 3.86 (s, 3H), 7.1-7.3 (m, 4H), 7.6-8.0 (m, 8H), 10.47 (s, 1H), 12.58 (s, 1H). | 446.3 [M-H]⁺ |

### Example 48

### 2-(4-methoxyphenylamino)-5-{4-[N-(methylsulfonyl)methylsulfonamido]benzamid o}thiazole-4-carboxamide:

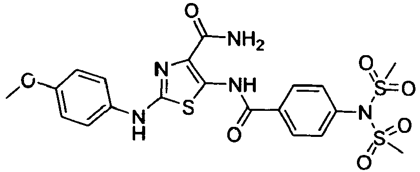

To a mixture of 5-(4-aminobenzamido)-2-(4-methoxyphenylamino)thiazole-4-carboxamide (0.150 g, 0.3916 mmol) and Et₃N (0.2 mL, 1.56 mmol) in THF (10 mL) was added dropwise methanesulfonyl chloride (0.09 mL, 1.174 mmol) at 0°C,. and the mixture was stirred at rt for 2h. The reaction mixture was concentrated. Water was added to the residual oil, and the resulting solids were collected by filtration to give 100 mg (47% yield) of the titled compound.
¹H-NMR (400 MHz, DMSO-d₆) δ (ppm) 3.59 (s, 6H), 3.72 (s, 3H), 6.88 (d, 2H, J = 8.8 Hz), 7.6-7.7 (m, 3H), 7.78 (d, 2H, J = 8.1 Hz), 7.86 (br, 1H), 7.97 (d, 2H, J = 8.1 Hz), 9.91 (s, 1H), 12.63 (s, 1H). LCMS m/z [M+H]⁺ 540.4.

### Example 53

### 2-(4-methoxyphenylamino)-5-[4-(methylsulfonamido)benzamido]thiazole-4-carbox amide:

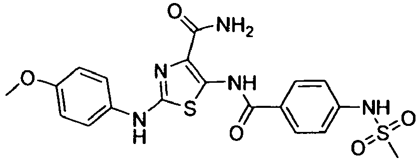

To a solution of 5-(4-aminobenzamido)-2-(4-methoxyphenylamino)thiazole-4-carboxamide (0.06 g, 0.156 mmol) in THF (10 mL) was added dropwise freshly distilled methanesulfonyl chloride (0.02 mL, 0.313 mmol) at 0°C, then Et₃N (0.06 mL, 0.468 mmol) was added to this solution at 0°C. The mixture was stirred at rt for 12h. To complete the reaction, another 0.5 mol equivalent of methanesulfonyl chloride and I mol equivalent Et₃N was added to the mixture, and the stirring was continued overnight. The reaction mixture was concentrated, and the residue was purified by silica gel column chromatography eluted with 50% ethyl acetate in hexanes to give 11.5 mg (15% yield) of the titled compound.
¹H-NMR (400 MHz, DMSO-d₆) δ (ppm) 33.12 (s, 3H), 3.72 (s, 3H), 6.87 (d, 2H, J = 8.9 Hz), 7.36 (d, 2H, J = 8.6 Hz), 7.64 (br, 2H), 7.65 (d, 4H, J = 9.0 Hz), 7.82 (br, I H), 7.85 (t, 2H, J = 8.6 Hz), 9.88 (s, 1H), 12.50 (s, 1H). LCMS m/z [M+H]⁺ 462.2.

### Example 64

### 5-(3-amino-4-methylbenzamido)-2-(4-methoxyphenylamino)thiazole-4-carboxamid e

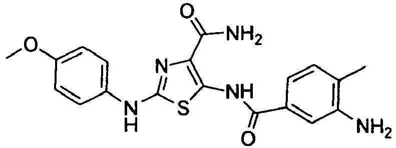

To a solution of 2-(4-methoxyphenylamino)-5-(4-methyl-3-nitrobenzamido)thiazole-4-carboxamide (0.2 g, 0.47 mmol) in THF-MeOH (1:1, 100 mL) was added 10% palladium on carbon catalyst (0.02 g) at rt, and the mixture was stirred under H₂ gas for 12 h. The reaction mixture was filtered through a bed of Celite. The filtrate was concentrated to give 0.165 g (89% yield) of the titled compound.
¹H-NMR (400 MHz, DMSO-d₆) δ (ppm) 2.12 (s, 3H), 3.72 (s, 3H), 5.25 (d, 2H, J = 8.8 Hz), 6.88 (d, 2H, J = 8.8 Hz), 6.95 (d, 1H, J = 7.5 Hz), 7.12 (d, 1H, J = 7.6 Hz), 7.17 (s, 1H), 7.58 (s, 1H), 7.65 (d, 2H, J = 8.8 Hz), 7.75 (s, 1H), 9.83 (s, 1H), 12.34 (s, 1H). LCMS m/z [M+H]⁺ 398.2.

### Example 85, 90, 93 and 108

The compounds shown in the following Table 12 were prepared by following the procedure described for above Example 64 using appropriate starting materials.

**Table 12**

| Ex. No. | ¹H-NMR δ (ppm) | LCMS m/z [M+H]⁺ |
|---|---|---|
| 85 | (DMSO-d₆): 2.09 (s, 3H), 6.95-7.00 (m, 1H), 7.14 (d, 1H, J = 7.4 Hz), 7.20 (s, 1H), 7.35-7.40 (m, 1H), 7.80 (s, 1H), 7.85 (s, 1H), 8.19 (d, 1H, J = 4.1 Hz), 8.45-8.55 (m, 1H), 8.79 (s, 1H), 10.45 (s, 1H), 12.43 (s, 1H). | 369.0 |
| 90 | (DMSO-d₆): 2.12 (s, 3H), 5.30 (br, 1H), 6.97 (d, 1H, J = 8.3 Hz), 7.14 (d, 1H, J = 8.1 Hz), 7.20 (s, 1H), 7.8-8.0 (m, 4H), 8.39 (d, 2H, J = 6.1 Hz), 10.98 (br, 1H), 12.47 (s, 1H). | 369.0 |
| 93 | (DMSO-d₆): 6.10 (br, 2H), 6.6-6.7 (m, 2H), 7.55-7.65 (m, 2H), 7.8-8.0 (m, 4H), 8.35-8.45 (m, 2H), 11.17 (br, 1H), 12.38 (s, 1H). | 355.2 |
| 108 | (DMSO-d₆): 6.68 (d, 2H, J=8.3 Hz), 7.60 (d, 2H, J=7.9 Hz), 7.8-7.9 (m, 2H), 7.99 (br, 1H), 8.35-8.40 (m, 1H), 8.6-8.7 (m, 1H), 9.40 (s, 1H), 11.25 (s, 1H), 12.35 (s, 1H) | 355.0 |

### Example 67

### 5-(4-methoxybenzamido)-2-[4-(2-methoxyethoxy)phenylamino]thiazole-4-carboxa mide

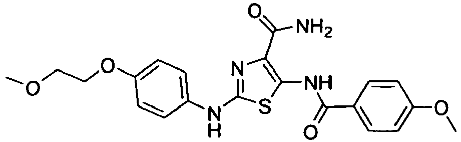

### (a) 1-(2-methoxyethoxy)-4-nitrobenzene

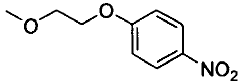

A mixture of 1-fluoro-4-nitrobenzene (2.00 g, 14.18 mmol), 2-methoxyethanol (1.28 g, 16.84 mmol) and KOH (1.37 g, 21.07 mmol) in DMSO (30 mL) was heated at 60°C for 20 h. The reaction mixture was diluted with water (50 mL) and extracted with AcOEt (3 x 100 mL). The combined organic layers were dried over Na₂SO₄ and concentrated. The residue was purified by silica gel column chromatography eluted with 10 % EtOAc in hexane to give 0.37 g (13% yield) of the titled compound.
¹H-NMR (400 MHz, DMSO-d₆) δ (ppm) 3.31 (s, 3H), 3.69 (t, 2H, J = 4.4 Hz), 4.25 (t, 2H, J = 4.1 Hz), 7.16 (d, 2H, J = 9.2 Hz), 8.20 (d, 2H, J = 9.0 Hz). LCMS m/z [M+H]⁺ 198.4.

### (b) 4-(2-methoxyethoxy)benzenamine

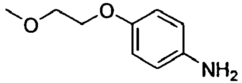

To a solution of 1-(2-methoxyethoxy)-4-nitrobenzene (0.37 g, 1.88 mmol) in THF-MeOH (1:1, 60 mL) was added 10% palladium on carbon catalyst (0.04 g) at rt, and the mixture was stirred under H₂ gas for 3 h. The reaction mixture was filtered through a bed of Celite. The filtrate was concentrated to give 0.31 g (99% yield) of the titled compound.
¹H-NMR (400 MHz, DMSO-d₆) δ (ppm) 3.28 (s, 3H), 3.57 (t, 2H, J = 4.7 Hz), 3.91 (t, 2H, J = 4.1 Hz), 4.58 (s, 2H), 6.49 (d, 2H, J = 8.6 Hz), 6.63 (d, 2H, J = 8.4 Hz). LCMS m/z [M+H]⁺ 168.4.

### (c) 1-isothiocyanato-4-(2-methoxyethoxy)benzene

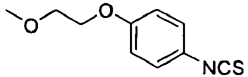

To a solution of 4-(2-methoxyethoxy)benzenamine (0.31 g, 1.85 mmol) in DCM (40 mL) was added thiocarbonyl diimidazole (0.39 g, 2.2 mmol) at 0°C for 2 h. The reaction mixture was concentrated, and the residue was purified by silica gel column chromatography eluted with 10 % EtOAc in hexane to give 0.37 g (96% yield) of the titled compound.
¹H-NMR (400 MHz, DMSO-d₆) δ (ppm) 3.29 (s, 3H), 3.64 (q, 2H, J = 3.0 Hz), 4.11 (q, 2H, J = 4.4 Hz), 6.99 (d, 2H, J = 9.0 Hz), 7.38 (d, 2H, J = 9.0 Hz). LCMS m/z [M+H]⁺ 210.4.

### (d) 5-amino-2-[4-(2-methoxyethoxy)phenylamino]thiazole-4-carboxamide

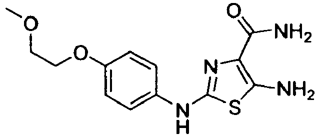

A mixture of 1-isothiocyanato-4-(2-methoxyethoxy)benzene (0.37 g, 1.78 mmol) and 2-amino-2-cyanoacetamide (0.18 g, 1.78 mmol) in EtOAc (15 mL) was refluxed for 1 h. The reaction mixture was concentrated, and the residue was purified by silica gel chromatography (eluent: 10% MeOH in DCM) to afford the titled compound (0.2 g, 36 % yield).
¹H-NMR (400 MHz, DMSO-d₆) δ (ppm) 3.4 (s, 3H), 3.6-3.7 (m, 2H), 4.0-4.1 (m, 2H), 6.60 (br, 1H), 6.75-6.90 (m, 4H), 7.49 (d, 2H, J = 8.7 Hz), 9.33 (s, 1H).

### (e) 5-(4-methoxybenzamido)-2-[4-(2-methoxyethoxy)phenylamino]thiazole-4-carboxa mide

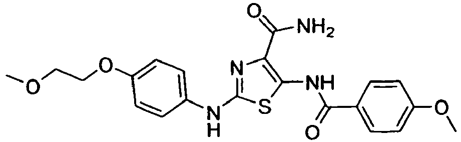

To a solution of p-anisoyl chloride (0.074 g, 0.44 mmol) in pyridine (5 mL) was added 5-amino-2-(4-(2-methoxyethoxy)phenylamino)thiazole-4-carboxamide (0.15 g, 0.49 mmol) in pyridine (3 mL) at 0°C, the mixture was allowed to warm to rt and stirred for 12 h at rt. The reaction mixture was concentrated, and the residue was dissolved in EtOAc and washed successively with 1M HCl and sat. NaHCO₃. The organic layer was dried over Na₂SO₄ and evaporated to afford the titled compound (0.026 g, 12% yield).
¹H-NMR (400 MHz, DMSO-d₆) δ (ppm) 3.27 (s, 3H), 3.6-3.7 (m, 2H), 3.86 (s, 3H), 4.0-4.1 (m, 2H), 6.88 (d, 2H, J = 8.6 Hz), 7.15 (d, 2H, J = 8.5 Hz), 7.6-7.7 (m, 3H), 7.79 (s, 1H), 7.85 (d, 2H, J = 8.4 Hz), 9.86 (s, 1H), 12.49 (s, 1H). LCMS m/z [M+H]⁺ 443.4.

### Example 70

### 2-(6-aminopyridin-3-ylamino)-5-(4-methoxybenzamido)thiazole-4-carboxamide

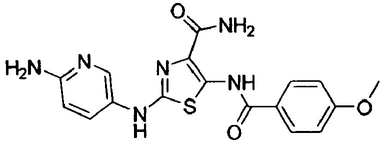

### (a) tert-butyl 5-isothiocyanatopyridin-2-ylcarbamate

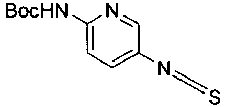

To a stirred solution of tert-butyl 5-aminopyridin-2-ylcarbamate (0.35 g, 1.67 mmol) in THF (20 mL) was added slowly triethylamine (0.70 mL, 5.02 mmol) and thiophosgene (0.13 mL, 1.67 mmol) at 0 °C, and the mixture was stirred for 40 min at rt. The reaction mixture was quenched with ice-water, and extracted with diethyl ether (70 mL). The organic layer was dried over Na₂SO₄ and concentrated in vacuo. The residual solids were filtered and washed with n-Hexane to afford 261 mg, (62% yield) of the titled compound.
¹H-NMR (400 MHz, CDCl₃) δ (ppm) 1.53 (s, 9H), 7.4-7.55 (m, 2H), 7.97 (d, 1H, J = 8.8 Hz), 8.17 (d, I H, J = 2.4 Hz).

### (b) tert-butyl 5-(5-amino-4-carbamoylthiazol-2-ylamino)pyridin-2-ylcarbamate

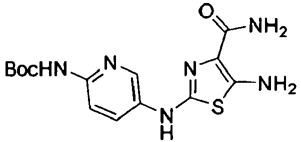

A mixture of tert-Butyl 5-isothiocyanatopyridin-2-ylcarbamate (261 mg, 1.04 mmol) and 2-amino- cyanoacetamide (103 mg, 1.04 mmol) in EtOAc (14 mL) was refluxed for 45 min. After cooling to rt, the resulting precipitates were filtered off, and the filtrate was concentrated in vacuo. The residue was purified with silica gel column chromatography eluted with 0-9% MeOH in CHCl₃ to afford the titled compound (219 mg, 60% yield).
¹H-NMR (400 MHz, DMSO-d₆) δ (ppm) 1.47 (s, 9H), 6.68 (s, 2H), 6.8-7.0 (m, 2H), 7.68 (d, 1 H, J = 8.8 Hz), 8.23 (dd, 1H, J = 8.8, 2.8 Hz), 8.28 (d, 1 H, J = 2.4 Hz), 9.44 (s, I H), 9.62 (s, I H).

### (c) tert-butyl 5-[5-(4-methoxybenzamido)-4-carbamoylthiazol-2-ylamino]pyridin-2-yl carbamate

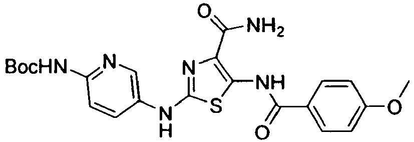

To a solution of tert-butyl 5-(5-amino-4-carbamoylthiazol-2-ylamino)pyridin-2-ylcarbamate (219 mg, 0.626 mmol) in pyridine (3 mL) was added 4-methoxybenzoyl chloride (0.089 mL, 0.657 mmol) at 0 °C. The mixture was stirred at rt overnight. The reaction mixture was diluted with EtOAc and washed with water, and dried over Na₂SO₄. The solvent was removed and the resulting solids were collected. The solids were washed with EtOAc and diethyl ether to afford the titled compound (219 mg, 72% yield).
¹H-NMR (400 MHz, DMSO-d₆) δ (ppm) 1.47 (s, 9H), 3.86 (s, 3H), 7.16 (d, 2H, J = 9.2 Hz), 7.75-7.85 (m, 2H), 7.87 (d, 2H, J = 8.8 Hz), 8.35-8.5 (m, 2H), 9.52 (s, I H), 10.13 (s, I H), 12.56 (s, I H). LCMS m/z [M+H]⁺ 485.0.

### (d) 2-(6-aminopyridin-3-ylamino)-5-(4-methoxybenzamido)thiazole-4-carboxamide

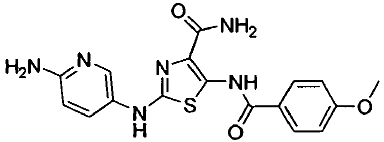

To a suspension of tert-butyl 5-[5-(4-methoxybenzamido)-4-carbamoylthiazol-2-ylamino]pyridin-2-ylcarbamate (100 mg, 0.206 mmol) in 1,4-Dioxane (5 mL) was added 4M HCl-Dioxane (5 mL) at 0 °C. and then the mixture was stirred at rt overnight. The reaction mixture was concentrated in vacuo. EtOAc and sat NaHCO₃ were added to the residual oil, and the resulting solids were collected by filtration. The solids were washed with EtOAc and water to afford the titled compound (50 mg, 63% yield).
¹H-NMR (400 MHz, DMSO-d₆) δ (ppm) 3.86 (s, 3H), 5.62 (s, 2H), 6.46 (d, 1H, J = 8.8 Hz), 7.15 (d, 2H, J = 8.8 Hz), 7.58 (br, 1H), 7.76 (br, 1H), 7.85 (d, 2H, J = 8.8 Hz), 7.92 (dd, 1H, J = 8.8, 2.8 Hz), 8.10 (d, 1H, J = 2.4 Hz), 9.59 (s, 1H), 12.48 (s, I H). LCMS m/z [M+H]⁺ 385.4.

### Example 79

Example 79 (Table 13) was prepared by following the procedure described for above Example 70 using appropriate starting materials.

**Table 13**

| Ex. No. | ¹H-NMR δ (ppm) | LCMS m/z [M+H]⁺ |
|---|---|---|
| 79 | (DMSO-d₆): 3.86 (s, 3H), 6.3-6.8 (m, 2H), 7.15 (d, 2H, J = 8.8 HZ), 7.75 (br, 2H), 7.85 (d, 2H, J = 8.8 Hz), 8.68 (br, 2H), 9.85 (br, 1H), 12.53 (s, 1H). | 385.9 |

### Example 80

### 5-(3-amino-4-methoxybenzamido)-2-(4-methoxyphenylamino)thiazole-4-carboxam ide

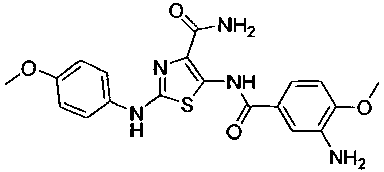

### (a) 5-(4-methoxy-3-nitrobenzamido)-2-(4-methoxyphenylamino)thiazole-4-carboxamid e

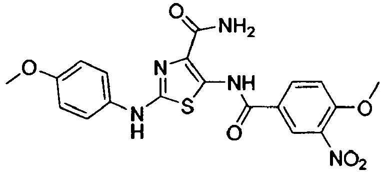

To a suspension of 4-methoxy-3-nitrobenzoic acid (112 mg, 0.569 mmol) in DCM (15 mL) was added oxalyl chloride (0.199 mL, 2.27 mmol) and a catalytic amount of DMF at 0 °C. The mixture was stirred for 4 h at rt. The solvent was evaporated, and the residual oxalyl chloride was removed with azeotropic distillation using toluene under nitrogen atmosphere. The resulting acid chloride was added to a solution of 5-amino-2-(4-methoxyphenylamino)- thiazole-4-carboxamide (100 mg, 0.378 mmol) in pyridine (2 mL) at 0 °C, and the mixture was stirred for overnight at rt. The reaction mixture was quenched by adding of ice-water and was diluted with EtOAc. The resulting solids were collected by filtration and washed with 50% EtOAc in Et₂O to afford the titled compound (98 mg, 58% yield).
¹H-NMR (400 MHz, DMSO-d₆) δ (ppm) 3.73 (s, 3H), 4.04 (s, 3H), 6.88 (d, 2H, J = 9.2 Hz), 7.61 (d, 1H, J = 8.8 Hz), 7.66 (d, 2H, J = 9.2 Hz), 7.68 (br, 1H), 7.86 (br, 1H), 8.13 (dd, 1H, J = 8.8, 2.4 Hz), 8.37 (d, 1H, J = 2.4 Hz), 9.91 (s, 1H), 12.64 (s, 1H).

### (b) 5-(3-amino-4-methoxybenzamido)-2-(4-methoxyphenylamino)thiazole-4-carboxam ide

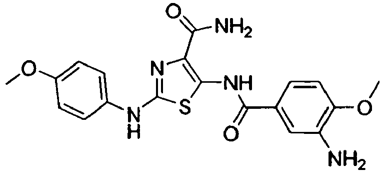

To a solution of 5-(4-methoxy-3-nitrobenzamido)-2-(4-methoxyphenylamino)thiazole-4-carboxamide (50 mg, 0.11 3 mmol) in EtOH-H₂O (12.5 mL, 4:1) was added Fe (63 mg, 1.13 mmol) and NH₄Cl (3.0 mg, 0.056 mmol), and the mixture was refluxed for I h. After cooling to rt, the insoluble material was filtered through a bed of Celite, and washed with EtOH and EtOAc. The filtrate was concentrated in vacuo, and the resulting solids were collected and washed with EtOAc to afford the titled compounds (25 mg, 54% yield).
¹H-NMR (400 MHz, DMSO-d₆) δ (ppm) 3.73 (s, 3H), 3.86 (s, 3H), 5.12 (s, 2H), 6.88 (d, 2H, J = 9.2 Hz), 6.97 (d, 1H, J = 8.4 Hz), 7.10 (dd, 1H, J = 8.4, 2.0 Hz), 7.21 (d, 1H, J = 2.0 Hz), 7.57 (br, 1 H), 7.65 (d, 2H, J = 9.2 Hz), 7.74 (br, 1 H), 9.83 (s, 1 H), 12.31 (s, 1H). LCMS m/z [M+H]⁺ 414.0.

### Example 83

### 5-(3-amino-4-fluorobenzamido)-2-(4-methoxyphenylamino)thiazole-4-carboxamide

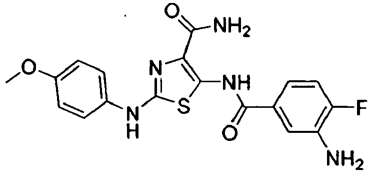

To a solution of 5-(4-fluoro-3-nitrobenzamido)-2-(4-methoxyphenylamino)thiazole-4-carboxamide (50 mg, 0.116 mmol) in EtOH-H₂O (10-2.5 mL) was added Fe (65 mg, 1.16 mmol) and NH₄Cl (3.1 mg, 0.058 mmol), and the mixture was refluxed for 1 h. After cooling to rt, the insoluble material was filtered through a bed of Celite, and washed with EtOH and EtOAc. The filtrate was concentrated in vacuo, and the resulting solids were collected and washed with EtOAc to afford the titled compounds (10 mg, 21% yield).
¹H-NMR (400 MHz, DMSO-d₆) δ (ppm) 3.73 (s, 3H), 5.59 (s, 2H), 6.88 (d, 2H, J = 8.8 Hz), 6.95-7.1 (m, 1H), 7.20 (dd, 1H, J = 10.8, 8.8 Hz), 7.34 (d, 1H, J = 6.4 Hz), 7.61 (br, 1H), 7.65 (d, 2H, J = 8.8 Hz), 7.78 (br, 1H), 9.84 (s, 1H), 12.39 (s, 1H). LCMS m/z [M+H]⁺ 401.9.

### Example 114 and 116

The compounds shown in the following Table 14 were prepared by following the procedure described for above Example 83 using appropriate starting materials.

**Table 14**

| Ex. No. | ¹H-NMR δ (ppm) | LCMS m/z [M+H]⁺ |
|---|---|---|
| 114 | (DMSO-d₆): 2.13 (s, 3H), 5.29 (s, 2H), 6.98 (br, 1H), 7.05-7.25 (m, 2H), 7.43 (br, 1H), 7.53 (br, 1H), 7.82 (br, 1H), 7.9-8.1 (m, 2H), 10.81 (s, 1H), 12.50 (s, 1H). | 386.9 |
| 116 | (DMSO-d₆): 6.09 (s, 2H), 6.67 (d, 2H, J=8.5 Hz), 7.43 (d, 1H, J=5.8 Hz), 7.52 (s, 1H), 7.60 (d, 2H, J=8.5 Hz), 7.76 (s, 1H), 7.94 (s, 1H), 7.99 (d, 1H, J=5.8 Hz), 10.78 (s, 1H), 12.40 (s, 1H) | 372.8 |

### Example 91

Example 91 (Table 15) was synthesized according to the procedure described in the following Example 94 using appropriate starting materials.

**Table 15.**

| Ex. No. | ¹H-NMR δ (ppm) | LCMS m/z [M+H]⁺ |
|---|---|---|
| 91 | (DMSO-d₆): 3.65-3.75 (m, 2H), 3.86 (s, 3H), 3.9-4.0 (m, 2H), 4.83 (br, 1H), 6.88 (d, 2H, J = 8.5 Hz), 7.15 (d, 2H, J = 8.4 Hz), 7.6-7.7 (m, 3H), 7.79 (s, 1H), 7.85 (d, 2H, J = 8.4 Hz), 9.84 (s, 1H), 12.49 (s, 1H). | 427.2 |

### Example 94

### 5-(4-methoxybenzamido)-2-[4-(prop-2-ynyloxy)phenylamino]thiazole-4-carboxami de

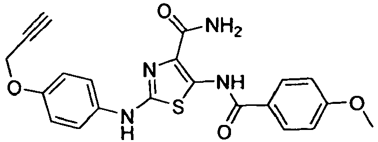

### (a) 1-nitro-4-(prop-2-ynyloxy)benzene

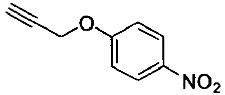

A mixture of 4-nitrophenol (2.00 g, 14.28 mmol), propargyl bromide (1.33 mL, 15.10 mmol) and 0.8 M NaOH (18 mL) and tetra-n-butylammonium bromide (0.46 g, 1.44 mmol) in toluene (8 mL) was stirred at 60°C for 24 h. The reaction mixture was cooled to room temperature, and the resulting solids were filtered. The solids were dissolved in dioxane, and water was added to this solution. The resulting solids were filtered and washed with water to give 1.75 g (68% yield) of the titled compound.
¹H-NMR (400 MHz, CDCl₃) δ (ppm) 2.58 (s, I H), 4.79 (d, 2H, J = 1.4 Hz), 7.05 (d, 2H, J = 9.2 Hz), 8.22 (d, 2H, J = 9.2 Hz).

### (b) 4-(prop-2-ynyloxy)benzenamine

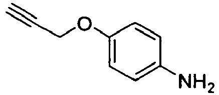

To a solution of 1-nitro-4-(prop-2-ynyloxy)benzene (1.0 g, 5.6 mmol) in MeOH (25 mL) was added 10% palladium on carbon catalyst (0.1 g) at rt, and the mixture was stirred under H₂ gas for 16 h. The reaction mixture was filtered through a bed of Celite. The filtrate was concentrated to give 0.65 g (78% yield) of the titled compound.
¹H-NMR (400 MHz, DMSO-d₆) δ (ppm) 2.50 (s, I H), 4.59 (s, 2H), 6.48 (d, 2H, J = 8.7 Hz), 8.62 (d, 2H, J = 9.5 Hz).

### (c) 1-isothiocyanato-4-(prop-2-ynyloxy)benzene

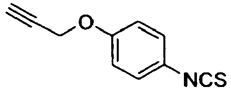

To a mixture of 4-(prop-2-ynyloxy)benzenamine (0.45 g, 3.06 mmol) and triethylamine (0.6 mL, 6.13 mmol) in DCM (25 mL) was added dropwise thiophosgene (0.28 mL, 3.67 mmol) at 0 °C, and the mixture was stirred at rt for 90 min. The reaction mixture was quenched with water and extracted with DCM (2 x 100 mL). The combined organic layers were dried over Na₂SO₄ and concentrated to give 0.51 g (88% yield) of the titled compound.
¹H-NMR (400 MHz, CDCl₃) δ (ppm) 2.52 (s, I H), 4.68 (d, 1 H, J = 1.2 Hz), 6.92 (d, 2H, J = 8.6 Hz), 7.17 (d, 2H, J = 8.6 Hz).

### (d) 5-amino-2-[4-(prop-2-ynyloxy)phenylamino]thiazole-4-carboxamide

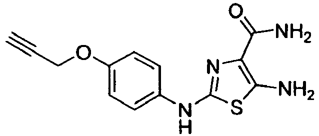

A mixture of 1-isothiocyanato-4-(prop-2-ynyloxy)benzene (0.51 g, 2.73 mmol) and 2-amino-2-cyanoacetamide (0.27 g, 2.73 mmol) in EtOAc (15 mL) was refluxed for 3 h. The reaction mixture was concentrated, and the residue was purified by silica gel chromatography (eluent: 5% MeOH in DCM) to afford the titled compound (0.2 g, 36 % yield).
¹H-NMR (400 MHz, DMSO-d₆) δ (ppm) 3.53 (s, 1H), 3.6-3.7 (m, 2H), 4.0-4.1 (m, 2H), 6.60 (br, 1H), 6.75-6.90 (m, 4H), 7.49 (d, 2H, J = 8.7 Hz), 9.33 (s, 1H). LCMS m/z [M+H]⁺ 289.0.

### (e) 5-(4-methoxybenzamido)-2-[4-(prop-2-ynyloxy)phenylamino]thiazole-4-carboxami de

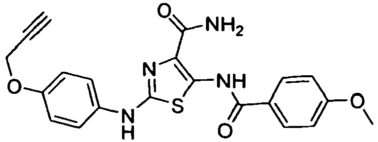

To a solution of p-anisoyl chloride (0.179 g, 1.05 mmol) in pyridine (5 mL) was added 5-amino-2-[4-(prop-2-ynyloxy)phenylamino]thiazole-4-carboxamide (0.2 g, 0.70 mmol) in pyridine (5 mL) at 0°C, the mixture was stirred for 3 h at 0°C. The reaction mixture was concentrated, and the residue was dissolved in 1M HCl (10 mL) and the resulting solids were filtered. The solids were washed with AcOEt (10 mL) and MeOH (10 mL), and dried to afford the titled compound (0.2 g, 68% yield).
¹H-NMR (400 MHz, DMSO-d₆) δ (ppm) 3.55 (s, 1H), 3.85 (s, 3H), 4.74 (s, 2H), 6.93 (d, 2H, J = 8.7 Hz), 7.15 (d, 2H, J = 8.5 Hz), 7.6-7.7 (m, 3H), 7.79 (s, 1H), 7.85 (d, 2H, J = 8.4 Hz), 9.91 (s, 1H), 12.51 (s, 1H). LCMS m/z [M+H]⁺ 423.0.

### Example 103

### 5-(3,4-diaminobenzamido)-2-(4-methoxyphenylamino)thiazole-4-carboxamide

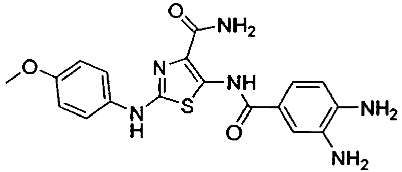

### (a) 5-(4-amino-3-nitrobenzamido)-2-(4-methoxyphenylamino)thiazole-4-carboxamide

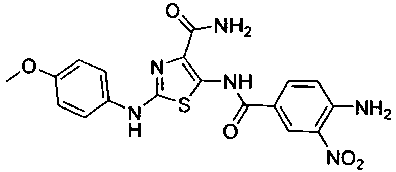

A mixture of 5-(4-fluoro-3-nitrobenzamido)-2-(4-methoxyphenylamino)thiazole-4-carboxamide (35 mg, 0.081 mmol) and 28% NH₃ aq. (0.7 mL)in DMA (0.3 mL) was treated using a microwave synthesizer for 30 min (Biotage, 60°C). The reaction mixture was diluted with water, and the resulting solids were collected and washed with water to afford the titled compound (30 mg, 86% yield).
¹H-NMR (400 MHz, DMSO-d₆) δ (ppm) 3.73 (s, 3H), 6.88 (d, 2H, J = 9.2 Hz), 7.16 (d, 1H, J = 6.8 Hz), 7.5-7.7 (m, 3H), 7.75-7.9 (m, 2H), 8.02 (br, 2H), 8.55 (d, 1H, J = 2.0 Hz), 9.86 (s, 1H), 12.55 (s, 1H).

### (b) 5-(3,4-diaminobenzamido)-2-(4-methoxyphenylamino)thiazole-4-carboxamide

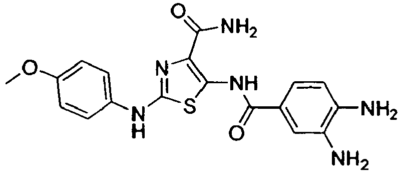

To a solution of 5-(4-amino-3-nitrobenzamido)-2-(4-methoxyphenylamino)thiazole-4-carboxamide (30 mg, 0.070 mmol) in EtOH-H₂O (10-2.5 mL) was added Fe (39 mg, 0.70 mmol) and NH₄Cl (1.9 mg, 0.035 mmol), and the mixture was refluxed for 3 h. After cooling to rt, the insoluble material was filtered through a bed of Celite, and washed with EtOH and EtOAc. The filtrate was concentrated in vacuo, and the resulting solids were collected and washed with EtOAc and water to afford the titled compounds (15 mg, 52 %).
¹H-NMR (400 MHz, DMSO-d₆) δ (ppm) δ 3.72 (s, 3H), 5.35 (br, 2H), 6.61 (d, 1H, J = 8.4 Hz), 6.88 (d, 2H, J = 8.8 Hz), 7.01 (d, 1 H, J = 8.4 Hz), 7.12 (d, 1H, J = 2.0 Hz), 7.51 (br, 1H), 7.64 (d, 2H, J = 8.8 Hz), 7.68 (br, 1H), 9.76 (s, 1H), 12.16 (s, 1H). LCMS m/z [M+H]⁺ 398.9.

### Example 117-119, 123-126 and 129

The compounds shown in the following Table 16 were prepared by following the procedure described for above Example 103 using appropriate starting materials.

**Table 16**

| Ex. No. | ¹H-NMR δ (ppm) | LCMS m/z [M+H]⁺ |
|---|---|---|
| 117 | (DMSO-d₆): 2.2-2.7 (m, 3H), 2.7-3.5 (m, 8H), 3.72 (s, 3H), 6.88 (d, 2H, J = 8.6 Hz), 7.0-7.15 (m, 2H), 7.26 (s, 1H), 7.60 (br, 1H), 7.65 (d, 2H, J = 8.6 Hz), 7.76 (br, 1H), 9.85 (s, 1H), 12.35 (s, 1H). | 482.6 |
| 118 | (DMSO-d₆): 1.2-1.8 (m, 6H), 2.2-3.5 (m, 8H), 3.72 (s, 3H), 4.85 (br, 2H), 5.1-5.4 (m, 1H), 6.56 (br, 1H), 6.87 (d, 2H, J = 8.4 Hz), 7.05-7.2 (m, 2H), 7.51 (br, 1H), 7.64 (d, 2H, J = 8.4 Hz), 7.68 (br, 1H), 9.76 (s, 1H), 12.19 (s, 1H). | 510.2 |
| 119 | (DMSO-d₆): 3.1-3.5 (m, 2H), 3.5-3.7 (m, 2H), 3,72 (s, 3H), 4.75 (br, 1H), 4.88 (s, 2H), 5.27 (br, 1H), 6.55 (d, 1H, J = 8.2 Hz), 6.87 (d, 2H, J = 8.0 Hz), 7.04-7.2 (m, 2H), 7.51 (br, 1H), 7.64 (d, 2H, J = 8.0 Hz), 7.89 (br, 1H), 9.76-(s, 1H), 12.18 (s, 1H). | 443.5 |
| 123 | (DMSO-d₆): 2.77(s, 3H), 2.8-3.8 (m, 8H), 5.26 (s, 2H), 7.05-7.15 (m, 2H), 7.28 (s, 1H), 7.45 (br, 1H), 7.53 (s, 1H), 7.81 (br, 1H), 7.95-8.05 (m, 2H), 10.87 (s, 1H), 12.51 (s, 1H). | 471.2 |
| 124 | (DMSO-d₆): 1.3-1.7 (m, 6H), 2.2-2.7 (m, 6H), 3.1-3.6 (m, 2H), 4.89 (br, 2H), 5.27 (br, 1H), 6.56 (d, 1H, J = 7.2 Hz), 7.0-7.25 (m, 2H), 7.43 (br, 1H), 7.52 (s, 1H), 7.75 (br, 1H), 7.93 (br, 1H), 7.99(d, 1H, J = 5.5 Hz), 10.76 (s, 1H), 12.34 (s, 1H). | 499.4 |
| 125 | (DMSO-d₆): 3.1-3.5 (m, 2H), 3.55-3.7 (m, 2H), 4.75 (br, 1H), 4.91 (s, 2H), 5.32 (br, 1H), 6.56 (d, 1H, J = 8.9 Hz), 7.05-7.2 (m, 2H), 7.43 (br, 1H), 7.52 (s, 1H), 7.75 (br, 1H), 7.92 (br, 1H), 7.95-8.05 (m, 1H), 10.75 (s, 1H), 12.34 (s, 1H). | 432.4 |
| 126 | (DMSO-d₆): 2.72 (s, 6H), 3.0-3.6 (m, 4H), 4.97 (br, 2H), 5.50 (br, 1H), 6.65 (d, 1H, J = 7.8 Hz), 7.1-7.35 (m, 2H), 7.44 (br, 1H), 7.51 (s, 1H), 7.74 (br, 1H), 7.93 (br, 2H), 7.99 (d, 1H, J = 5.3 Hz), 10.61 (s, 1H), 12.37 (s, 1H). | 459.0 |
| 129 | (DMSO-d₆): 2.3-2.6 (m, 6H), 2.6-3.1 (m, 2H), 3.1-3.5 (m, 2H), 3.72 (s, 3H), 4.90 (br, 2H), 5.2-5.4 (m, 1H), 6.60 (d, 1H, J = 7.6 Hz), 6.87 (d, 2H, J = 8.5 Hz), 7.05-7.2 (m, 2H), 7.53 (br, 1H), 7.64 (d, 2H, J = 8.6 Hz), 7.70 (br, 1H), 9.78 (s, 1H), 12.21 (s, 1H). | 470.6 |

### Example 120

### 5-[3-amino-4-(2-hydroxyethoxy)benzamido]-2-(4-methoxyphenylamino)thiazole-4-carboxamide

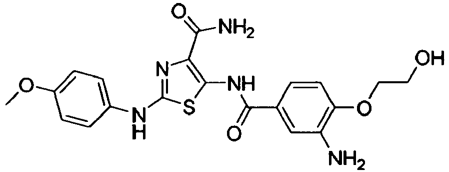

### (a) 5-[4-(2-hydroxyethoxy)-3-nitrobenzamido]-2-[(4-methoxyphenyl)amino]thiazole-4-carboxamide

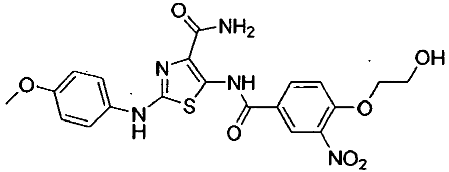

A mixture of 5-(4-fluoro-3-nitrobenzamido)-2-[(4-methoxyphenyl)amino]thiazole-4-carboxamide (0.1 g, 0.23 mmol), ethylene glycol (0.04 mL, 0.69 mmol) and K₂CO₃ (0.32 g, 2.3 mmol) in NMP (2 mL) was treated using a microwave synthesizer at 120 °C for 45 min. The reaction mixture was diluted with water (5 mL), and the resulting solids were filtered and washed successively with ether (20 mL) and EtOAc (20 mL) to afford the titled compound (0.038 g, 35% yield).
¹H-NMR (400 MHz, DMSO-d₆) δ (ppm) 3.65-3.8 (m, 5H), 4.2-4.4 (m, 2H), 6.88 (d, 2H, J = 8.8 Hz), 7.55-7.75 (m, 4H), 7.88 (br, 1H), 8.08 (d, 1H, J = 9.2 Hz), 8.35 (s, I H), 9.92 (s, 1H), 12.63 (s, I H). LCMS m/z [M+H]⁺ 474.2.

### (b) 5-[3-amino-4-(2-hydroxyethoxy)benzamido]-2-(4-methoxyphenylamino)thiazole-4-carboxamide

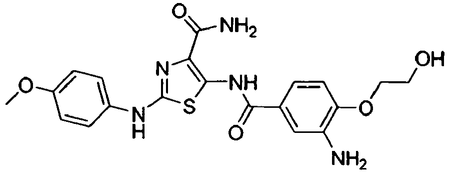

To a solution of 5-[4-(2-hydroxyethoxy)-3-nitrobenzamido]-2-[(4-methoxyphenyl)amino]thiazole-4-carb oxamide (0.09 g, 0.19 mmol) in THF-MeOH (1:1, 30 mL) was added 10% palladium on carbon catalyst (0.01 g) at rt, and the mixture was stirred under H₂ gas for 12 h. The reaction mixture was filtered through a bed of Celite. The filtrate was concentrated to give 0.005 g (6% yield) of the titled compound.
¹H-NMR (400 MHz, DMSO-d₆) δ (ppm) 3.7-3.9 (m, 5H), 3.9-4.1 (m, 2H), 6.88 (d, 2H, J = 7.5 Hz), 6.95 (d, 1H, J = 8.0 Hz), 7.0-7.1 (m, 2H), 7.15 (s, 1H), 7.20 (s, 1H), 7.28 (br, 1H), 7.59 (br, 1H), 7.65 (d, 2H, J = 8.2 Hz), 7.76 (br, I H), 9.85 (s, I H), 12.32 (s, 1H). LCMS m/z [M+H]⁺ 444.4.

### Example 128

Example 128 (Table 17) was prepared by following the procedure described for above Example 120 using appropriate starting materials.

**Table 17**

| Ex. No. | ¹H-NMR δ (ppm) | LCMS m/z [M+H]⁺ |
|---|---|---|
| 128 | (DMSO-d₆): δ 3.65-3.8 (m, 2H), 3.9-4.1 (m, 2H), 4.95 (br, 1H), 5.27 (br, 2H), 6.96 (d, 1H, J = 8.6 Hz), 7.09 (d, 1H, J = 7.7 Hz), 7.20 (s, 1H), 7.35-7.47 (m, 1H), 7.52 (s, 1H), 7.75-7.85 (m, 1H), 7.9-8.05 (m, 2H), 10.79 (s, 1H), 12.47 (s, 1H). | 433.2 |

### Example 127

### 5-[3-amino-4-(2-methoxyethoxy)benzamido]-2-(2-fluoropyridin-4-ylamino)thiazole -4-carboxamide

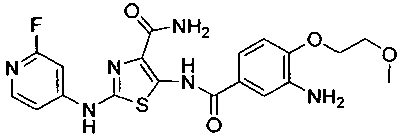

### (a) 4-(2-methoxyethoxy)-3-nitrobenzoic acid

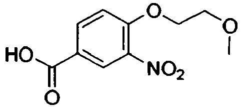

A solution of 2-methoxy ethanol (0.95 g, 12.5 mmol) in DMF (3 mL) was added slowly to a suspension of NaH (0.64 g, 60%) in DMF (3 mL) at 0 °C, and the mixture was stirred at rt for 1h. Then, a solution of 4-fluoro-3-nitrobenzoic acid (1 g, 5.4 mmol) in DMF (4 mL) was added dropwise to this mixture at 0 °C, and the stirring was continued at rt for 16 h. The reaction mixture was quenched with cold 1M HCl and extracted with EtOAc (3x100 mL). The combined organic layers were washed successively with water and brine, and dried over Na₂SO₄. The solvent was removed, and the residue was purified by silica gel chromatography (eluent: 30% EtOAc in hexane) to afford the titled compound (1 g, 76% yield).
¹H-NMR (400 MHz, DMSO-d₆) δ (ppm) 3.31 (s, 3H), 3.65-3.75 (m, 2H), 4.3-4.45 (m, 2H), 7.48 (d, 1H, J = 8.8 Hz), 8.15 (dd, I H, J = 8.8, 1.8 Hz), 8.33 (d, 1H, J = 1.9 Hz), 13.26 (br, I H). LCMS m/z [M+H]⁺ 242.2.

### (b) 5-[4-(2-methoxyethoxy)-3-nitrobenzamido]-2-(2-fluoropyridin-4-ylamino)thiazole-4-carboxamide

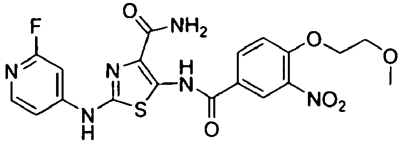

To a mixture of 4-(2-methoxyethoxy)-3-nitrobenzoic acid (0.2 g, 0.82 mmol) and catalytic amount of DMF in dry DCM (15 mL) was added dropwise oxalyl chloride (1 mL) at 0 °C, and the mixture was stirred for 2 h at rt. The solvent was evaporated, and the residual oxalyl chloride was removed with azeotropic distillation using toluene under nitrogen atmosphere. The resulting acid chloride was then dissolved in pyridine (15 mL) and cooled to 0 °C. To this solution, a solution of 5-amino-2-[(2-fluoropyridin-4-yl)amino]thiazole-4-carboxamide (0.17 g, 0.066 mmol) in pyridine (10 mL) was added at 0 °C, and the mixture was stirred for 12 h at rt. After removal of the solvent, ice-water was added to the residue, and the resulting solids were collected. The solids were washed with MeOH to give 35 mg (9% yield) of the titled compound.
¹H-NMR (400 MHz, DMSO-d₆) δ (ppm) 3.2-3.4 (m, 3H), 3.65-3.8 (m, 2H), 4.35-4.5 (m, 2H), 7.44 (br, 1H), 7.53 (s, 1H), 7.62 (d, 1H, J = 8.8 Hz), 7.90 (br, I H), 8.00 (d, 1H, J = 5.6 Hz), 8.06 (br, 1H), 8.11 (d, 1H, J = 8.7 Hz), 8.37 (s, 1H), 10.84 (s, I H), 12.76 (s, 1H). LCMS m/z [M+H]⁺ 477.0.

### (c) 5-[3-amino-4-(2-methoxyethoxy)benzamido]-2-(2-fluoropyridin-4-ylamino)thiazole -4-carboxamide

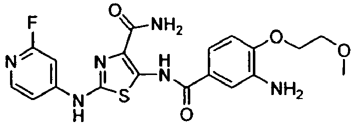

To a solution of 5-[4-(2-Methoxyethoxy)-3-nitrobenzamido]-2-(2-fluoropyridin-4-ylamino)thiazole-4-ca rboxamide (0.03 g, 0.063 mmol) in THF-MeOH (1:1,40 mL) was added 10% palladium on carbon catalyst (0.01 g) at rt, and the mixture was stirred under H₂ gas for 12 h. The reaction mixture was filtered through a bed of Celite. The filtrate was concentrated to give 0.01 g (36% yield) of the titled compound.
¹H-NMR (400 MHz, DMSO-d₆) δ (ppm) 3.34 (s, 3H), 3.7-3.8 (m, 2H), 4.25-4.35 (m, 2H), 7.28 (d, I H, J = 8.6 Hz), 7.47 (d, 1H, J = 5.1 Hz), 7.5-7.6 (m, 2H), 7.66 (s, 1H), 7.85 (br, 1H), 7.99 (d, 1H, J = 5.7 Hz), 8.03 (br, 1H), 11.03 (s, 1H), 12.61 (s, 1H). LCMS m/z [M+H]⁺ 447.5.

### EXAMPLE 130

### Preparation of Tablets:

Tablets each containing 100mg of 5-(4-acetamidobenzamido)-2-(phenylamino)thiazole-4-carboxamide (Compound 1) are obtained by the following procedure.

**Table 18**

| Formulation: | |
|---|---|
| Ingredients | Amount |
| Compound 1 | 100 parts by weight |
| Cornstarch | 46 parts by weight |
| Microcrystalline cellulose | 98 parts by weight |
| Hydroxypropyl cellulose | 2 parts by weight |
| Magnesium stearate | 4 parts by weight |

### Procedure:

Compound 1, cornstarch and microcrystalline cellulose are mixed and the mixture is added to hydroxypropyl cellulose dissolved in 50 parts by weight of water, followed by sufficient kneading. The kneaded mixture is passed through a sieve to granulate, dried mixed with magnesium stearate and then compressed into tablets of 250mg each.

### EXAMPLE 131

### Preparation of Granules:

Granules containing 5-(4-acetamidobenzamido)-2-(phenylamino) thiazole-4-carboxamide (Compound I) are obtained by the following procedure.

**Table 19**

| Formulation: | |
|---|---|
| Ingredients | Amount |
| Compound 1 | 200 parts by weight |
| Lactose | 185 parts by weight |
| Cornstarch | 109 parts by weight |
| Hydroxypropyl cellulose | 6 parts by weight |

### Procedure:

Compound 1, lactose and cornstarch are mixed and the mixture is added to hydroxypropyl cellulose dissolved in 120 parts by weight of water, followed by sufficient kneading. The kneaded mixture is passed through a 20 mesh sieve to granulate, dried and then size-adjusted to obtain granules containing 200mg of Compound 1 per 500mg of granule.

### EXAMPLE 132

### Preparation of Capsules:

Capsules each containing 100mg of 5-(4-acetamidobenzamido)-2-(phenylamino)thiazole-4-carboxamide (Compound 1) are obtained by the following procedure.

**Table 20**

| Formulation: | |
|---|---|
| Ingredients | Amount |
| Compound 1 | 100 parts by weight |
| Lactose | 35 parts by weight |
| Cornstarch | 60 parts by weight |
| Magnesium stearate | 5 parts by weight |

### Procedure:

Compound 1, lactose, cornstarch and magnesium stearate are well mixed and 200mg each of the powder mixture is encapsulated to obtain capsules.

The followings are details of referential descriptions about evaluation methods for utility of the present invention mentioned in "BACKGROUND OF THE INVENTION"

### REFERENTIAL METHODS

### METHODS SUMMARY:

The methodological details of immunoprecipitation, immunoblotting, immunofluorescence microscopy, immunohistochemistry, mass spectrometry, two-hybrid assay, luciferase reporter assay, colony formation, real-time RT-PCR, axis formation assay and animal cap assay are available in FULL METHODS. All antibodies used in this study are commercially available. Mouse and *Xenopus* experiments were carried out according to the guidelines of the National Cancer Center Research Institute (Tokyo, Japan), which meet all the ethical requirements stipulated by Japanese law. The minimum number of mice necessary for obtaining reliable results were used and euthanatized. Patients submitted written informed consent authorizing the collection and use of their materials for research purposes. The experimental protocols were reviewed and approved by the institutional ethics and recombination safety committees.

### FULL METHODS:

### Cell lines:

The human embryonic kidney cell line HEK293 and the human colorectal cancer cell line DLD1 were obtained from the Health Science Research Resources Bank (Osaka, Japan). The human cervical cancer cell line HeLa was obtained from the Riken Cell Bank (Tsukuba, Japan). Human colorectal cancer cell lines HCT-116 and WiDr were purchased from the American Type Culture Collection (Rockville, MD).

### Immunoprecipitation:

Total cell lysates were prepared as described previously. The lysates were incubated at 4°C overnight with the indicated antibody or relevant control IgG and precipitated with Dynabeads protein G (Dynal Biotech, Oslo, Norway).

### Immunoblot analysis:

Protein samples were fractionated by SDS-PAGE and blotted onto Immobilon-P membranes (Millipore, Billerica, MA). After incubation with the primary antibodies at 4°C overnight, the blots were detected with the relevant horseradish peroxidase-conjugated anti-mouse or anti-rabbit IgG antibody and ECL Western blotting detection reagents (GE Healthcare, Giles, UK). Blot intensity was quantified using a LAS-3000 scanner and Science Lab 2003 software (Fuji Film, Tokyo, Japan).

### Mass spectrometry (MS):

Protein bands in SDS-PAGE gels were visualized by Coomassie blue staining and digested using modified trypsin (Promega) as described previously. The tryptic peptides were concentrated and desalted with a 500-µm i.d. × 1 mm HiQ sil C18-3 trapping column (KYA Technologies, Tokyo, Japan). The peptides were then fractionated with a 0-80% acetonitrile gradient (200 nL/minute for 1 hour) using a 150-µm i.d. × 5 cm C18W-3 separation column (KYA) and analysed with a Q-Star Pulsar-i mass spectrometer equipped with a nanospray ionization source (Applied Biosystems, Foster City, CA). Reliability of protein identification was estimated by calculating the Confidence Value using ProID software (Applied Biosystems).

### Plasmids:

Human TNIK (Traf2- and Nck-interacting kinase) expression constructs [pCIneoHA-TNIK and its mutant (K54R)] were kindly provided by Drs. M. Umikawa and K. Kariya (University of the Ryukyus, Nishihara-cho, Japan). cDNA sequences encoding different parts of TNIK protein were subcloned into pBind (Promega, Madison, WI). Human TCF4 (T-cell factor-4) (splice form E) and β-catenin cDNA lacking the 134-amino-acid sequence in its NH₂-terminus were subcloned into pFLAG-CMV4 (Sigma-Aldrich, St. Louis, MO). Full-length human TCF4 cDNA and its truncated forms were subcloned into pAct (Promega). The mutant form of TCF4, designated as TCF4S154A, was constructed using a QuikChange mutagenesis kit (Stratagene, La Jolla, CA) with oligos GGCCCCATCACCGGCACACATTGTCTCTA ([SEQ ID NO.4]) and GGGGCATCCTTGAGGGCTTGTCTACTCTG ([SEQ ID NO.5]) to change the serine (S) 154 residue to alanine (A). Full-length human TCF4 and TCF4S154A cDNAs were subcloned into pEU-E01-MCS (CellFree Science, Matsuyama, Japan).

### Mammalian two-hybrid assay:

Physical interactions between the TNIK and TCF4 proteins were assessed using the CheckMate Mammalian Two-hybrid system (Promega), according to instructions provided by the supplier. HEK293 cells were co-transfected in triplicate with pBind, pAct and pG5luc (Promega) plasmids using the Lipofectamine 2000 reagent (Invitrogen, Carlsbad, CA).

### Nuclear protein extraction:

Nuclear proteins were extracted using NE-PER Nuclear and Cytoplasmic Extraction Reagents (Pierce, Rockford, IL).

### Recombinant protein production:

Glutathione S-transferase (GST)-fusion proteins were synthesized using the ENDEXT Wheat Germ Expression Kit (CellFree Sciences, Matsuyama, Japan). HA (hemagglutinin)-tagged recombinant TNIK proteins were synthesized using rabbit reticulocyte lysate (TnT T7 Quick Coupled Transcription/Translation System) (Promega, Madison, WI).

### Immunofluorescence cytochemistry:

Cells cultured on glass coverslips (Asahi Technoglass, Tokyo, Japan) were fixed with 4% paraformaldehyde at room temperature for 10 minutes and permeabilized with 0.2% Triton X-100. After blocking with 10% normal swine serum (Vector Laboratories, Burlingame, CA), the cells were incubated with primary antibodies at 4°C overnight and subsequently with Alexa fluor-594 anti-mouse and Alexa fluor-488 anti-rabbit antibodies (Invitrogen). The specimens were examined with a laser scanning microscope (LSM5 PASCAL; Carl Zeiss, Jena, Germany).

### RNA interference:

Two small interfering RNAs (siRNAs), TNIK-J-004542-12 (sense: [SEQ ID NO.6] 5'-CGACAUACCCAGACUGAUAUU-3'; antisense: [SEQ ID NO.7] 5'-PUAUCAGUCUGGGUAUGUCGUU-3') and TNIK-J-004542-13 (sense: [SEQ ID NO.8] 5'-GACCGAAGCUCUUGGUUACUU-3'; antisense: [SEQ ID NO.9] 5'-PGUAACCAAGAGCUUCGGUCUU-3'), were synthesized and annealed by Dharmacon (Chicago, IL). Two control RNAs (X and IX) were purchased from Dharmacon.

The letters "T" described in next column, mean "U". For convenience, sometimes "T" is used instead of "U" in RNA sequence.

The SureSilencing short hairpin (sh)RNA plasmid for human TNIK (T1, [SEQ ID NO.10] ACACACTGGTTTCCATGTAAT; T2, [SEQ ID NO.11] AGAGAAGGAACCTTGATGATT; T3, [SEQ ID NO.12] AGAAAGATTTCGGTGGTAAAT) and negative control (C, [SEQ ID NO.13] GGAATCTCATTCGATGCATAC) were purchased from SuperArray Bioscience (Frederick, MD).

### Luciferase reporter assay:

A pair of luciferase reporter constructs, TOP-FLASH and FOP-FLASH (Upstate, Charlottesville, VA), were used to evaluate TCF/LEF (lymphoid enhancer factor) transcriptional activity. Cells were transiently transfected in triplicate with one of the luciferase reporters and phRL-TK (Promega). Luciferase activity was measured with the Dual-luciferase Reporter Assay system (Promega) using *Renilla reniformis* luciferase activity as an internal control.

### Colony formation assay:

Twenty four hours after transfection, 750, 400, 300 and 1000 µg/ml G418 (Geneticin, Invitrogen) was added to the culture media of HEK293, HeLa, DLD1 and HCT-116 cells, respectively. Cells were stained with Giemsa solution (Wako, Osaka, Japan) after selection for 8 days.

### Real-time RT-PCR:

Total RNA was prepared with an RNeasy Mini Kit (Qiagen, Valencia, CA). DNase-I-treated RNA was random-primed and reverse-transcribed using SuperScript II reverse transcriptase (Invitrogen). The TaqMan universal PCR master mix and pre-designed TaqMan Gene Expression probe and primer sets were purchased from Applied Biosystems. Amplification data measured as an increase in reporter fluorescence were collected using the PRISM 7000 Sequence Detection system (Applied Biosystems). mRNA expression level relative to the internal control [β-actin (*ACTB*) for human or ornithine decarboxylase (*odc*) for *Xenopus*] was calculated by the comparative threshold cycle (C_{T}) method.

### Mouse experiments:

5×10⁶ HCT-116, DLD1 or WiDr cells suspended in 0.1 ml of PBS were subcutaneously inoculated into the flanks of 5-week-old female BALB/c nu/nu nude mice (SLC, Tokyo, Japan). One week later the developed tumours were treated with siRNA together with atelocollagen (AteloGene; KOKEN, Tokyo, Japan) (Takeshita, F. et al. Efficient delivery of small interfering RNA to bone-metastatic tumors by using atelocollagen in vivo. Proc Natl Acad Sci U S A 102, 12177 (2005)) . The final concentration of siRNA was 30 µM and that of atelocollagen was 0.5%. A 0.2-ml volume of siRNA solution was injected directly into each tumour. Tumour volume was determined as V=A×B²π/6, where A and B represent the largest and smallest dimensions (Bergers, G. et al. Effects of angiogenesis inhibitors on multistage carcinogenesis in mice. Science 284, 808 (1999)) .

### Immunohistochemistry:

Fifty cases of sporadic colorectal cancer were selected from the pathology archive panel of the National Cancer Center Hospital (Tokyo, Japan). Immunoperoxidase staining was performed using the avidin-biotin complex method as described previously.

### Xenopus experiments:

Preparation of *Xenopus laevis* embryos and microinjection have been described previously. Eggs were fertilized *in vitro* and dejellied with 1% sodium thioglycolate solution. Embryos were staged according to Niewkoop and Faber. Capped mRNA was synthesized by *in-vitro* transcription (mMESSAGE mMACHINE kit; Ambion, Austin, TX). Injection was performed in Steinberg's solution containing 5% Ficoll.

pCS2-FLAG-Xenopus β-catenin (Xβ-catenin) was kindly provided by Dr. S. Sokol (Mount Sinai School of Medicine, New York, NY) and pCS2-nβ-Gal was kindly provided by Drs. D. Turner, R. Rupp and J. Lee (Fred Hutchinson Cancer Research Center, Seattle, WA). pCS2+-Myc and pCS2+-HA were kindly provided by Dr. M. Taira (University of Tokyo, Tokyo, Japan).

The open reading frame (ORF) and 5'-untranslated region (5'UTR) of LOC443633 (XTNIK), which are recognized by antisense morpholino oligonucleotide (MO) XTNIK-MOI or MO3, were PCR-amplified from the *Xenopus laevis* IMAGE cDNA clone MXL1736-98358477 (Open Biosystems, Huntsville, AL) and subcloned into pCS2+-Myc (pCS-XTNIK-WT-Myc). The mutant form of pCS-XTNIK-WT-Myc (pCS-XTNIK-K54R-Myc) was constructed by mutagenesis with oligos [SEQ ID NO.14] AGGGTCATGGATGTCACAGGGGATG and [SEQ ID NO.15] AATAGCTGCAAGCTGTCCGGTTTTAAC to change the lysine (K) 54 residue to arginine (R).

The ORF sequence of XTNIK, which is not recognized by XTNIK-MO1 or MO3, was constructed by mutagenesis with oligos [SEQ ID NO.16] ATGGCcAGtGAtTCtCCGGCTCGTAGCCTGGATGA (small letters indicate modifications) and [SEQ ID NO.17] ATCGATGGGATCCTGCAAAAAGAACAA (pCS2-XTNIK_{ORF}-HA).

### Antisense morpholino oligonucleotides (MOs):

The antisense MOs for *Xenopus* TNIK (XTNIK-MO1 and -MO3) and the corresponding control MOs [carrying 5 nucleotide substitutions within XTNIK-MO1 and -MO3 sequences (5mis-Controls-1 and -3)] were obtained from Gene Tools (Philomath, OR). A database search confirmed the absence of a significant homologous sequence to the complements of XTNIK-MOI and -MO3 in *Xenopus laevis.* The sequences of MOs used in this study were: XTNIK-MO1 ([SEQ ID NO.18]; 5'-GGGAGTCGCTCGCCATGTTTCCTTT-3'), XTNIK-MO3 ([SEQ ID NO.19]; 5'-CCCCGTTCTTTCCACCTTGCGGCTG-3'), 5mis-Control-1 ([SEQ ID NO.20]; 5'-GGCAGTGGCTCCCCATCTTTCGTTT-3') and 5mis-Control-3 ([SEQ ID NO.21]; 5'-CCGCGTTGTTTCGACCTTCCGCCTG-3').

### Abbreviations:

5'UTR, 5'-untranslated region; AC, animal cap; APC, adenomatous polyposis coli; Atelo, atelocollagen; β-Cat, β-catenin; CML, chronic myeloid leukemia; CNH, citron homology; Cont, control; CSK3β, glycogen synthase kinase 3β; GST, glutathione S-transferase; HA, hemagglutinin; IP, Immunoprecipitation; LEF, lymphoid enhancer factor; MO(s), morpholino oligonucleotide(s); N.D., not detected; n-βgal, nuclear β -galactosidase; NLS, nuclear localization signal; N.S., not significant; ORF, open reading frame; pSer, phosphoserine; RT, reverse transcription; shRNA, short hairpin RNA; siRNA, small interfering RNA; TCF(4), T-cell factor(-4); TNIK, Traf2- and Nck-interacting kinase; WT, wild type.

## Claims

1. A compound represented by the general formula (I) or a pharmaceutically acceptable salt thereof as an active ingredient: 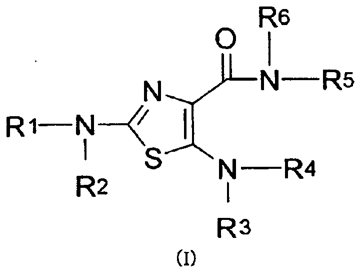 wherein R1, R2, R3, R4, R5 and R6 represent independently a hydrogen atom or a substituent group, for use as a TNIK inhibitor in the treatment of cancer.

2. A compound represented by the following general formula or a pharmaceutically acceptable salt thereof: 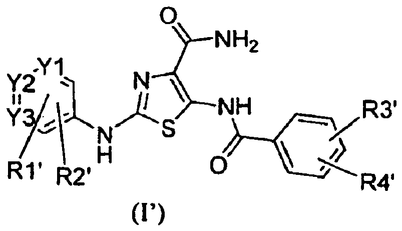 wherein R1' and R2' independently represent a hydrogen atom, a halogen atom, a hydroxy group, a substituted or unsubstituted alkyl group, C2-C4 alkenyl group, C2-C4 alkynyl group, a substituted or unsubstituted alkoxy group, a substituted or unsubstituted amino group, an acylamino group, a nitro group, a substituted or unsubstituted alkoxycarbonylamino group, R3' and R4' independently represent a hydrogen atom, a halogen atom, a hydroxy group, a substituted or unsubstituted alkyl group, a substituted or unsubstituted alkoxy group, a substituted or unsubstituted amino group, a substituted or unsubstituted acylamino group or a substituted or unsubstituted alkyl sulfonamido group, a nitro group and Y1, Y2 and Y3 independently represent a nitrogen atom or a carbon atom, for use as a medicament.

3. The compound for use according to claim 2, wherein R1' and R2' independently represent a hydrogen atom, a halogen atom, a hydroxy group, a substituted or unsubstituted alkyl group, a substituted or unsubstituted alkoxy group, a substituted or unsubstituted amino group, an acylamino group, a nitro group, a substituted or unsubstituted alkoxycarbonylamino group, R3' and R4' independently represent a hydrogen atom, a halogen atom, a hydroxy group, a substituted or unsubstituted alkyl group, a substituted or unsubstituted alkoxy group, a substituted or unsubstituted amino group, a substituted or unsubstituted acylamino group or a substituted or unsubstituted alkyl sulfonamido group, and Y1, Y2 and Y3 independently represent a nitrogen atom or a carbon atom.

4. The compound for use according to claim 2 or 3, being used as a TNIK inhibitor in the treatment of cancer.

5. The compound for use according to any of claims 1-4, being provided in a pharmaceutical composition, which comprises an effective amount of the compound or pharmaceutically acceptable salt thereof as an active ingredient.

6. The compound for use according to any of claims 1-5, which is administered in an effective amount.

7. The compound for use according to any of claims 1-6, wherein the cancer is solid cancer.

8. The compound for use according to any of claims 1-7, wherein the cancer is colorectal cancer.

9. The compound for use according to any of claims 1-7, wherein the cancer is pancreatic cancer.

10. The compound for use according to any of claims 1-7, wherein the cancer is non-small cell lung cancer.

11. The compound for use according to any of claims 1-7, wherein the cancer is prostate cancer.

12. The compound for use according to any of claims 1-7, wherein the cancer is breast cancer.

13. The compound for use according to any of claims 1-12, wherein the TNIK inhibitor is administered orally.

14. The compound for use according to any of claims 1-12, wherein the TNIK inhibitor is administered intravenously.

## Patentansprüche

1. Verbindung, die durch die allgemeine Formel (I) dargestellt ist, oder ein pharmazeutisch verträgliches Salz davon, als ein Wirkstoff: 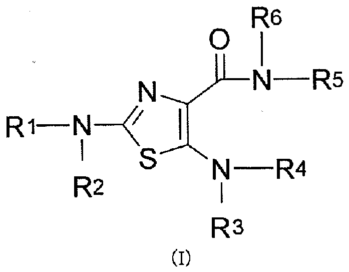 wobei R1, R2, R3, R4, R5 und R6 unabhängig ein Wasserstoffatom oder eine Substituentengruppe darstellen, zur Verwendung als ein TNIK-Inhibitor in der Behandlung von Krebs.

2. Verbindung, die durch die folgende allgemeine Formel dargestellt ist, oder ein pharmazeutisch akzeptables Salz davon: 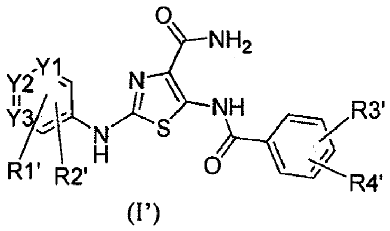 wobei R1' und R2' unabhängig ein Wasserstoffatom, ein Halogenatom, eine Hydroxygruppe, eine substituierte oder unsubstituierte Alkylgruppe, eine C2-C4 Alkenylgruppe, eine C2-C4 Alkinylgruppe, eine substituierte oder unsubstituierte Alkoxygruppe, eine substituierte oder unsubstituierte Aminogruppe, eine Acylaminogruppe, eine Nitrogruppe, eine substituierte oder unsubstituierte Alkoxycarbonylaminogruppe darstellen, R3' und R4' unabhängig ein Wasserstoffatom, ein Halogenatom, eine Hydroxygruppe, eine substituierte oder unsubstituierte Alkylgruppe, eine substituierte oder unsubstituierte Alkoxygruppe, eine substituierte oder unsubstituierte Aminogruppe, eine substituierte oder unsubstituierte Acylaminogruppe oder eine substituierte oder unsubstituierte Alkylsulfonamidogruppe, eine Nitrogruppe darstellen, und Y1, Y2 und Y3 unabhängig ein Stickstoffatom oder ein Kohlenstoffatom darstellen, zur Verwendung als ein Medikament.

3. Verbindung zur Verwendung nach Anspruch 2, wobei R1' und R2' unabhängig ein Wasserstoffatom, ein Halogenatom, eine Hydroxygruppe, eine substituierte oder unsubstituierte Alkylgruppe, eine substituierte oder unsubstituierte Alkoxygruppe, eine substituierte oder unsubstituierte Aminogruppe, eine Acylaminogruppe, eine Nitrogruppe, eine substituierte oder unsubstituierte Alkoxycarbonylaminogruppe darstellen, R3' und R4' unabhängig ein Wasserstoffatom, ein Halogenatom, eine Hydroxygruppe, eine substituierte oder unsubstituierte Alkylgruppe, eine substituierte oder unsubstituierte Alkoxygruppe, eine substituierte oder unsubstituierte Aminogruppe, eine substituierte oder unsubstituierte Acylaminogruppe oder eine substituierte oder unsubstituierte Alkylsulfonamidogruppe darstellen, und Y1, Y2 und Y3 unabhängig ein Stickstoffatom oder ein Kohlenstoffatom darstellen.

4. Verbindung zur Verwendung nach Anspruch 2 oder 3, die als ein TNIK-Inhibitor in der Behandlung von Krebs verwendet wird.

5. Verbindung zur Verwendung nach einem der Ansprüche 1-4, die in einer pharmazeutischen Zusammensetzung bereitgestellt ist, welche eine wirksame Menge der Verbindung oder eines pharmazeutisch verträglichen Salzes davon als einen Wirkstoff umfasst.

6. Verbindung zur Verwendung nach einem der Ansprüche 1-5, welches in einer wirksamen Menge verabreicht wird.

7. Verbindung zur Verwendung nach einem der Ansprüche 1-6, wobei der Krebs solider Krebs ist.

8. Verbindung zur Verwendung nach einem der Ansprüche 1-7, wobei der Krebs kolorektaler Krebs ist.

9. Verbindung zur Verwendung nach einem der Ansprüche 1-7, wobei der Krebs Bauchspeicheldrüsenkrebs ist.

10. Verbindung zur Verwendung nach einem der Ansprüche 1-7, wobei der Krebs nicht-kleinzelliger Lungenkrebs ist.

11. Verbindung zur Verwendung nach einem der Ansprüche 1-7, wobei der Krebs Prostatakrebs ist.

12. Verbindung zur Verwendung nach einem der Ansprüche 1-7, wobei der Krebs Brustkrebs ist.

13. Verbindung zur Verwendung nach einem der Ansprüche 1-12, wobei der TNIK-Inhibitor oral verabreicht wird.

14. Verbindung zur Verwendung nach einem der Ansprüche 1-12, wobei der TNIK-Inhibitor intravenös verabreicht wird.

## Revendications

1. Composé représenté par la formule générale (I) ou un sel pharmaceutiquement acceptable de celui-ci comme principe actif : 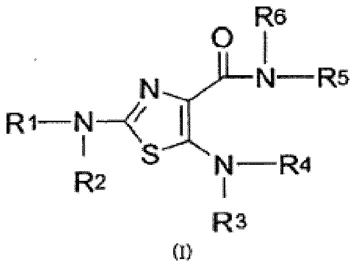 dans laquelle R1, R2, R3, R4, R5 et R6 représentent indépendamment un atome d'hydrogène ou un groupe substituant, pour une utilisation comme inhibiteur de TNIK dans le traitement du cancer.

2. Composé représenté par la formule générale suivante ou un sel pharmaceutiquement acceptable de celuici : 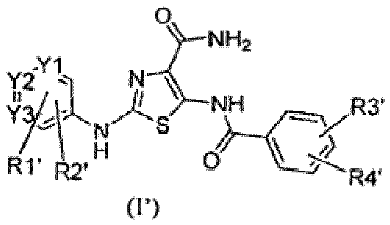 dans laquelle R1' et R2' représentent indépendamment un atome d'hydrogène, un atome d'halogène, un groupe hydroxy, un groupe alkyle substitué ou non substitué, un groupe alcényle en C2-C4, un groupe alcynyle en C2-C4, un groupe alcoxy substitué ou non substitué, un groupe amino substitué ou non substitué, un groupe acylamino, un groupe nitro, un groupe alcoxycarbonylamino substitué ou non substitué, R3' et R4' représentent indépendamment un atome d'hydrogène, un atome d'halogène, un groupe hydroxy, un groupe alkyle substitué ou non substitué, un groupe alcoxy substitué ou non substitué, un groupe amino substitué ou non substitué, un groupe acylamino substitué ou non substitué ou un groupe alkyl sulfonamido substitué ou non substitué, un groupe nitro et Y1, Y2 et Y3 représentent indépendamment un atome d'azote ou un atome de carbone, pour une utilisation comme médicament.

3. Composé pour une utilisation selon la revendication 2, dans lequel R1' et R2' représentent indépendamment un atome d'hydrogène, un atome d'halogène, un groupe hydroxy, un groupe alkyle substitué ou non substitué, un groupe alcoxy substitué ou non substitué, un groupe amino substitué ou non substitué, un groupe acylamino, un groupe nitro, un groupe alcoxycarbonylamino substitué ou non substitué, R3' et R4' représentent indépendamment un atome d'hydrogène, un atome d'halogène, un groupe hydroxy, un groupe alkyle substitué ou non substitué, un groupe alcoxy substitué ou non substitué, un groupe amino substitué ou non substitué, un groupe acylamino substitué ou non substitué ou un groupe alkyl sulfonamido substitué ou non substitué, et Y1, Y2 et Y3 représentent indépendamment un atome d'azote ou un atome de carbone.

4. Composé pour une utilisation selon la revendication 2 ou 3, étant utilisé comme inhibiteur de TNIK dans le traitement du cancer.

5. Composé pour une utilisation selon l'une quelconque des revendications 1 à 4, étant fourni dans une composition pharmaceutique, qui comprend une quantité efficace du composé ou d'un sel pharmaceutiquement acceptable de celui-ci comme principe actif.

6. Composé pour une utilisation selon l'une quelconque des revendications 1 à 5, qui est administré en une quantité efficace.

7. Composé pour une utilisation selon l'une quelconque des revendications 1 à 6, dans lequel le cancer est un cancer solide.

8. Composé pour une utilisation selon l'une quelconque des revendications 1 à 7, dans lequel le cancer est un cancer colorectal.

9. Composé pour une utilisation selon l'une quelconque des revendications 1 à 7, dans lequel le cancer est un cancer du pancréas.

10. Composé pour une utilisation selon l'une quelconque des revendications 1 à 7, dans lequel le cancer est un cancer du poumon non à petites cellules.

11. Composé pour une utilisation selon l'une quelconque des revendications 1 à 7, dans lequel le cancer est un cancer de la prostate.

12. Composé pour une utilisation selon l'une quelconque des revendications 1 à 7, dans lequel le cancer est un cancer du sein.

13. Composé pour une utilisation selon l'une quelconque des revendications 1 à 12, dans lequel l'inhibiteur de TNIK est administré par voie orale.

14. Composé pour une utilisation selon l'une quelconque des revendications 1 à 12, dans lequel l'inhibiteur de TNIK est administré par voie intraveineuse.


### SEQUENCE LISTING

<110> Yamada, Tesshi Shitashige, Miki Yokota, Koichi Sawa, Masaaki Moriyama, Hideki
<120> TNIK INHIBITOR AND THE USE
<130> PCT09CB001
<140> US 12/503,374
   <141> 2009-07-15
<150> US 61/118809
   <151> 2008-12-01
<150> US 12/503374
   <151> 2009-07-15
<160> 21
<170> PatentIn version 3.1
<210> 1
   <211> 40
   <212> DNA
   <213> Artificial
<220>
   <223> forward primer
<400> 1
   aatttcaggg cgccatggcg agcgactccc cggctcgaag 40
<210> 2
   <211> 42
   <212> DNA
   <213> Artificial
<220>
   <223> reverse primer
<400> 2
   attcgaaagc ggccgctcat cctcgcttct tctttgttct at 42
<210> 3
   <211> 7
   <212> PRT
   <213> Artificial
<220>
   <223> substrate peptides
<220>
   <221> MOD_RES
   <222> (1)..(1)
   <223> Acp
<400> 3
   Lys Tyr Lys Thr Leu Arg Gln
   1 5
<210> 4
   <211> 29
   <212> DNA
   <213> Artificial
<220>
   <223> oligo for mutagenesis
<400> 4
   ggccccatca ccggcacaca ttgtctcta 29
<210> 5
   <211> 29
   <212> DNA
   <213> Artificial
<220>
   <223> oligo for mutagenesis
<400> 5
   ggggcatcct tgagggcttg tctactctg 29
<210> 6
   <211> 21
   <212> RNA
   <213> Artificial
<220>
   <223> sense RNA for siRNA
<400> 6
   cgacauaccc agacugauau u 21
<210> 7
   <211> 21
   <212> RNA
   <213> Artificial
<220>
   <223> antisense RNA for siRNA
<220>
   <221> modified_base
   <222> (1)..(1)
   <223> phosphorylated uracil
<400> 7
   uaucagucug gguaugucgu u 21
<210> 8
   <211> 21
   <212> RNA
   <213> Artificial
<220>
   <223> sense RNA for siRNA
<400> 8
   gaccgaagcu cuugguuacu u 21
<210> 9
   <211> 21
   <212> RNA
   <213> Artificial
<220>
   <223> antisense RNA for siRNA
<220>
   <221> modified_base
   <222> (1)..(1)
   <223> phosphorylated guanine
<400> 9
   guaaccaaga gcuucggucu u 21
<210> 10
   <211> 21
   <212> RNA
   <213> Artificial
<220>
   <223> shRNA
<400> 10
   acacacuggu uuccauguaa u 21
<210> 11
   <211> 21
   <212> RNA
   <213> Artificial
<220>
   <223> shRNA
<400> 11
   agagaaggaa ccuugaugau u 21
<210> 12
   <211> 21
   <212> RNA
   <213> Artificial
<220>
   <223> shRNA
<400> 12
   agaaagauuu cggugguaaa u 21
<210> 13
   <211> 21
   <212> RNA
   <213> Artificial
<220>
   <223> shRNA
<400> 13
   ggaaucucau ucgaugcaua c 21
<210> 14
   <211> 25
   <212> DNA
   <213> Artificial
<220>
   <223> oligo for mutagenesis
<400> 14
   agggtcatgg atgtcacagg ggatg 25
<210> 15
   <211> 27
   <212> DNA
   <213> Artificial
<220>
   <223> oligo for mutagenesis
<400> 15
   aatagctgca agctgtccgg ttttaac 27
<210> 16
   <211> 35
   <212> DNA
   <213> Artificial
<220>
   <223> oligo for mutagenesis
<400> 16
   atggccagtg attctccggc tcgtagcctg gatga 35
<210> 17
   <211> 27
   <212> DNA
   <213> Artificial
<220>
   <223> oligo for mutagenesis
<400> 17
   atcgatggga tcctgcaaaa agaacaa 27
<210> 18
   <211> 25
   <212> DNA
   <213> Artificial
<220>
   <223> antisense morpholino oligonucleotide for xenopus
<400> 18
   gggagtcgct cgccatgttt ccttt 25
<210> 19
   <211> 25
   <212> DNA
   <213> Artificial
<220>
   <223> antisense morpholino oligonucleotide for xenopus
<400> 19
   ccccgttctt tccaccttgc ggctg 25
<210> 20
   <211> 25
   <212> DNA
   <213> Artificial
<220>
   <223> control morpholino oligonucleotide for xenopus
<400> 20
   ggcagtggct ccccatcttt cgttt 25
<210> 21
   <211> 25
   <212> DNA
   <213> Artificial
<220>
   <223> control morpholino oligonucleotide for xenopus
<400> 21
   ccgcgttgtt tcgaccttcc gcctg 25